# EUROPEAN PATENT SPECIFICATION

(11) **EP 1 613 651 B1**
(45) Date of publication and mention of the grant of the patent: **07.01.2015**
(21) Application number: 04725135.0
(22) Date of filing: 01.04.2004
(51) Int. Cl.: C07K 14/705, C07K 7/06, C07K 7/08

(54) **METHODS AND COMPOSITIONS FOR THE INHIBITION OF MODULATION OF T CELL COSTIMULATORY PATHWAY BY A PATHOGENIC AGENT**
VERFAHREN UND ZUSAMMENSETZUNGEN ZUR HEMMUNG DER MODULATION VON T-ZELL KOSTIMULIERUNGSWEGEN DURCH EIN PATHOGENES AGENS
PROCEDES ET COMPOSITIONS POUR INHIBER LA MODULATION DE LA VOIE DE COSTIMULATION DES LYMPHOCYTES T PAR UN AGENT PATHOGENE

(30) Priority: 03.04.2003 WO PCT/IL03/00278
(43) Date of publication of application: 11.01.2006
(60) Divisional application: 10011769.6
(73) Proprietor: YISSUM RESEARCH DEVELOPMENT COMPANY OF THE HEBREW UNIVERSITY OF JERUSALEM, 91390 Jerusalem (IL)
(72) Inventor: KAEMPFER, Raymond, 93707 Jerusalem (IL); ARAD, Gila, 90805 Mevaseret Zion (IL)
(74) Representative: Bentham, Andrew
(86) International application number: PCT/IL2004/000299
(87) International publication number: WO 2004/087196

(56) References cited:
- WO-A-97/37687
- WO-A-02/074803
- WO-A-02/096941
- WO-A-03/084995
- US-B1- 6 337 316
- ARAD G ET AL: "Superantigen antagonist protects against lethal shock and defines a new domain for T-cell activation" NATURE MEDICINE, NATURE PUBLISHING, CO, US, vol. 6, no. 4, April 2000 (2000-04), pages 414-421, XP002255219 ISSN: 1078-8956 cited in the application
- SCHWARTZ J C ET AL: "Structural basis for co-stimulation by the human CTLA-4/B7-2 complex." NATURE. 29 MAR 2001, vol. 410, no. 6828, 29 March 2001 (2001-03-29), pages 604-608, XP002306812 ISSN: 0028-0836 cited in the application

### SEQUENCE LISTING

<110> Yissum Research and Development
<120> Methods and compositions for the inhibition of modulation of T cell costimulatory pathway by a Pathogenic Agent
<130> 17428/WO/04
<160> 59
<170> PatentIn version 3.1
<210> 1
   <211> 12
   <212> PRT
   <213> Artificial sequence
<220>
   <223> Synthetic Peptide p12A
<400> 1
   Tyr Asn Lys Lys Lys Ala Thr Val Gln Glu Leu Asp
   1               5                   10
<210> 2
   <211> 12
   <212> PRT
   <213> Artificial sequence
<220>
   <223> Synthetic Peptide derived from residues 150-161 of SEB
<400> 2
   Thr Asn Lys Lys Lys Val Thr Ala Gln Glu Leu Asp
   1               5                   10
<210> 3
   <211> 14
   <212> PRT
   <213> Artificial sequence
<220>
   <223> Synthetic Peptide p12B
<400> 3
   Val Gln Tyr Asn Lys Lys Lys Ala Thr Val Gln Glu Leu Asp
   1               5                   10
<210> 4
   <211> 9
   <212> PRT
   <213> Artificial sequence
<220>
   <223> Synthetic Scrambled Control Peptide p1TAsc
<400> 4
   Cys His Gly His Leu Val Pro Lys Lys
   1 5
<210> 5
   <211> 8
   <212> PRT
   <213> Artificial sequence
<220>
   <223> Synthetic Scrambled Control Peptide p2TAsc
<400> 5
   Ala Ser Met Asp Tyr Pro Val Leu
   1 5
<210> 6
   <211> 12
   <212> PRT
   <213> Artificial sequence
<220>
   <223> Synthetic Peptide pe12
<400> 6
   Ser His Phe Thr His Asn Arg His Gly His Ser Thr
   1 5 10
<210> 7
   <211> 12
   <212> PRT
   <213> Artificial sequence
<220>
   <223> Synthetic Peptide pd7
<400> 7
   Trp His Ala His Pro His Lys Lys Pro Val Val Ala
   1 5 10
<210> 8
   <211> 12
   <212> PRT
   <213> Artificial sequence
<220>
   <223> Synthetic Peptide pc3
<400> 8
   Phe His Lys His Lys Asn Pro Gly Ser Pro Ile Ile
   1 5 10
<210> 9
   <211> 9
   <212> PRT
   <213> Artificial sequence
<220>
   <223> Synthetic Peptide p1TA
<400> 9
   His Val Lys Gly Lys His Leu Cys Pro
   1 5
<210> 10
   <211> 6
   <212> PRT
   <213> Artificial sequence
<220>
   <223> Synthetic Peptide of CD28 dimerization domain
<400> 10
   His Val Lys Gly Lys His
   1 5
<210> 11
   <211> 16
   <212> PRT
   <213> Artificial sequence
<220>
   <223> Synthetic Peptide
<400> 11
   Ala Ala Ala Ala Ala Ala Ala His Val Lys Gly Lys His Leu Cys Pro
   1 5 10 15
<210> 12
   <211> 8
   <212> PRT
   <213> Artificial sequence
<220>
   <223> Synthetic Peptide p2TA
<400> 12
   Ser Pro Met Leu Val Ala Tyr Asp
   1 5
<210> 13
   <211> 12
   <212> PRT
   <213> Artificial sequence
<220>
   <223> Synthetic Peptide TSST-1 antagonist domain
<400> 13
   Phe Asp Lys Lys Gln Leu Ala Ile Ser Thr Leu Asp
   1 5 10
<210> 14
   <211> 9
   <212> PRT
   <213> Artificial sequence
<220>
   <223> Synthetic Peptide p1TB
<400> 14
   Tyr Val Ile Asp Pro Glu Pro Cys Pro
   1 5
<210> 15
   <211> 8
   <212> PRT
   <213> Artificial sequence
<220>
   <223> Synthetic Peptide p2TB
<400> 15
   Pro Ala Val Val Leu Ala Ser Ser
   1 5
<210> 16
   <211> 9
   <212> PRT
   <213> Artificial sequence
<220>
   <223> Synthetic Peptide p1TC
<400> 16
   Tyr Glu Ser Gln Leu Cys Cys Gln Leu
   1 5
<210> 17
   <211> 8
   <212> PRT
   <213> Artificial sequence
<220>
   <223> Synthetic Peptide p2TC
<400> 17
   Gly Glu Ile Asn Gly Ser Ala Asn
   1 5
<210> 18
   <211> 6
   <212> PRT
   <213> Artificial sequence
<220>
   <223> Synthetic Peptide of CTLA4 dimer interface
<400> 18
   Tyr Val Ile Asp Pro Glu
   1 5
<210> 19
   <211> 202
   <212> PRT
   <213> Homo sapiens
<400> 19
   Asn Lys Ile Leu Val Lys Gln Ser Pro Met Leu Val Ala Tyr Asp Asn
   1 5 10 15
   Ala Val Asn Leu Ser Cys Lys Tyr Ser Tyr Asn Leu Phe Ser Arg Glu
   20 25 30
   Phe Arg Ala Ser Leu His Lys Gly Leu Asp Ser Ala Val Glu Val Cys
   35 40 45
   Val Val Tyr Gly Asn Tyr Ser Gln Gln Leu Gln Val Tyr Ser Lys Thr
   50 55 60
   Gly Phe Asn Cys Asp Gly Lys Leu Gly Asn Glu Ser Val Thr Phe Tyr
   65 70 75 80
   Leu Gln Asn Leu Tyr Val Asn Gln Thr Asp Ile Tyr Phe Cys Lys Ile
   85 90 95
   Glu Val Met Tyr Pro Pro Pro Tyr Leu Asp Asn Glu Lys Ser Asn Gly
   100 105 110
   Thr Ile Ile His Val Lys Gly Lys His Leu Cys Pro Ser Pro Leu Phe
   115 120 125
   Pro Gly Pro Ser Lys Pro Phe Trp Val Leu Val Val Val Gly Gly Val
   130 135 140
   Leu Ala Cys Tyr Ser Leu Leu Val Thr Val Ala Phe Ile Ile Phe Trp
   145 150 155 160
   Val Arg Ser Lys Arg Ser Arg Leu Leu His Ser Asp Tyr Met Asn Met
   165 170 175
   Thr Pro Arg Arg Pro Gly Pro Thr Arg Lys His Tyr Gln Pro Tyr Ala
   180 185 190
   Pro Pro Arg Asp Phe Ala Ala Tyr Arg Ser
   195 200
<210> 20
   <211> 188
   <212> PRT
   <213> Homo sapiens
<400> 20
   Lys Ala Met His Val Ala Gln Pro Ala Val Val Leu Ala Ser Ser Arg
   1 5 10 15
   Gly Ile Ala Ser Phe Val Cys Glu Tyr Ala Ser Pro Gly Lys Ala Thr
   20 25 30
   Glu Val Arg Val Thr Val Leu Arg Gln Ala Asp Ser Gln Val Thr Glu
   35 40 45
   Val Cys Ala Ala Thr Tyr Met Met Gly Asn Glu Leu Thr Phe Leu Asp
   50 55 60
   Asp Ser Ile Cys Thr Gly Thr Ser Ser Gly Asn Gln Val Asn Leu Thr
   65 70 75 80
   Ile Gln Gly Leu Arg Ala Met Asp Thr Gly Leu Tyr Ile Cys Lys Val
   85 90 95
   Glu Leu Met Tyr Pro Pro Pro Tyr Tyr Leu Gly Ile Gly Asn Gly Thr
   100 105 110
   Gln Ile Tyr Val Ile Asp Pro Glu Pro Cys Pro Asp Ser Asp Phe Leu
   115 120 125
   Leu Trp Ile Leu Ala Ala Val Ser Ser Gly Leu Phe Phe Tyr Ser Phe
   130 135 140
   Leu Leu Thr Ala Val Ser Leu Ser Lys Met Leu Lys Lys Arg Ser Pro
   145 150 155 160
   Leu Thr Thr Gly Val Tyr Val Lys Met Pro Pro Thr Glu Pro Glu Cys
   165 170 175
   Glu Lys Gln Phe Gln Pro Tyr Phe Ile Pro Ile Asn
   180 185
<210> 21
   <211> 156
   <212> PRT
   <213> Homo sapiens
<400> 21
   Leu Arg Ile Lys Val Leu Thr Gly Glu Ile Asn Gly Ser Ala Asn Tyr
   1 5 10 15
   Glu Met Phe Ile Phe His Asn Gly Gly Val Gln Ile Leu Cys Lys Tyr
   20 25 30
   Pro Asp Ile Val Gln Gln Phe Lys Met Gln Leu Leu Lys Gly Gly Gln
   35 40 45
   Ile Leu Cys Asp Leu Thr Lys Thr Lys Gly Ser Gly Asn Thr Val Ser
   50 55 60
   Ile Lys Ser Leu Lys Phe Cys His Ser Gln Leu Ser Asn Asn Ser Val
   65 70 75 80
   Ser Phe Phe Leu Tyr Asn Leu Asp His Ser His Ala Asn Tyr Tyr Phe
   85 90 95
   Cys Asn Leu Ser Ile Phe Asp Pro Pro Pro Phe Lys Val Thr Leu Thr
   100 105 110
   Gly Gly Tyr Leu His Ile Tyr Glu Ser Gln Leu Cys Cys Gln Leu Lys
   115 120 125
   Phe Trp Leu Pro Ile Gly Cys Ala Ala Phe Val Val Val Cys Ile Leu
   130 135 140
   Gly Cys Ile Leu Ile Cys Trp Leu Thr Lys Lys Met
   145 150 155
<210> 22
   <211> 6
   <212> PRT
   <213> Artificial sequence
<220>
   <223> Synthetic Peptide B7 binding domain
<400> 22
   Met Tyr Pro Pro Pro Tyr
   1 5
<210> 23
   <211> 6
   <212> PRT
   <213> Artificial sequence
<220>
   <223> Synthetic Peptide
<400> 23
   Val Val Leu Ala Ser Ser
   1 5
<210> 24
   <211> 6
   <212> PRT
   <213> Artificial sequence
<220>
   <223> Synthetic Peptide
<400> 24
   Met Leu Val Ala Tyr Asp
   1 5
<210> 25
   <211> 12
   <212> PRT
   <213> Artificial sequence
<220>
   <223> Synthetic Peptide pa2
<400> 25
   Phe His Lys His Ser Pro Arg Ser Pro Ile Phe Ile
   1 5 10
<210> 26
   <211> 12
   <212> PRT
   <213> Artificial sequence
<220>
   <223> Synthetic Peptide pb11
<400> 26
   Ser Trp Pro His His His Arg Met Pro Leu Leu Ala
   1 5 10
<210> 27
   <211> 12
   <212> PRT
   <213> Artificial sequence
<220>
   <223> Synthetic Peptide pc11
<400> 27
   Phe His Lys Thr Pro Arg Ile Ala Pro Pro Pro Leu
   1 5 10
<210> 28
   <211> 12
   <212> PRT
   <213> Artificial sequence
<220>
   <223> Synthetic Peptide pf11
<400> 28
   His Ser Ser His His Ser His Arg Ala Pro Thr Thr
   1 5 10
<210> 29
   <211> 12
   <212> PRT
   <213> Artificial sequence
<220>
   <223> Synthetic Peptide pg3
<400> 29
   His Asn Ser Tyr His His Gln His Lys Pro Thr Ser
   1 5 10
<210> 30
   <211> 12
   <212> PRT
   <213> Artificial sequence
<220>
   <223> Synthetic Peptide pb12
<400> 30
   Tyr His Arg Pro His Glu His Lys Met Phe Gln Pro
   1 5 10
<210> 31
   <211> 12
   <212> PRT
   <213> Artificial sequence
<220>
   <223> Synthetic Peptide pa8.1
<400> 31
   Ala His Lys Ala His Lys His Met Pro Trp Ile Asn
   1 5 10
<210> 32
   <211> 12
   <212> PRT
   <213> Artificial Sequence
<220>
   <223> Synthetic Peptide pb3
<400> 32
   Ala Pro Trp Thr His His Ser Lys His Ser His Pro
   1 5 10
<210> 33
   <211> 12
   <212> PRT
   <213> Artificial sequence
<220>
   <223> Synthetic Peptide pb5
<400> 33
   Lys Pro Phe His His Asp His Ser Lys Gln His Gln
   1 5 10
<210> 34
   <211> 12
   <212> PRT
   <213> Artificial sequence
<220>
   <223> Synthetic Peptide pb11
<400> 34
   Ala Arg Leu His Thr His Gln His Ser Asn Met Arg
   1 5 10
<210> 35
   <211> 12
   <212> PRT
   <213> Artificial sequence
<220>
   <223> Synthetic Peptide pf3
<400> 35
   Gly Gln Thr His His His His Arg Phe Phe Gly Ser
   1 5 10
<210> 36
   <211> 12
   <212> PRT
   <213> Artificial sequence
<220>
   <223> Synthetic Peptide pf8
<400> 36
   Ile His Lys Pro His His His Arg Thr Pro Leu Trp
   1 5 10
<210> 37
   <211> 12
   <212> PRT
   <213> Artificial sequence
<220>
   <223> Synthetic Peptide pe6
<400> 37
   Ala Pro Met Tyr His Lys His Arg Leu Glu Lys His
   1 5 10
<210> 38
   <211> 12
   <212> PRT
   <213> Artificial sequence
<220>
   <223> Synthetic Peptide pf4
<400> 38
   Trp His Lys Ile Pro Gln Lys Ala Pro Leu Asn Pro
   1 5 10
<210> 39
   <211> 12
   <212> PRT
   <213> Artificial sequence
<220>
   <223> Synthetic Peptide pa8.2
<400> 39
   Tyr Pro His Ile His Thr His Arg Pro Pro Val His
   1 5 10
<210> 40
   <211> 12
   <212> PRT
   <213> Artificial sequence
<220>
   <223> Synthetic Peptide pb3
<400> 40
   Ala Trp Asn Ser Pro His Gln His His His Arg Lys
   1 5 10
<210> 41
   <211> 12
   <212> PRT
   <213> Artificial sequence
<220>
   <223> Synthetic Peptide pb2
<400> 41
   Trp Pro Arg His His His Ser Gly Glu Leu Lys Thr
   1 5 10
<210> 42
   <211> 12
   <212> PRT
   <213> Artificial sequence
<220>
   <223> Synthetic Peptide pc2
<400> 42
   Ser His Trp His Ser Lys Leu Arg Tyr Phe Pro Pro
   1 5 10
<210> 43
   <211> 12
   <212> PRT
   <213> Artificial sequence
<220>
   <223> Synthetic Peptide pc8
<400> 43
   Leu Pro His His Lys His Arg Pro Asn Leu Pro Ser
   1 5 10
<210> 44
   <211> 12
   <212> PRT
   <213> Artificial sequence
<220>
   <223> Synthetic Peptide pc9
<400> 44
   Phe His Lys His Asn Tyr Lys Ser Pro Pro Ile Ile
   1 5 10
<210> 45
   <211> 12
   <212> PRT
   <213> Artificial sequence
<220>
   <223> Synthetic Peptide pf12
<400> 45
   Trp Pro Met Lys His His His Leu Val Thr Ala Arg
   1 5 10
<210> 46
   <211> 12
   <212> PRT
   <213> Artificial sequence
<220>
   <223> Synthetic Peptide pc4
<400> 46
   His Ile Lys His Leu Ser His Trp Thr Pro Lys Pro
   1 5 10
<210> 47
   <211> 12
   <212> PRT
   <213> Artificial sequence
<220>
   <223> Synthetic Peptide pe11
<400> 47
   Ala His Arg His Gln His Gln His Pro His Ala Gln
   1               5                   10
<210> 48
   <211> 12
   <212> PRT
   <213> Artificial sequence
<220>
   <223> Synthetic Peptide pb5
<400> 48
   Leu Pro Trp His Arg His Gly Pro Ala Pro Ser Phe
   1               5                   10
<210> 49
   <211> 12
   <212> PRT
   <213> Artificial sequence
<220>
   <223> Synthetic Peptide pe11
<400> 49
   Ala Pro Trp Ser His His His Gly Lys Leu Pro Arg
   1               5                   10
<210> 50
   <211> 12
   <212> PRT
   <213> Artificial sequence
<220>
   <223> Synthetic Peptide pg7
<400> 50
   Gly Leu Trp His Ala Pro His Pro Ala His Arg His
   1               5                   10
<210> 51
   <211> 12
   <212> PRT
   <213> Artificial sequence
<220>
   <223> Synthetic Peptide pa12
<400> 51
   Thr Gln Gly His His His His Arg His Pro Arg Ile
   1 5 10
<210> 52
   <211> 12
   <212> PRT
   <213> Artificial sequence
<220>
   <223> Synthetic Peptide pb8
<400> 52
   Ser Pro His Asn His His Thr His Lys Pro Lys Ser
   1 5 10
<210> 53
   <211> 12
   <212> PRT
   <213> Artificial sequence
<220>
   <223> Synthetic Peptide pb12
<400> 53
   Leu Pro Met Lys His Ser Trp His Ser His Thr Leu
   1 5 10
<210> 54
   <211> 12
   <212> PRT
   <213> Artificial sequence
<220>
   <223> Synthetic Peptide pc8
<400> 54
   Ala Val Lys His His Tyr His Arg His Pro Ile Ile
   1 5 10
<210> 55
   <211> 12
   <212> PRT
   <213> Artificial sequence
<220>
   <223> Synthetic Peptide pd8
<400> 55
   Thr His Pro His Leu His His Arg His Leu Ala Pro
   1 5 10
<210> 56
   <211> 12
   <212> PRT
   <213> Artificial sequence
<220>
   <223> Synthetic Peptide pg6
<400> 56
   Gly Lys Met His Leu His His Pro His Ser Gln Pro
   1 5 10
<210> 57
   <211> 9
   <212> PRT
   <213> Artificial Sequence
<220>
   <223> Synthetic Peptide p1TD
<400> 57
   Arg Val Thr Glu Arg Arg Ala Glu Val
   1 5
<210> 58
   <211> 8
   <212> PRT
   <213> Artificial Sequence
<220>
   <223> Synthetic Peptide p2TD
<400> 58
   Pro Ala Leu Leu Val Val Thr Glu
   1 5
<210> 59
   <211> 255
   <212> PRT
   <213> Homo sapiens
<400> 59
   Pro Pro Thr Phe Phe Pro Ala Leu Leu Val Val Thr Glu Gly Asp Asn
   1               5                   10                  15
   Ala Thr Phe Thr Cys Ser Phe Ser Asn Thr Ser Glu Ser Phe Val Leu
               20                  25                  30
   Asn Trp Tyr Arg Met Ser Pro Ser Asn Gln Thr Asp Lys Leu Ala Ala
           35                  40                  45
   Phe Pro Glu Asp Arg Ser Gln Pro Gly Gln Asp Cys Arg Phe Arg Val
       50                  55                  60
   Thr Gln Leu Pro Asn Gly Arg Asp Phe His Met Ser Val Val Arg Ala
   65                  70                  75                  80
   Arg Arg Asn Asp Ser Gly Thr Tyr Leu Cys Gly Ala Ile Ser Leu Ala
                   85                  90                  95
   Pro Lys Ala Gln Ile Lys Glu Ser Leu Arg Ala Glu Leu Arg Val Thr
               100                 105                 110
   Glu Arg Arg Ala Glu Val Pro Thr Ala His Pro Ser Pro Ser Pro Arg
           115                 120                 125
   Pro Ala Gly Gln Phe Gln Thr Leu Val Val Gly Val Val Gly Gly Leu
       130                 135                 140
   Leu Gly Ser Leu Val Leu Leu Val Trp Val Leu Ala Val Ile Cys Ser
   145                 150                 155                 160
   Arg Ala Ala Arg Gly Thr Ile Gly Ala Arg Arg Thr Gly Gln Pro Leu
                   165                 170                 175
   Lys Glu Asp Pro Ser Ala Val Pro Val Phe Ser Val Asp Tyr Gly Glu
               180                 185                 190
   Leu Asp Phe Gln Trp Arg Glu Lys Thr Pro Glu Pro Pro Val Pro Cys
           195                 200                 205
   Val Pro Glu Gln Thr Glu Tyr Ala Thr Ile Val Phe Pro Ser Gly Met
       210                 215                 220
   Gly Thr Ser Ser Pro Ala Arg Arg Gly Ser Ala Asp Gly Pro Arg Ser
   225                 230                 235                 240
   Ala Gln Pro Leu Arg Pro Glu Asp Gly His Cys Ser Trp Pro Leu
                   245                 250                 255

## Description

### Field of the Invention

The invention relates to specific antagonist immunomodulatory peptides, compositions thereof and also such peptides and compositions for use in methods for the treatment of immune-related disorders.

### Background of the invention

A family of pyrogenic exotoxins, also known as superantigenic toxins, is produced by *Staphylococcus aureus* and *Streptococcus pyogenes.* The exotoxins comprised of the S. *aureus* enterotoxins (SEs) cause the majority of human food poisoning cases manifested by vomiting and diarrhea after ingestion [Schlievert, J. Infect. Dis. 167: 997 (1993)]. *S*. *aureus* is found widespread in nature, often in association with humans. Among the 5 major serological types within the family of SEs (labeled SEA to SEE and SEG), SEB is the most prominent [Marrack and Kappler, Science 248 : 705 (1990)]. SEB has also been recognized as a leading cause of human cases of non-menstrual toxic shock syndrome that can accompany surgical or injurious wound infections, as well as viral infections of the respiratory tract of influenza patients to which children are especially vulnerable [Schlievert (1993) *ibid.;* Tseng et al., Infect. Immun. 63:2880 (1995)]. Toxic shock syndrome, in its most severe form, causes shock and death [Murray et al., ASM News 61:229 (1995); Schlievert (1993) *ibid*.]*.* More generally, members of the staphylococcal exotoxin family, including SEA to SEE and toxic shock syndrome toxin 1 (TSST-1), have been implicated in toxic shock syndrome, in atopic dermatitis [Schlievert (1993) *ibid.]* and in Kawasaki's syndrome [Bohach et al., Crit.Rev.Microbiol. 17:251 (1990)].

Bypassing the restricted presentation of conventional antigens, superantigens produced by *Staphylococcus aureus* and *Streptococcus pyogenes* bind directly to most major histocompatibility (MHC) class II molecules and activate virtually all T cells bearing particular domains in the variable portion of the β chain of the T-cell receptor (TCR), without need for processing by antigen-presenting cells [Scholl, P. et al., Proc. Natl. Acad. Sci. USA 86:4210-4214 (1989); Fraser, J.D. Nature 339(6221):221-3 (1989); Choi, Y.W. et al., Nature 346(6283):471-3 (1990); Janeway, C.A. Jr. et al., Immunol. Rev. 107:61-88 (1989)]. This results in an excessive induction of T helper 1 (Th1) cytokines interleukin-2 (IL2), interferon-γ (IFN-γ) and tumor necrosis factor β, mediators of toxic shock [Marrack, P. and Kappler, J. Science 248:705-711 (1990a); Marrack, P. et al., J. Exp. Med. 171(2):455-64 (1990b); Miethke, T. et al., J. Exp. Med. 175(1):91-8 (1992); Hackett, S.P. and Stevens, D.L. J. Infect. Dis. 168:232-235 (1993); Arad, G. et al., Nat. Med. 6(4):414-21 (2000)]. Superantigens thus use the same ligands as conventional antigens but do so in a distinct manner [Sundberg, E.J. et al., Structure (Camb) 10:687-699 (2002a); Sundberg, E.J. et al., Curr. Opin. Immunol. 14:36-44 (2002b)]. Induction of human Th1 cytokine gene expression by divergent superantigens is inhibited by a superantigen mimetic peptide that protects mice from the lethal effect of these toxins [Arad (2000) *ibid*.]. The peptide shows homology to a β-strand-hinge-α-helix domain that is structurally conserved among the superantigens yet remote from their binding sites for MHC class II molecules and TCR. The antagonist activity of this peptide identified a novel superantigen domain that is critical for their action [Arad (2000) *ibid*.]. This finding raised the possibility that superantigens may use this domain to bind to a third receptor.

CD28 and B7-2 serve as principal costimulatory ligands for conventional antigens [reviewed by Lenschow, D.J. et al., Annu. Rev. Immunol. 14:233-58 (1996); Salomon, B. and Bluestone, J.A. Annu. Rev. Immunol. 19:225-52 (2001); Acuto, O. and Michel, F. Nat. Rev. Immunol. 3(12):939-51 (2003)]. The present inventors have shown that to deliver the signal for Th1 activation, a superantigen must bind directly to CD28 [WO 03/084995]. Signaling is blocked by peptide mimetics of the contact region in each ligand: the β-strand-hinge-α-helix domain in superantigens [Arad (2000) *ibid.*] and two noncontiguous domains in CD28 that form the predicted homodimerization interface.

CD28 belongs to a triad of costimulatory ligands whose genes are tightly linked: CD28, cytotoxic T-lymphocyte-associated protein 4 (CTLA4)(CD152) and inducible costimulator (ICOS) [reviewed by Sharpe, A.H. and Freeman, G.J., Nat. Rev. Immunol. 2(2):116-26 (2002); Carreno, B.M. and Collins, M. Annu. Rev. Immunol. 20:29-53 (2002)]. Via their coligands from the B7 family, these proteins function as costimulatory receptors that regulate signaling by ordinary antigens. CD28 acts as the critical early signal transducer for the innate immune response, balanced by ICOS and CTLA4 [reviewed by Rudd, C.E. and Schneider H. Nat. Rev. Immunol. 3(7):544-56 (2003)]. The present invention now shows that through its β-strand-hinge-α-helix domain, the major superantigen staphylococcal enterotoxin B (SEB) binds with high affinity to each member of this conserved receptor family. Peptides deriving from either rim of the bipartite dimer interface in CTLA4 [Schwartz, J. C. et al., Nature 410:604-608 (2001); Stamper, C. C. et al., Nature 410(6828):608-11 (2001)] or in CD28 and ICOS as predicted by sequence alignment, although unique for each costimulatory receptor, are potent antagonists that block superantigen-mediated induction of human Th1 cytokine gene expression and protect mice from lethal challenge with SEB. Apparently, the mode of action of these peptides is to compete with CD28 for its binding site in superantigens. SEB induces a vigorous expression of Th1 and Th2 cytokine genes but only induction of the Th1 response is dependent on CD28 signaling.

Direct binding to CD28 underlies the toxicity of the superantigens. The findings of the present invention reveal a mechanism of subversion of the innate immune response in which the superantigen co-opts a costimulatory ligand of the host for use as its obligatory receptor. This strategy may be used more widely by pathogens.

Therefore, it is an object of the invention to provide compositions for inhibiting the activation of a T cell costimulatory pathway, namely the CD28/B7 pathway, by a pathogenic agent, in a subject in need thereof. Such compositions are based on a substance which inhibits the direct interaction of a component derived from said pathogenic agent and a binding site within a T cell costimulatory pathway member molecule, which site is derived from the dimer interface of said T cell costimulatory pathway member.

Another object of the invention is to provide peptides which inhibit the direct interaction of a component derived from said pathogenic agent and a binding site within the dimer interface of a T cell costimulatory pathway member, preferably, CD28, CTLA4 and ICOS. Such peptides are provided by the invention and are peptides comprising an amino acid sequence derived from a dimer interface of a T cell costimulatory pathway member, for example the peptides of SEQ ID NO: 9,11, 12, 14, 15, 16,17, 57 and 58.

Another object of the invention is to provide compositions for use in treatment of immune-related disorders caused by a pathogenic agent, particularly, a superantigen exotoxin.

These and other objects of the invention will become apparent as the description proceeds.

### Summary of the Invention

In a first aspect, the invention relates to an isolated and purified peptide consisting of:
(a) a peptide comprising an amino acid sequence that is a dimer interface of a T cell costimulatory pathway member selected from the dimer interface within the human CD28, the human CTLA4, the human ICOS and the human PD-1, said amino acid sequence being selected from amino acid residues 10-15 or 116-121 of SEQ ID NO: 19, amino acid residues 10-15 or 115-120 of SEQ ID NO: 20, amino acid residues 10-15 or 119-124 ofSEQ ID NO: 21, and amino acid residues 8-13 or 110-116 ofSEQ ID NO: 59, where the peptide:
   (i) is a human CD28 dimer interface peptide consisting of the amino acid sequence HVKGKHLCP as denoted by SEQ ID NO: 9 or the amino acid sequence SPMLVAYD as denoted by SEQ ID NO: 12;
   (ii) is a human CTLA4 dimer interface peptide consisting of the amino acid sequence YVIDPEPCP as denoted by SEQ ID NO: 14 or the amino acid sequence PAWLASS as denoted by SEQ ID NO: 15;
   (iii) is a human ICOS dimer interface peptide consisting of the amino acid sequence YESQLCCQL as denoted by SEQ ID NO: 16 or the amino acid sequence GEINGSAN as denoted by SEQ ID NO: 17; or
   (iv) is a human PD-1 dimer interface peptide consisting of the amino acid sequence RVTERRAEV as denoted by SEQ ID NO: 57 or the amino acid sequence PALLWTE as denoted by SEQ ID NO: 58;
(b) a peptide which is at least 80% homologous to the human CD28 peptide of SEQ ID No: 9 or 12, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a peptide of (a), (b) or (c) that is extended at the N terminus and/or the C terminus:
   (i) by a lauryl cysteine at the N terminus and a cysteine at the C terminus; or
   (ii) by an organic moiety that is not a naturally occurring or synthetic amino acid residue; or
   (iii) by identical hydrophobic amino acid residue(s) which may be naturally occurring or synthetic amino acid residues; or
   (iv) by a lysyl-palmitoyl tail, wherein said tail is at the N terminus, wherein the resultant peptide of (c) maintains the ability to inhibit the interaction between a T cell costimulatory pathway member and a superantigen;
(e) a dimer or multimer of (a), (b), (c), or (d) wherein the resultant peptide of (e) maintains the ability to inhibit the interaction between a T Cell costimulatory pathway member and a superantigen.

Hence, the peptide of the invention is an immunomodulatory peptide capable of modulating a T cell costimulatory pathway.

In one preferred embodiment, the peptide of the invention may consist of an amino acid sequence from the dimer interface of a T cell costimulatory pathway member, selected from a CD28/B7 family member which is any one of CD28, CTLA4 and ICOS and the corresponding domains in PD-1.

According to a second aspect, the invention relates to a composition comprising as an active ingredient a purified peptide of the invention or any combination thereof and optionally further comprises pharmaceutically acceptable carrier, diluent, adjuvant and/ or excipient.

The invention further provides a composition of the invention for use in a method of treatment of an immune disorder related to an imbalance in the Th1-Th2 response in a subject in need thereof, wherein said immune disorder is any one of an autoimmune disease, malignant and non-malignant proliferative disorder, graft rejection pathology, and a disorder induced by a pyrogenic exotoxin or by a mixture of at least two pyrogenic exotoxins selected from toxic shock and incapacitation or for use in a method of preventing death induced by a pyrogenic exotoxin or by a mixture of at least two pyrogenic exotoxins.

In yet another embodiment, the invention relates to the use of a peptide of the invention for the preparation of a composition for use in the treatment of immune disorders related to an imbalance in the Th1-Th2 response in a subject in need thereof, wherein said immune disorder is any one of an autoimmune disease, malignant and non-malignant proliferative disorder, graft rejection pathology, and a disorder induced by a pyrogenic exotoxin or by a mixture of at least two pyrogenic exotoxins selected from toxic shock and incapacitation or for use in a method of preventing and death, induced by a pyrogenic exotoxin or by a mixture of at least two pyrogenic exotoxins.

Still further, the invention relates to a method of screening for a test substance which specifically binds to a T cell costimulatory pathway member and thereby inhibits activation of T-lymphocytes mediated by a pathogenic-agent, which screening method comprises the steps of:
(a) obtaining candidate antagonist substances which bind to a T cell costimulatory pathway member;
(b) selecting from the substances obtained in step (a), a substance that inhibits direct interaction between said T cell costimulatory pathway member and said pathogenic agent or a component derived from said agent, preferably said component is a superantigen; and
(c) determining the inhibitory effect of the substance obtained in step (b) on the superantigen-mediated activation of T-lymphocytes, preferably, Th1 lymphocytes, wherein said candidate antagonist substance is obtained by the steps of: (i) providing a mixture comprising an isolated peptide of the invention; (ii) contacting said mixture with said test substance under suitable conditions for said binding; and (iii) determining the effect of the test substance on an end-point indication, whereby modulation of said end point is indicative of binding of said test substance to said peptide.

The invention will be further described on the hand of the following figures, which are illustrative only and do not limit the scope of the invention which is defined by the appended claims.

### Brief Description of the Figures

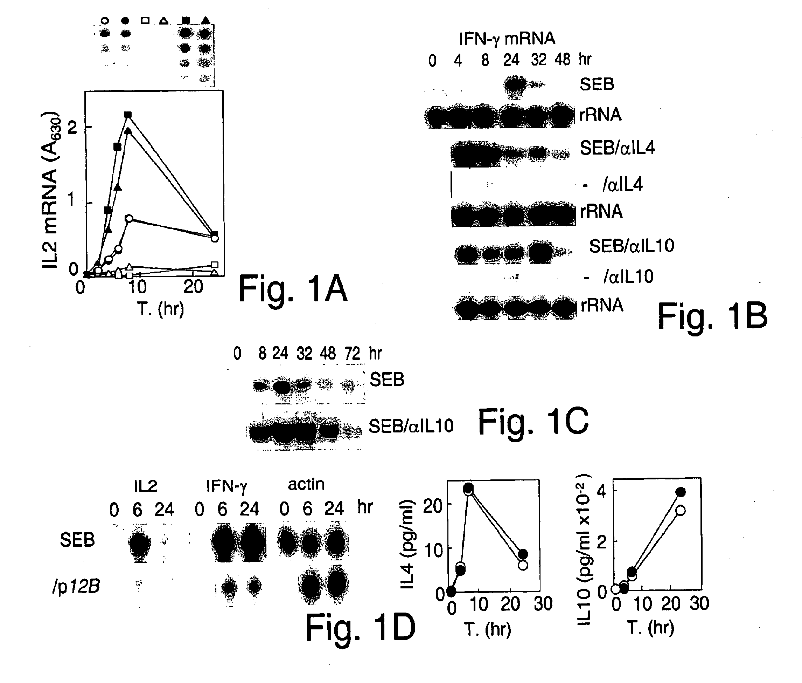

**Figure 1A-1F** *Selective Inhibition of Thl Activation by Superantigen Minsetic Peptide* **Fig.1A** Inhibition of SEB-induced IL2 gene expression by concomitant induction of IL4 and IL10. Human PBMC were incubated with 1:10⁴- fold diluted rat neutralizing Ab against human IL4 (αIL4; Genzyme) (□) or IL210 (αIL10; Pharmingen)(Δ), with SEB (○), or with SEB and isotype-matched antihuman IgG (●), αIL4 (■) or αIL10 (▲). IL2 mRNA was quantitated as described in Experimental Procedures by hybridization with IL2 antisense RNA probe; film blackening was read at 630 nm. Autoradiogram shows data for 6 hr.
**Figs. 1B,** 1C Inhibition of SEB-induced IFN-γ gene expression by concomitant induction of IL4 and IL10. PBMC were incubated with SEB or without SEB (-), and with αIL4 or αIL10 as shown; IFN-γ mRNA was quantitated by RNase protection analysis as described in Experimental Procedures, with rRNA as loading control. (B) and (C) represent separate experiments.
**Fig. 1D** *p12B* inhibits SEB-mediated induction of IL2 and IFN-γ mRNA but not of IL4 and IL10. PBMC were induced with SEB in absence (○) or presence of 10 µg/ml *p12B* (●). IL2 mRNA and IFN-γ mRNA were quantitated by RNase protection analysis, with actin mRNA as loading control. IL4 and IL10 in the culture medium were determined by Quantikine ELISA kits (R&D Systems).

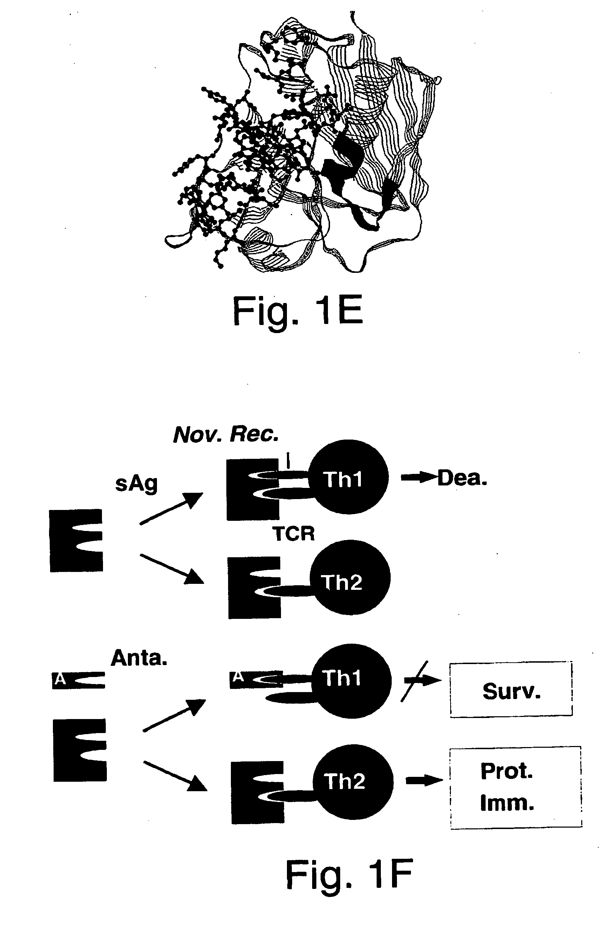

**Fig. 1E** The antagonist domain in SEB. In the SEB structure (pdb3seb.ent), generated with RasMol, the 160-161 domain is shown as magenta ribbon. Amino acid side chains contacting MHC II are shown in red and those contacting the TCR in green [Papageorgiou, A.C. et al., J. Mol. Biol. 277:61-79 (1998)].
**Fig. 1F** Working hypothesis: a novel receptor is used selectively for Th1 cell activation by superantigen. To activate Th1 cells, a superantigen must engage not only MHC II and TCR but also a novel receptor dispensable for Th2 cell activation. Binding of antagonist peptide to this receptor results in a selective block of Th1 cell activation, to yield survival as well as Th2-mediated protective immunity. For clarity, antigen-presenting cell (APC) and MHC II were omitted. Abbreviations: T (time), hr. (hour), pg/ml (Pico gram/milliliter), sAg (superantigen), Nov. Rec. (novel receptor), Dea. (Death), Surv. (survival), Prot. Imm. (protective immunity), Anta. (antagonist).
**Figure 2A-2K** *SEB Signals Through CD28*
**Figs. 2A-2C** Anti-B7-2 mAb inhibits SEB-induced expression of IL2 and IFN-γ mRNA but not of IL10. PBMC were incubated without SEB (□, Δ) or with 100 ng/ml SEB (○, ■, ▲), either without mAb (0) or with 1:10⁴-diluted anti-B7-1 (□, ■) or anti-B7-2 (Δ, ▲) (R&D Systems). IL2 mRNA and IFN-γ mRNA were analyzed as in Figure 1A (dots show 8-hr values). IL10 in culture medium was assayed by ELISA.
**Figs. 2D-2K** Effect of sCD28, sB7-2 and sCTLA4 on induction of IL2 mRNA, IFN-γ mRNA and IL10 by SEB.

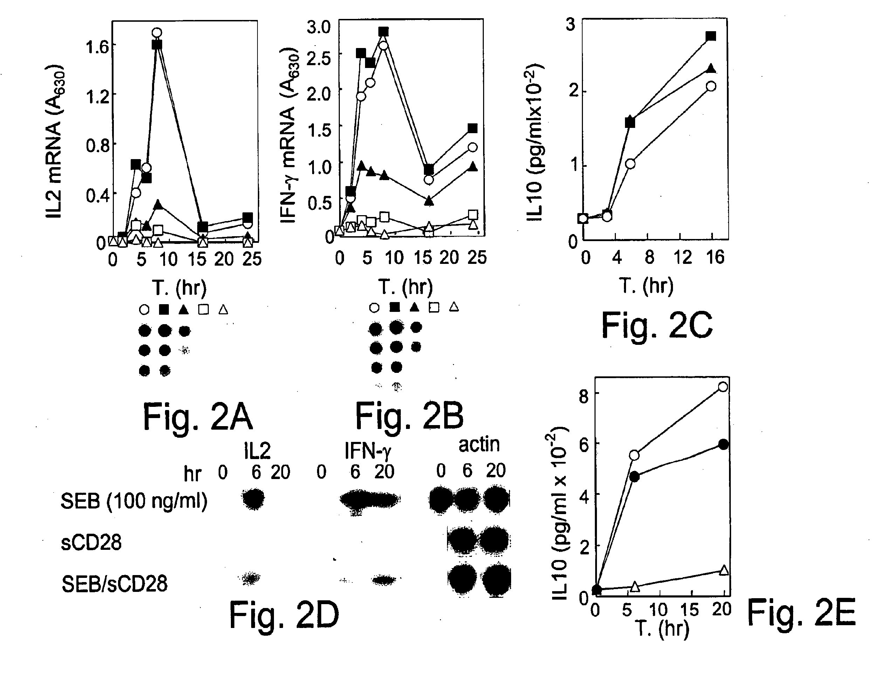

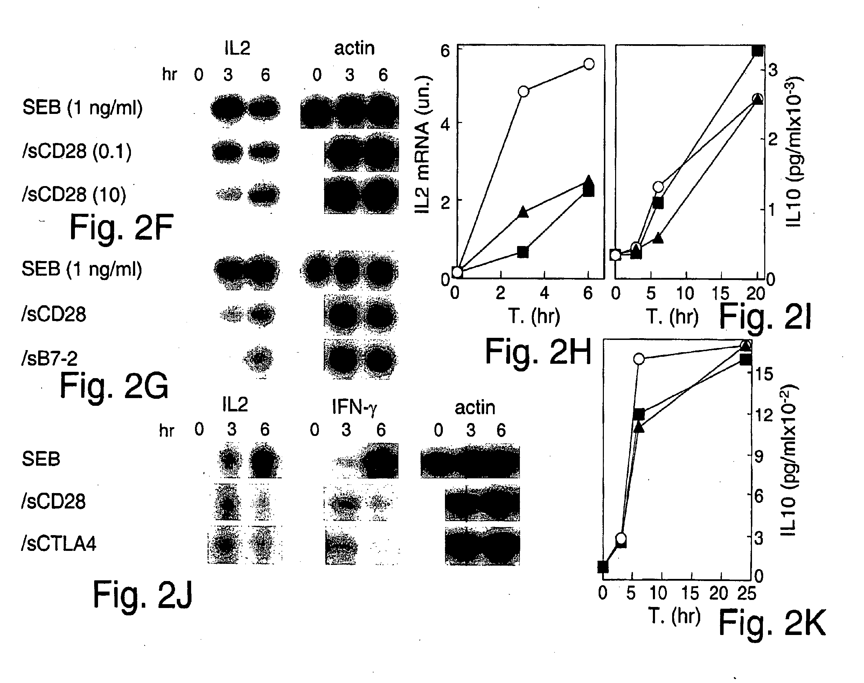

**Figs. 2D-2E** PBMC were induced with SEB (○), 1 µg/ml sCD28 (R&D Systems) (Δ) or both (●); IL2 and TFN-γ mRNA was determined by RNase protection analysis with actin mRNA as loading control, and IL10 by ELISA. (Fig. 2F) PBMC were incubated with 1 ng/ml SEB alone or with sCD28 (in µg/ml); IL2 mRNA was determined. Figs. 2G-2I PBMC were incubated with 1 ng/ml SEB alone (○) or with 100 ng/ml sCD28 (▲) or sB7-2 (■); IL2 mRNA in (Fig. 2G) was quantitated (Fig. 2H) and IL10 was determined.
**Figs 2J-2K** PBMC were induced with SEB alone (○) or with 10 ng/ml sCD28 (A) or 1 µg/ml sCTLA4 (■); IL2 mRNA, IFN-γ mRNA and IL10 were determined. Abbreviations: T (time), hr. (hour), Un, (units), pg/ml (Pico gram/milliliter).

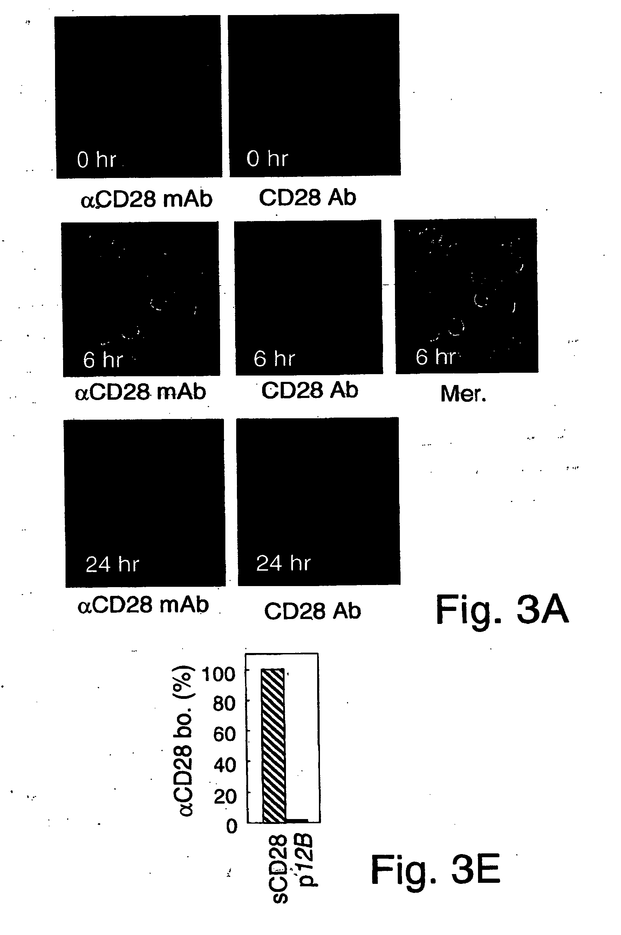

**Figure 3A-3G** *Superantigen Mimetic Peptide p12B Inhibits Signaling Via CD28*
**Fig. 3A** SEB induces a change in cell surface presentation of CD28. CD4 cells were enriched to 90% from PBMC by use of RosetteSep (Stem Cell Technologies), incubated at a density of 4x10⁶ cells/ml with SEB and at times shown stained with αCD28 mAb (R&D Systems)(secondary Ab: Cy-2, green; Jackson Laboratories) or polyclonal CD28 Ab (secondary Ab: Cy-3, red; Jackson Laboratories). Confocal fluorescence microscopy is shown. Mer. (Merge), double staining with both Abs.

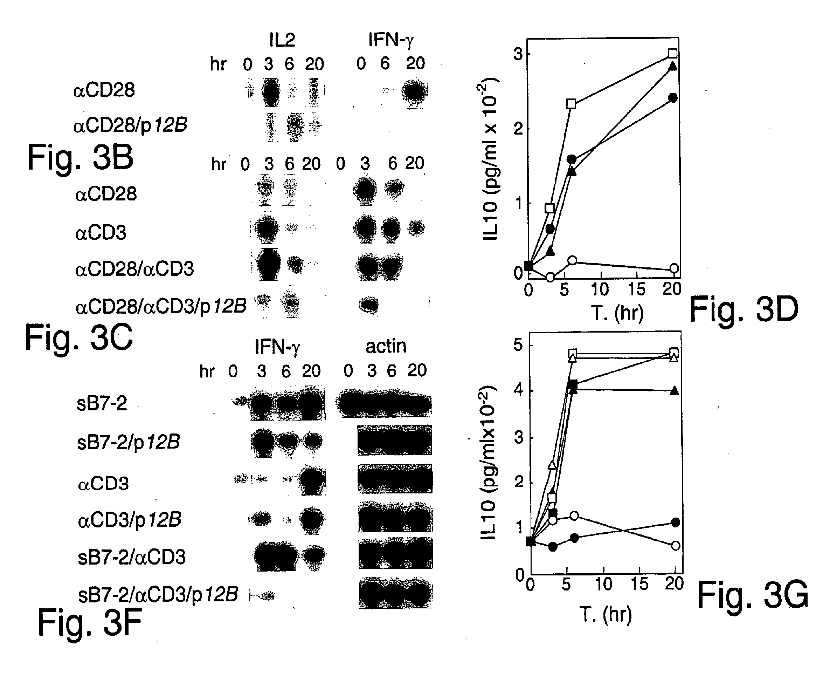

**Figs. 3B-3E** *p12B* inhibits induction of Th1 cytokine mRNA by αCD28 mAb.
**Fig. 3B** PBMC were incubated with 2.5 µg/ml αCD28 in the absence or presence of 10 µg/ml *p12B*; IL2 and IFN-γ mRNA was determined; actin mRNA (not shown) served as loading control. Figs.3C-3E PBMC were incubated with 2.6 µg/ml αCD28 (○) or 0.1 µg/ml αCD3 mAb (R&D Systems)(□) or both (A, ●), in the absence (▲) or presence of 10 µg/ml *p12B* (●). IL2 mRNA, IFN-γ mRNA and IL10 were determined; actin mRNA (not shown) served as loading control. To show that αCD28 does not bind *p12B,* sCD28 or *p12B* was immobilized and binding of αCD28 was assayed by ELISA using alkaline phosphatase-coupled anti-mouse IgG (Jackson Laboratories)(Fig. 3E).
**Figs. 3F,** 3G *p12B* inhibits induction of IFN-γ mRNA by sB7-2/αCD3. PBMC were incubated with 0.1 µg/ml αCD3 (□, ■), 1 µg/ml sB7-2 (○, ●) or both (Δ, ▲), in the absence (□, ○, Δ) or presence of 10 µg/ml *p12B* (□*,* ○, Δ). IFN-γ mRNA, actin mRNA and IL10 were determined. Abbreviations: T (time), hr. (hour), pg/ml (Pico gram/milliliter), mAb (monoclonal antibody), Ab (antibody), Bo. (bound).

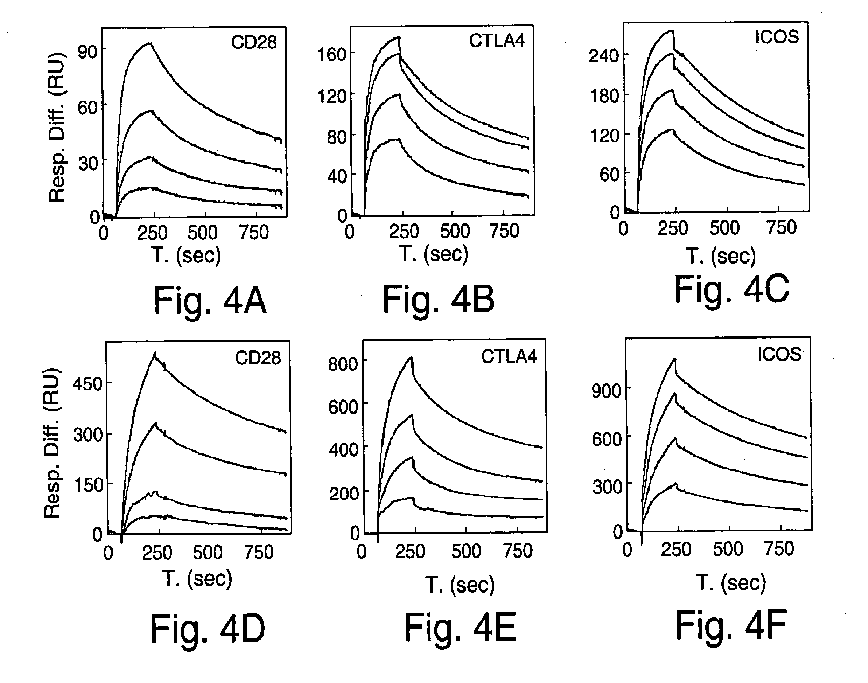

**Figure 4A-4I** *SEB Binds to CD28, CTLA4 and ICOS Through its Antagonist Domain*
**Figs. 4A-4C** Representative profiles of the relative surface plasmon resonance responses for binding of soluble CD28, CTLA4 and ICOS in concentrations ranging from 0.25 µM in twofold increments to immobilized SEB (695 RU) were determined as described in Experimental Procedures.
**Figs. 4D-4F** Representative profiles of the relative surface plasmon resonance responses for binding of soluble CD28, CTLA4 and ICOS in concentrations ranging from 0.126, 0.063 and 0.063 µM, respectively, in twofold increments to immobilized *p12CC* (1,950 RU).

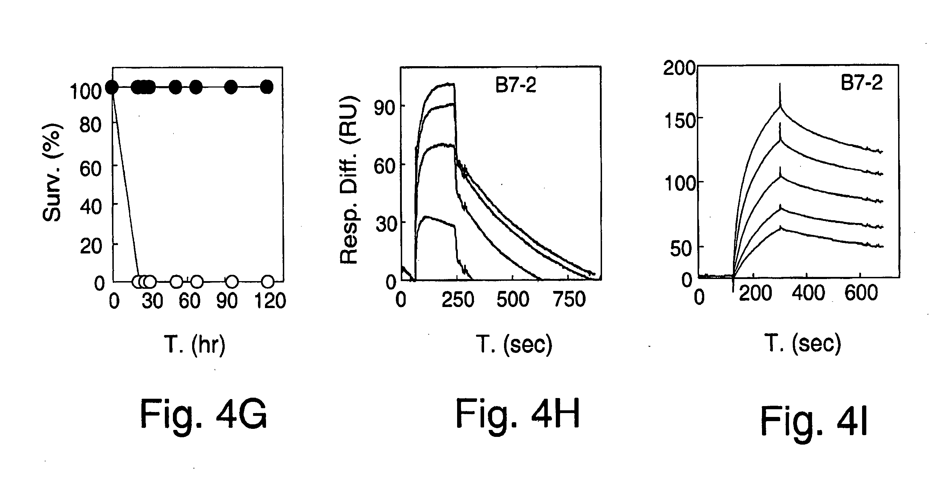

**Fig. 4G** Polyclonal rabbit anti-p*12B* Ab (αp*12B*; Genemed Synthesis) protects mice from lethal SEB challenge. Groups of 5 mice were challenged with 20 µg of SEB directly (○) or 30 min after intraperitoneal injection of 200 µl of undiluted *αp12B* (●). Survival is shown.
**Fig. 4H** Representative profiles of the relative surface plasmon resonance responses for binding of sB7-2 in concentrations ranging from 0.25 µM in twofold increments to immobilized SEB (696 RU).
**Fig. 4I** Representative profiles of the relative surface plasmon resonance responses for binding of sB7-2 in concentrations ranging from 31.25 nM in twofold increments to immobilized sCD28 (3,400 RU). Abbreviations: T (time), hr. (hour), sec (seconds), Resp. diff. (response difference), Surv. (survival).

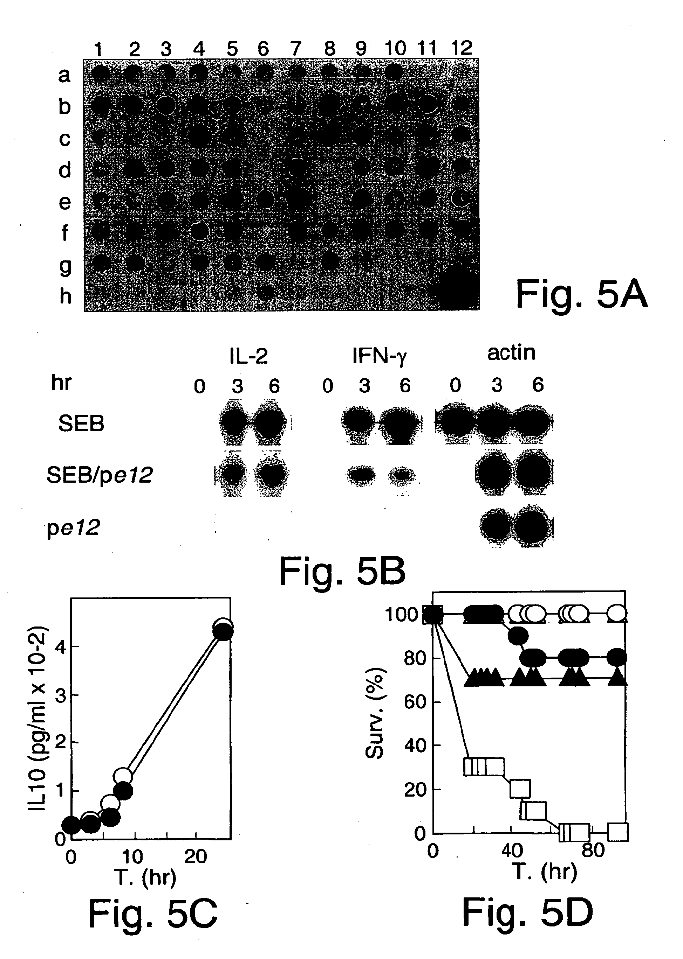

**Figure 5A-5F** *Novel SEB Antagonist Peptides Are Selected by Affinity for CD28*
**Fig. 5A** Screening assay for phages that bind tightly to sCD28. After 4 rounds of panning a random 12-mer phage display library on sCD28 and displacement by SEB, phages were immobilized-on- ECL-plus membranes and binding of sCD28 was detected with HRP- conjugated sCD28 (R&D Systems). Positive control, αCD28 mAb (h12). Negative control, phage lacking insert (h5-h7).
**Figs. 5B, 5C** Antagonist activity of *pe12.* PBMC were induced with 100 ng/ml SEB (○), 1 µg/ml *pe12* (see A) or both (●). IL2, IFN-γ and actin mRNA and IL10 were determined.
**Fig. 5D** p*e12* and pc3 protect mice from killing by SEB. Groups of 10 mice were challenged with 7.5 µg SEB alone (□) or with 0.2 µg pe12
(●) or 0.5 µg pc3 (▲) (from another ECL assay). Controls received 1 µg of p*e12* (○) or *pc3* (Δ) 30 min before injection of D-galactosamine without SEB. Survival was monitored.

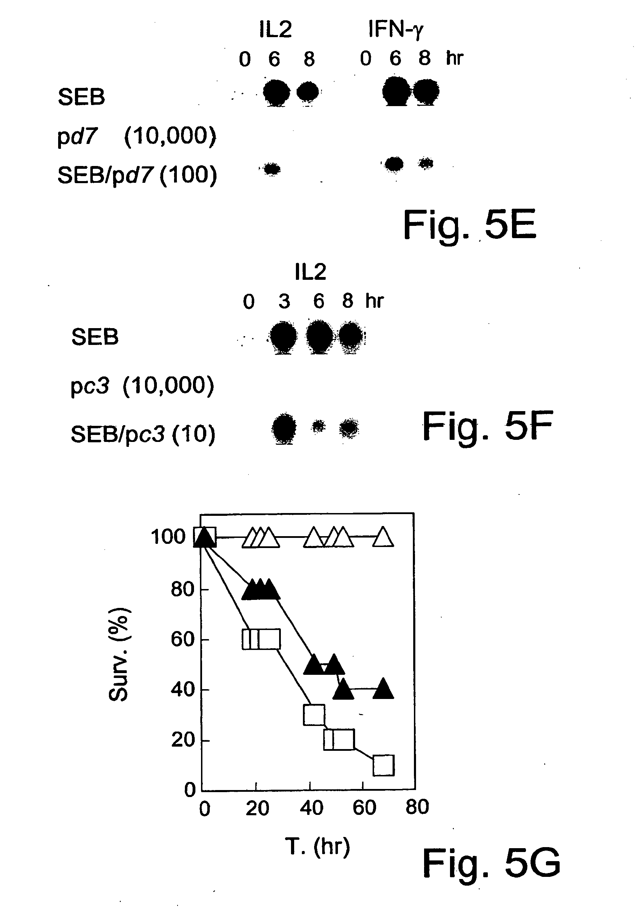

**Fig. 5E** Antagonist activity of *pd7.* PBMC were induced with 100 ng/ml SEB, *pd7* at the indicated concentrations in ng/ml, or both. IL2 and IFN-γ mRNA were determined.
**Fig. 5F** Antagonist activity of *pc3.* PBMC were induced with 100 ng/ml SEB, *pc3* at the indicated concentrations in ng/ml, or both. IL2 mRNA was determined.
**Fig. 5G** *pd7* protects mice from killing by SEB. Groups of 10 mice were challenged with 6 µg SEB alone (□) or with 0.5 µg *pd7* (▲). Controls received 2.6 µg of *pd7* (Δ) 30 min before injection of D-galactosamine without SEB. Survival was monitored.
   Abbreviations: T (time), hr. (hour), pg/ml (Pico gram/milliliter), Surv. (survival).

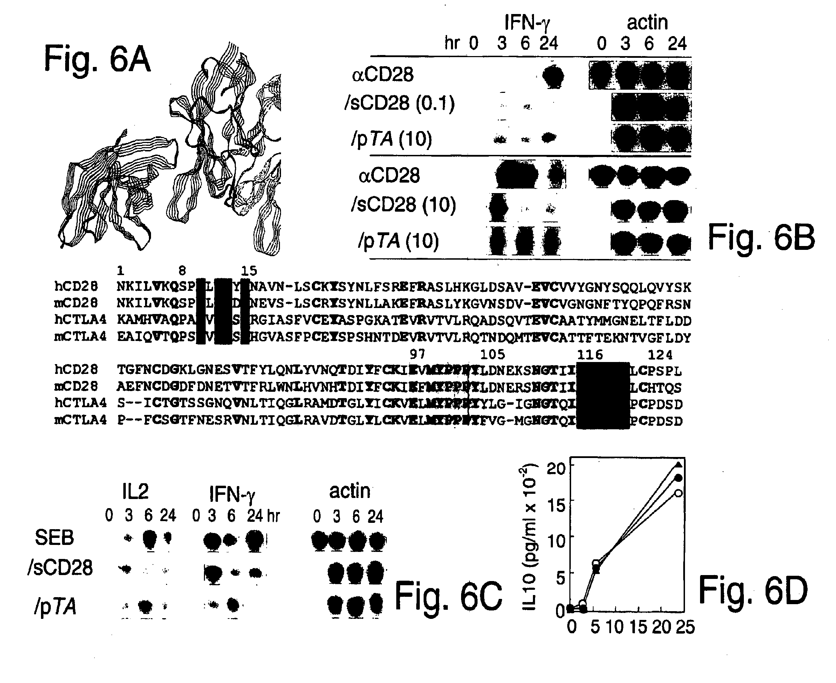

**Figure 6A-6I** *Peptide Mimetics of the Dimer Interface Predicated for CD28 are Superantigen Antagonists*
**Fig. 6A** CTLA4/B7-2 complex and the dimer interface in CTLA4. In ribbon diagram of the CTLA4/B7-2 complex (1I85.pdb; [Schwartz (2001) *ibid*.], generated with RasMol, one B7-2 monomer is shown in magenta, the other in grey and CTLA4 in blue, with MYPPPY (SEQ ID NO: 22) in yellow, YVIDPE (SEQ ID NO: 18) (HVKGKH in CD28, SEQ ID NO: 10) in red, and VVLASS (SEQ ID NO: 23) (MLVAYD (SEQ ID NO: 24) in CD28) in green, as in the sequence alignment of human (h) and murine (m) CD28 and CTLA4 shown below; conserved residues appear in bold face.
**Fig. 6B** Effect of sCD28 and pTA on induction of IFN-γ mRNA by αCD28. Human PBMC were induced with 260 ng/ml αCD28 mAb alone or with sCD28 or *pTA,* in µg/ml. IFN-γ and actin mRNA was determined.
**Figs. 6C-6F** p*TA* antagonizes induction of Th1 cytokine mRNA by SEB. PBMC were induced by SEB alone (○) or with 0.1 µg/ml sCD28 (●) or 10 µg/ml p*TA* (▲). IL2, ITN-γ and actin mRNA, IL10 and IL4 were determined. In a separate experiment (F), *pTA* was added in increasing concentrations (µg/ml); IFN-γ and actin mRNA was determined.

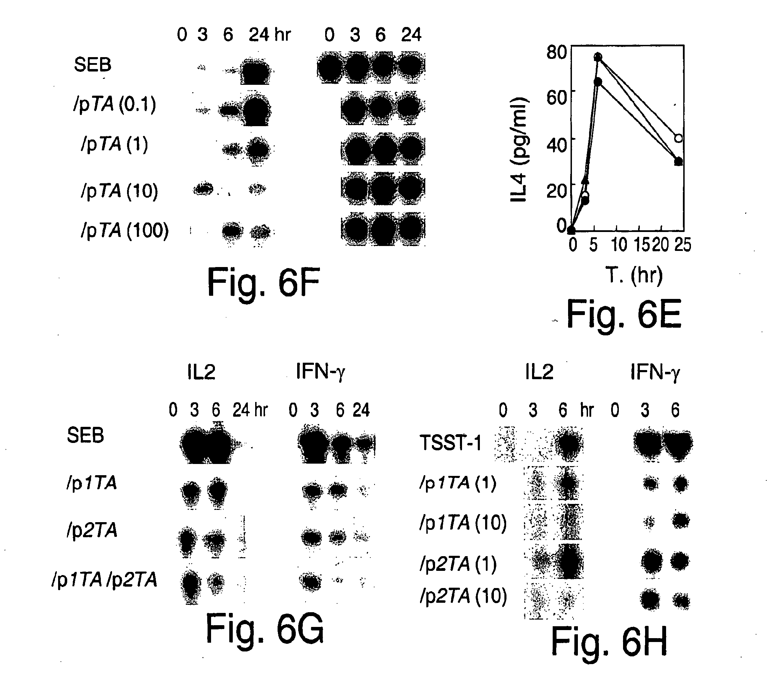

**Figs. 6G, 6H** *p1TA* and *p2TA* antagonize induction of IL2 and IFN-γ mRNA by SEB or TSST-1. (G) PBMC were induced with 100 ng/ml SEB alone or with 0.1·µg/ml *p1TA, p2TA* or both. (H) PBMC were induced with 100 ng/ml TSST-1 (Sigma) alone or with p1TA or *p2TA* as shown, in µg/ml. IL2 and IFN-γ mRNA was determined; actin mRNA (not shown) served as loading control.

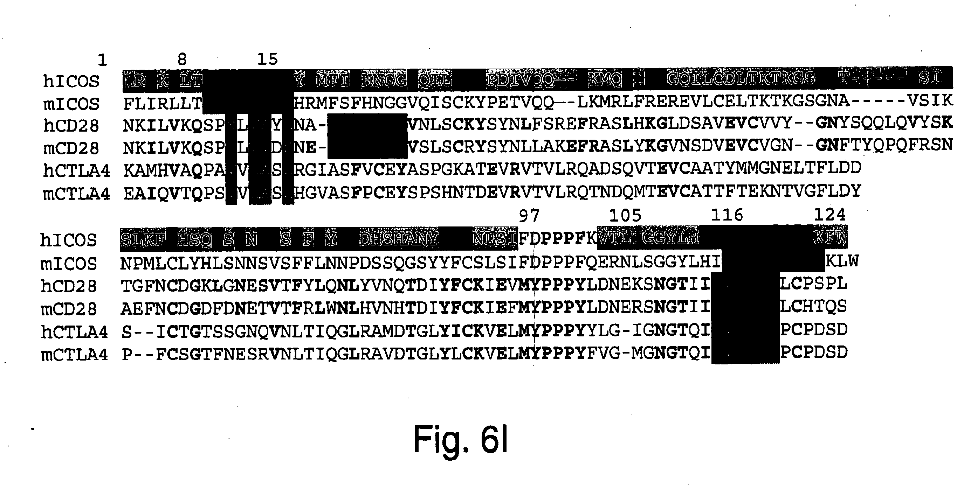

**Fig. 6I** Sequence Alignment of TCOS with CD28 and CTLA4. Amino acid sequences of the extracellular domains of human (h) ICOS (accession number AAH28006), murine (m) ICOS (accession number NP_059508), CD28 and CTLA4 are shown. The CD28 sequence is numbered. Residues conserved between hICOS and hCD28 are shown in dark bluegreen; yellow marks B7 binding site. Conserved residues appear in bold face. A gap in CD28 used for the alignment with ICOS is shown in magenta. Sequences in ICOS colored cyan overlap with the two dimer interface sequences (red and green) in CD28 and CTLA4; the corresponding ICOS peptide *p1TC* aligns with CD28 peptide *p1TA* and CTLA4 peptide *p1TB,* and the corresponding ICOS peptide *p2TC* aligns with CD28 peptide p*2TA* and CTLA4 peptide *p2TB.* Abbreviations: T (time), hr. (hour), pg/ml (Pico gram/milliliter).

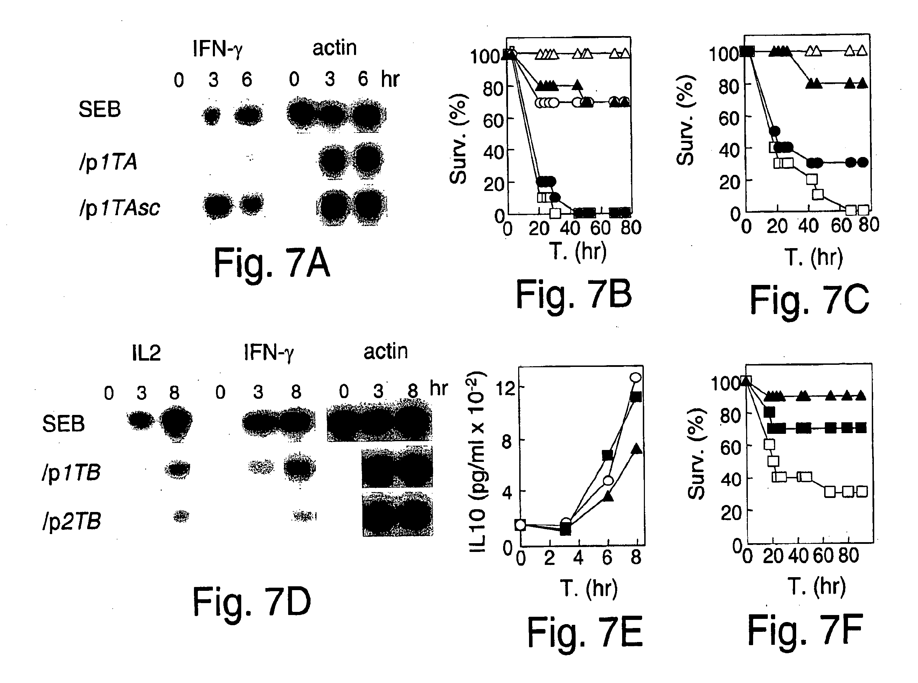

**Figure 7A-7J** *CD28, CTLA4 and ICOS Mimetic Peptides Protect Mice from Lethal Shock*
**Fig. 7A** Antagonist activity *of p1TA* is sequence specific. PBMC were induced with SEB alone or with 1 µg/ml *p1TA* or its scrambled form *p1TAsc* (CHGHLVPHK; also denoted by SEQ ID NO: 4). IFN-γ and actin mRNA was determined.
**Fig. 7B, 7C** CD28 mimetic peptides protect mice from lethal challenge with SEB. Groups of 10 mice were challenged with 6 µg SEB alone (Δ) or with *p1TA* (1 µg)(▲), *p12B* (5 µg)(○) or *p1TAsc* (1 µg)(●)(Fig. 7B), or with 0.2 µg *p2TA* (▲) or its scrambled form *p2TAsc* (ASMDYPVL; also denoted by SEQ ID NO: 5)(●) (Fig. 7C). Controls received 25 µg of *p1TA* (Fig. 7B) or *p2TA* (Fig. 7C) 30 min before injection of D-galactosamine without SEB (Δ).
**Fig. 7D-7F** Antagonist activity of CTLA mimetic peptides. PBMC were induced with 100 ng/ml SEB alone (○) or with 1 µg/ml *p1TB* (A) or *p2TB* (■). IL2, IFN-γ and actin mRNA and IL10 were determined.
Groups of 10 mice were challenged with 6 µg SEB alone (□) or with 0.5 µg *p1TB* (▲) or *p2TB* (■).

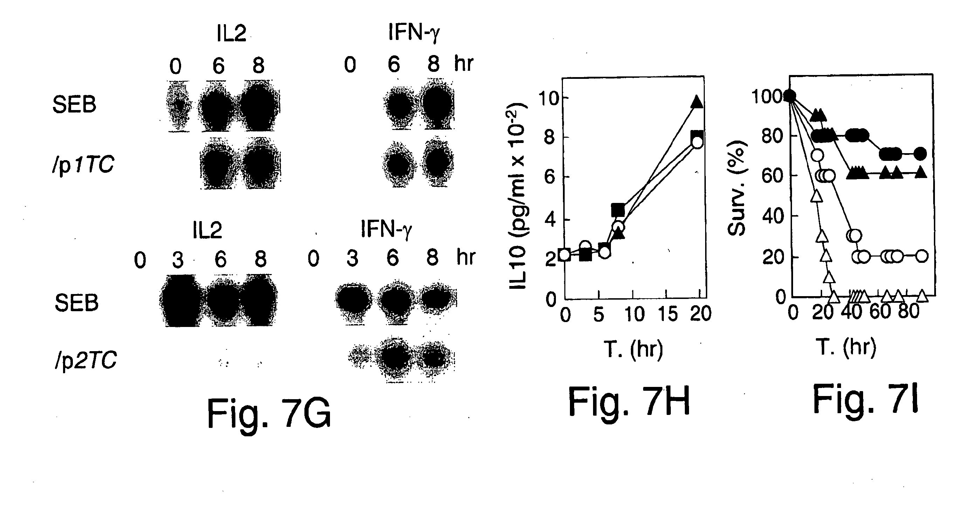

**Fig. 7G-7I** Antagonist activity of ICOS mimetic peptides. PBMC were induced with 100 ng/ml SEB alone (○) or with 1 µg/ml *p1TC* (▲) or 0.1 µg/ml *p2TC* (■). IL2, IFN-γ and actin mRNA and IL10 were determined. Groups of 10 mice were challenged with 5 µg SEB alone
(○) or with 2.6 µg *p1TC* (●) and with 6 µg SEB alone (Δ) or with 0.2 µg *p2TC* (▲).

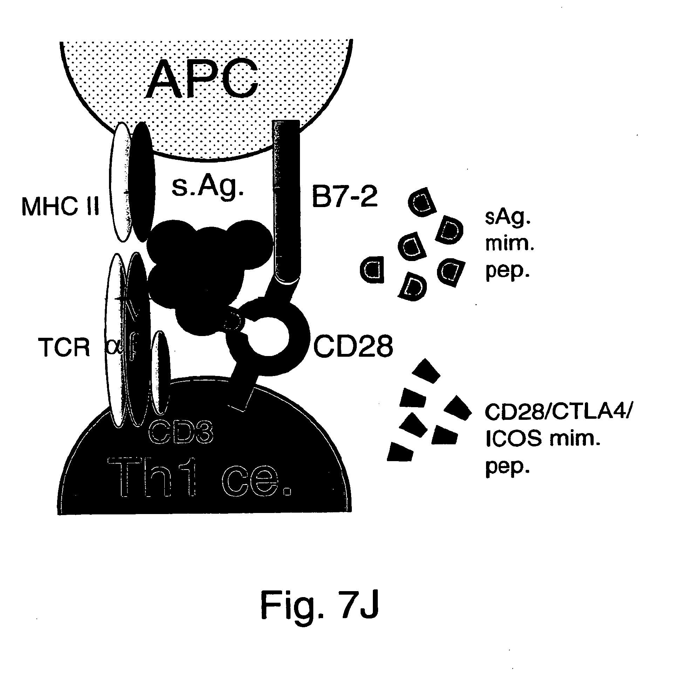

**Fig. 7J** Model for Th1 cell activation by superantigens. Direct binding of superantigen to CD28 is required for activation and can be blocked by peptide mimetics of the contact region in each ligand: the antagonist domain in superantigens and the two rims (red and green) of the predicted dimer interface in CD28.
   Abbreviations: T (time), hr. (hour), pg/ml (Pico gram/milliliter), Surv. (survival), sAg (superantigen), mim. Pep. (mimetic peptide) Ce. (cell), APC (antigen presenting cell).

### Detailed Description of the Invention

It is known that CD28 acts as a costimulatory ligand for conventional antigens. In the present study, the-inventors show that, in order to deliver the signal for Th1 activation, a superantigen must bind directly to CD28. Thus, as demonstrated by the following Examples, CD28 serves as the third superantigen receptor, in addition to the MHC class II molecule and TOR.

Figure 6A shows that the binding site for superantigens in CD28 is the bipartite dimer interface predicted from alignment with CTLA4. The superantigen domain that engages CD28 is remote from the binding sites for both MHC class II molecule and TCR, leaving it accessible for interaction with the CD28 family molecules (Figure 1E). This domain contains at least part of a β-strand-hinge-α-helix motif, which is conserved among the bacterial superantigens [Arad (2000) *ibid*.].

As shown by the inventors, SEB induces a vigorous and concomitant expression of Th1 and Th2 cytokine genes, but only induction of the Th1 response is dependent on CD28 signaling. Thus, it seems that superantigens co-opt a costimulatory ligand of the host for use as their obligatory receptor, binding it directly. This strategy may be employed more widely by pathogens. Toll-like receptors recognize microbial components and thereby activate the innate immune response [reviewed by Akira, S. et al. Nat. Immunol. 2:675-680 (2001); Janeway, C.A. Jr. and Medzhitov, R. Annu. Rev. Immunol. 20:197-216 (2002)]. In the present study, the inventors show that CD28 acts not only as a costimulatory ligand, but also as a sensor of bacterial superantigens.

The present invention provides independent lines of evidence to support the concept that there is direct binding of superantigens to CD28. SPR (Surface Plasmon Resonance) equilibrium binding analysis showed that CD28 binds directly to SEB, with an affinity of 28 nM (Figure 4A). Soluble CD28 blocked induction of Th1 cytokine mRNA by SEB (Figure 2D). Superantigen mimetic peptide *p12B,* homologous to the β-strand-hinge-α-helix 'antagonist domain' in SEB, and CD28 mimetic peptides *p1TA* and *p2TA* (SEQ ID NO: 9 and 12, respectively), corresponding to two noncontiguous sequences that form the predicted homodimerization interface in CD28, each blocked superantigen-mediated induction of IL2 and IFN-γ mRNA in human PBMC (Figures 1D, 6G, and 6H) and protected mice from lethal challenge with SEB (Figures 7B and 7C).

Novel peptide antagonists of SEB, effective in vivo, were selected from a random phage display library solely by their affinity for the superantigen binding site in CD28 (Figure 5). Moreover, *p12B* blocked the induction of Th1 cytokine mRNA by αCD28, alone or in combination with αCD3 (Figures 3B and 3C). Thus, the peptide blocked CD28-mediated Th1 activation even in the absence of superantigen, suggesting that it binds to CD28. Indeed, in SPR kinetics, CD28 bound the *p12* peptide with an affinity similar to that for SEB (Figure 4D). Thus, SEB uses its antagonist domain to bind CD28.

Thus, in a first aspect, the present invention relates to an isolated and purified peptide comprising an amino acid sequence derived from a dimer interface of a T cell co-stimulatory pathway member. Hence, the invention provides an isolated and purified peptide consisting of:
(a) a peptide comprising an amino acid sequence that is a dimer interface of a T cell costimulatory pathway member selected from the dimer interface within the human CD28, the human CTLA4, the human ICOS and the human PD-1, said amino acid sequence being selected from amino acid residues 10-15 or 116-121 of SEQ ID NO: 19, amino acid residues 10-15 or 115-120 of SEQ ID NO: 20, amino acid residues 10-15 or 119-124 of SEQ ID NO: 21, and amino acid residues 8-13 or 110-116 of SEQ ID NO: 59, where the peptide:
   (i) is a human CD28 dimer interface peptide consisting of the amino acid sequence HVKGKHLCP as denoted by SEQ ID NO: 9 or the amino acid sequence SPMLVAYD as denoted by SEQ ID NO: 12;
   (ii) is a human CTLA4 dimer interface peptide consisting of the amino acid sequence YVIDPEPCP as denoted by SEQ ID NO: 14 or the amino acid sequence PAWLASS as denoted by SEQ ID NO: 15;
   (iii) is a human ICOS dimer interface peptide consisting of the amino acid sequence YESQLCCQL as denoted by SEQ ID NO: 16 or the amino acid sequence GEINGSAN as denoted by SEQ ID NO: 17; or
   (iv) is a human PD-1 dimer interface peptide consisting of the amino acid sequence RVTERRAEV as denoted by SEQ ID NO: 57 or the amino acid sequence PALLWTE as denoted by SEQ ID NO: 58;
(b) a peptide which is at least 80% homologous to the human CD28 peptide of SEQ ID No: 9 or 12, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a peptide of (a), (b) or (c) that is extended at the N terminus and/or the C terminus:
   (i) by a lauryl cysteine at the N terminus and a cysteine at the C terminus; or
   (ii) by an organic moiety that is not a naturally occurring or synthetic amino acid residue; or
   (iii) by identical hydrophobic amino acid residue(s) which may be naturally occurring or synthetic amino acid residues; or
   (iv) by a lysyl-palmitoyl tail, wherein said tail is at the N terminus, wherein the resultant peptide of (c) maintains the ability to inhibit the interaction between a T cell costimulatory pathway member and a superantigen;
(e) a dimer or multimer of (a), (b), (c), or (d) wherein the resultant peptide of (e) maintains the ability to inhibit the interaction between a T Cell costimulatory pathway member and a superantigen.

The T cell co-stimulatory pathway is the CD28/B7 pathway. Accordingly, the CD28/B7 pathway member may be any one of CD28, CTLA-4, ICOS and PD-1.

According to one specifically preferred embodiment, the pathway member may be the CD28 molecule, and the dimer interface within CD28 comprises amino acid residues 10-15 or 116-121 of the human CD28 amino acid sequence, as denoted by SEQ ID NO : 19, where the isolated and purified peptide consists of:
(a) a peptide consisting of an amino acid sequence that is selected from the amino acid sequence HVKGKHLCP as denoted by SEQ ID NO: 9 and the amino acid sequence SPMLVAYD as denoted by SEQ ID NO: 12;
(b) a peptide which is at least 80% homologous to the peptide of (a),
   wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a peptide of (a), (b) or (c) that is extended at the N terminus and/or the C terminus:
   (v) by a lauryl cysteine at the N terminus and a cysteine at the C terminus; or
   (vi) by an organic moiety that is not a naturally occurring or synthetic amino acid residue; or
   (vii) by identical hydrophobic amino acid residue(s) which may be naturally occurring or synthetic amino acid residues; or
   (viii) by a lysyl-palmitoyl tail, wherein said tail is at the N terminus, wherein the resultant peptide of (c) maintains the ability to inhibit the interaction between a T cell costimulatory pathway member and a superantigen; or
(e) a dimer or multimer of (a), (b), (c) or (d), wherein the resultant peptide of (e) maintains the ability to inhibit the interaction between a T Cell costimulatory pathway member and a superantigen.

According to another preferred embodiment, the pathway member may be the CTLA-4 molecule, and the dimer interface within CTLA-4 comprises amino acid residues 10-15 or 115-120 of the human CTLA-4 amino acid sequence, as denoted by SEQ ID NO: 20, where the isolated and purified peptide of the invention consists of:
(a) a peptide consisting of an amino acid sequence that is selected from one of the amino acid sequence YVIDPEPCP as denoted by SEQ ID NO: 14 and the amino acid sequence PAWLASS as denoted by SEQ ID NO: 15;
(b) a peptide of (a) that is extended at the N terminus and/or the C terminus:
   (i) by lauryl cysteine at the N terminus and a cysteine at the C terminus; or
   (ii) by an organic moiety that is not a naturally occurring or synthetic amino acid residue; or
   (iii) by identical hydrophobic amino acid residue(s) which may be naturally occurring or synthetic amino acid residues; or
   (iv) by a lysyl-palmitoyl tail, wherein said tail is at the N terminus, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a dimer or multimer of (a), (b) or (c), wherein the resultant peptide of (e) maintains the ability to inhibit the interaction between a T Cell costimulatory pathway member and a superantigen.

In yet another embodiment, the pathway member may be the ICOS molecule and the dimer interface within ICOS comprises all or part of amino acid residues 10-15 or 119-124 of the human ICOS amino acid sequence as denoted by SEQ ID NO: 21, where the isolated and purified peptide of the invention consists of:
(a) peptide consisting of an amino acid sequence that is selected from the amino acid sequence YESQLCCQL as denoted by SEQ ID NO: 16 and the amino acid sequence GEINGSAN as denoted by SEQ ID NO: 17;
(b) a peptide of (a) or (b) that is extended at the N terminus and/or the C terminus:
   (i) by lauryl cysteine at the N terminus and a cysteine at the C terminus; or
   (ii) by an organic moiety that is not a naturally occurring or synthetic amino acid residue; or
   (iii) by identical hydrophobic amino acid residue(s) which may be naturally occurring or synthetic amino acid residues; or
   (iv) by a lysyl-palmitoyl tail, wherein said tail is at the N terminus, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a dimer or multimer of (a), (b) or (c), wherein the resultant peptide of (d) maintains the ability to inhibit the interaction between a T Cell costimulatory pathway member and a superantigen.

Still further, the pathway member may be the PD-1 molecule. Although PD-1 is known as a monomer, the domains in PD-1 which overlap with the dimer interface of CTLA4 are folded similarly.

The peptide of the invention is an immunomodulatory peptide capable of modulating a T cell costimulatory pathway.

The peptide of the invention may comprise an amino acid sequence from the dimer interface of a T cell co-stimulatory pathway member, which is a CD28/B7 family member.

More specifically, the peptide of the invention comprises an amino acid sequence from the dimer interface of any one of CD28, CTLA-4, ICOS and PD-1.

The structure of CD28 has not been resolved but likely is similar to that of CTLA4 (Figure 6A) [Schwartz (2001) *ibid.;* Luhder (2003) *ibid*.]. CD28 and CTLA4 show overall sequence homology, with identity in their B7 binding domains, yet differ completely in two sequences that create the dimer interface in CTLA4, most likely to prevent heterodimer formation [Schwartz (2001) *ibid.*; Collins (2002) *ibid*.]. In the folded CTLA4 protein, these remote sequences are juxtaposed (Figure 6A). Remarkably, as shown by the Examples, the corresponding CD28 mimetic peptides p*1TA* and *p2TA* were protective in vivo when present in about equimolar ratio to SEB. These results provide powerful evidence that the inventors have identified the correct superantigen binding site in CD28 and that this site is a composite formed from sequences found in *p1TA* and *p2TA* (SEQ ID NO: 9 and 12, respectively). Peptides deriving from each rim of the dimer interface, predicted for CD28 solely on the basis of its alignment with CTLA4, blocked the action of superantigens as widely different as SEB and TSST-1, showing that both use CD28 as a receptor.

CD28 belongs to a triad of costimulatory ligands: CD28, CTLA4, and ICOS. These proteins show up to 33% identity and are encoded by tightly clustered genes [Carreno and Collins (2002) *ibid.*]. The inventors have shown that SEB binds directly to each one of them, with a similar affinity (Figures 4A, 4B, and 4C). Binding occurs at the dimer interface of each costimulatory receptor, which is unique. Peptides deriving from either rim of the bipartite dimer interface in CTLA4 or that predicted for ICOS by alignment (Fig. 6I) are strong superantigen antagonists that, like CD28 mimetic peptides *p1TA* and *p2TA,* protect mice from lethal challenge with SEB at a very low molar ratio to the toxin (Figure 7). Evidently, the mode of action of these antagonists is to compete with CD28 for its binding site in superantigens, the antagonist domain, since CD28, CTLA44 and ICOS each bind directly to this domain, with substantial affinity (Figures 4D, 4E, and 4F).

The amino acid sequences of the bipartite dimer interface in CTLA4 and those predicted for CD28 [Schwartz (2001) *ibid.]* and ICOS (present study) lack any homology, likely to prevent heterodimer formation. The functional analyses of the present invention show that each uses this interface to bind the superantigen. Apparently, though distinct in sequence, the three dimer interfaces are folded similarly. The antagonist domain in superantigens likewise shows spatial conservation despite sequence heterogeneity [Arad (2000) *ibid.].* Thus, in both sets of ligands, the receptor triad and superantigens, structural features generate the contact surface.

The structure of the costimulatory receptor programmed death-1 (PD-1) has been resolved and can be superimposed to the structure of CTLA4, allowing the alignment of their amino acid sequences [Zhang (2004) *ibid.].* The sequences YVIDPEPCP *(p1TB,* SEQ ID NO: 14) and PAVVLASS (*p2TB,* SEQ ID NO: 15), related to the dimer interface in CTLA4, are aligned with the PD-1 sequences RVTERRAEV (*p1TD,* SEQ ID NO: 57) and PALLVVTE (*p2TD,* SEQ ID NO: 58), respectively. Although PD-1 is a monomer, the domains in PD-1 overlapping with *p1TD* and *p2TD* are folded similarly to those in CTLA4 overlapping with *p1TB* and *p2TB* [Zhang (2004) *ibid.].* Therefore, peptides *p1TD* and *p2TD* derived from these two noncontiguous domains in PD-1 are potential competitors for the binding site for CD28 in a superantigen, which will result in the inhibition of superantigen action.

In a specifically preferred embodiment, the peptide of the invention is derived from a dimer interface within the CD28 molecule which comprises residues 10-15 or 116-121 of the human CD28 amino acid sequence as denoted by SEQ ID NO: 12. It should be noted that the human CD28 amino acid sequence shown by Fig. 6A and denoted by SEQ ID NO: 19 represents only the extracellular part of the human CD28 sequence according to GenBank Accession No. P10747. As shown by Fig. 6A, the predicted CD28 dimer interface corresponds to the CTLA-4 dimer interface, in position but not in sequence (positions 10-15 and 115-120 of CTLA-4). It should be further noted that the human CTLA-4 amino acid sequence shown by Fig. 6A represents only the extracellular part of the human CTLA-4 sequence according to GenBank Accession No. AAO17066.

A specific preferred peptide of the invention is designated *p1TA* and has the amino acid sequence HVKGKHLCP as denoted by SEQ ID NO: 9.

Another specific preferred peptide of the invention is designated *p2TA* and has the amino acid sequence SPMLVAYD, as denoted by SEQ ID NO: 12.

Alternatively, the peptide of the invention may comprise an amino acid sequence from the dimer interface within the CTLA-4 molecule, which dimer interface comprises amino acid residues 10-15 or 115-120 of the human CTLA-4 amino acid sequence as denoted by SEQ ID NO: 20, where the peptide consists of:
(a) a peptide consisting of an amino acid sequence that is selected from one of the amino acid sequence YVIDPEPCP as denoted by SEQ ID NO: 14 and the amino acid sequence PAVVLASS as denoted by SEQ ID NO: 15;
(b) a peptide of (a) that is extended at the N terminus and/or the C terminus:
   (i) by lauryl cysteine at the N terminus and a cysteine at the C terminus; or
   (ii) by an organic moiety that is not a naturally occurring or synthetic amino acid residue; or
   (iii) by identical hydrophobic amino acid residue(s) which may be naturally occurring or synthetic amino acid residues; or
   (iv) by a lysyl-palmitoyl tail, wherein said tail is at the N terminus, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a dimer or multimer of (a), (b) or (c), wherein the resultant peptide of (e) maintains the ability to inhibit the interaction between a T Cell costimulatory pathway member and a superantigen.

More specifically, such peptide consists of the amino acid sequence YVIDPEPCP as denoted by SEQ ID NO: 14 or the amino acid sequence PVVLASS, as denoted by SEQ ID NO: 15.

Accordingly, one specific preferred peptide is designated *p1TB* and has the amino acid sequence YVIDPEPCP, as denoted by SEQ ID NO: 14.

Another preferred specific peptide is designated *p2TB* and has the amino acid sequence PAWASS as denoted by SEQ ID NO: 15.

In yet another alternative, the peptide of the invention may comprise an amino acid sequence from the dimer interface within the ICOS molecule, which dimer interface comprises amino acid residues 10-15 or 119-124 of the human ICOS amino acid sequence as denoted by SEQ ID NO: 21, where the peptide consists of:
(a) peptide consisting of an amino acid sequence that is selected from the amino acid sequence YESQLCCQL as denoted by SEQ ID NO: 16 and the amino acid sequence GEINGSAN as denoted by SEQ ID NO: 17;
(b) a peptide of (a) or (b) that is extended at the N terminus and/or the C terminus:
   (i) by lauryl cysteine at the N terminus and a cysteine at the C terminus; or
   (ii) by an organic moiety that is not a naturally occurring or synthetic amino acid residue; or
   (iii) by identical hydrophobic amino acid residue(s) which may be naturally occurring or synthetic amino acid residues; or
   (iv) by a lysyl-palmitoyl tail, wherein said tail is at the N terminus, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a dimer or multimer of (a), (b) or (c), wherein the resultant peptide of (d) maintains the ability to inhibit the interaction between a T Cell costimulatory pathway member and a superantigen.

More specifically, the peptide of the invention may consist of the amino acid sequence YESQLCCQL as denoted by SEQ ID NO: 16 or the amino acid sequence GEINGSAN, as denoted by SEQ ID NO: 17.

One specific example peptide is designated *p1TC* and has the amino acid sequence YESQLCCQL, as denoted by SEQ ID NO: 16.

Another specific example is a peptide designated *p2TC* which has the amino acid sequence GEINGSAN, as denoted by SEQ ID NO: 17.

According to another preferred embodiment, the peptide of the invention comprises an amino acid sequence derived from domains in the PD-1 molecule that correspond to the dimer interface in CTLA4, comprising amino acid residues 8-13 or 110-116 [Zhang (2004) *ibid.*] of the human PD-1 sequence as denoted by SEQ ID NO: 59, where the peptide consists of:
(a) a peptide consisting of an amino acid sequence that is selected from the amino acid sequence RVTERRAEV as denoted by SEQ ID NO: 57 and the amino acid sequence PALLVVTE as denoted by SEQ ID NO: 58;
(b) a peptide of (a) that is extended at the N terminus and/or the C terminus:
   (i) by lauryl cysteine at the N terminus and a cysteine at the Cterminus; or
   (ii) by an organic moiety that is not a naturally occurring or synthetic amino acid residue; or
   (iii) by identical hydrophobic amino acid residue(s) which may be naturally occurring or synthetic amino acid residues; or
   (iv) by a lysyl-palmitoyl tail, wherein said tail is at the N terminus, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a dimer or multimer of (a), (b) or (c), wherein the resultant peptide of (c) maintains the ability to inhibit the interaction between a T Cell costimulatory pathway member and a superantigen.

More specifically, the peptide of the invention may consist of an amino acid sequence - derived from any one of the amino acid sequences RVTERRAEV (as denoted by SEQ ID NO: 57) and PALLVVTE (as denoted by SEQ ID NO: 58).

A specific preferred peptide of the invention is designated *plTD* and has the amino acid sequence RVTERRAEV, as denoted by SEQ ID NO: 57.

Another specific preferred peptide of the invention is designated *p2TD* and has the amino acid sequence PALLWTE, as denoted by SEQ ID NO: 58.

The homology or similarity between any peptide of the present invention and the corresponding dimer interface within the CD28 molecule may range between 80% to 100% homology, preferably, 80% to 90% homology.

A peptide derived from the spatially conserved domain of a pyrogenic exotoxin which forms therein a central turn starting within a β-strand 7 and connecting the β-strand 7, via short β-strand 8, to an α-helix 4, and ending within α-helix 4, based on the domain numbering of SEB, for example *p12A* and *p12B* (SEQ ID NO: 1 and 3, respectively) specifically binds to the dimer interface of the CD28 family molecules, such peptides are out of the ambit of the application.

Therefore, according to a preferred embodiment the invention relates to peptides which bind to the dimer interface of all three members of the CD28 family, CD28, CTLA-4 and ICOS, as well as to PD-1, provided that said peptide is not derived from the spatially conserved domain of a pyrogenic exotoxin which forms therein a central turn starting within a (β-strand 7 and connecting the β-strand 7, via short β-strand 8, to an α-helix 4, and ending within α-helix 4, based on the domain numbering of SEB [Arad (2000) *ibid.]*

As described by Example 8 and Figure 5A, the inventors have performed screening of phage display library on immobilized sCD28, which comprises the dimer interface of CD28 and displaced bound phages with SEB. In this screening different peptides were isolated and further analyzed for their antagonist activity. Therefore, also referred to are peptides which comprise an amino acid sequence as denoted by anyone of SEQ ID NO: 6,7 and 8, 25,26, 27, 28,29, 30,31, 32,33, 34,35, 36,37, 38,39,40,41,42,43,44,45,46,47, 48, 49,50, 51,52, 53, 54,55 and 56.

Also referred to is the peptide designated *pe12* which has the amino acid sequence SHFTHNRHGHST, as denoted by SEQ ID NO: 6.

Another specific peptide referred to is designated *pd7.* This peptide has the amino acid sequence WHAHPHKKPVVA, as denoted by SEQ ID NO: 7.

Also referred to is the peptide designated *pc3* which and has the amino acid sequence FHKHKNPGSPII, as denoted by SEQ ID NO: 8.

The peptides of the invention inhibit the direct interaction between a T cell costimulatory pathway member, preferably, the CD28 molecule and a pyrogenic exotoxin. Therefore, these peptides serve as antagonists of toxin-mediated activation of T lymphocytes, and protect against toxic shock induced by a pyrogenic exotoxin or by a mixture of pyrogenic exotoxins.

It should be appreciated that by the term "insertions", as used herein it is meant any addition of amino acid residues to the peptides of the invention, of 1 amino acid residue.

Also referred to are longer peptides which comprise part or all of the amino acid sequence of the peptides referred to, or in which the basic peptidic sequence of any of the peptides referred to is repeated from about 2 to about 100 times.

The lack of structure of linear peptides renders them vulnerable to proteases in human serum and acts to reduce their affinity for target sites, because only few of the possible conformations may be active. Therefore, it is desirable to optimize antagonist peptide structure, for example by creating different derivatives of the various peptides.

In order to improve peptide structure, the peptides of the invention can be coupled through their N-terminus to a lauryl-cysteine (LC) residue and/or through their C-terminus to a cysteine (C) residue, or to other residue/s suitable for linking the peptide to adjuvant/s for immunization, as will be described in more detail hereafter.

The peptides of the invention may all be positively charged, negatively charged or neutral. In addition, they may be in the form of a dimer, a multimer or in a constrained conformation, which can be attained by internal bridges, short-range cyclizations, extension or other chemical modifications.

Further, the peptides of the invention may be extended at the N-terminus and/or C-terminus thereof with identical or different hydrophobic amino acid residue/s which may be naturally occurring or synthetic amino acid residue/s. A preferred synthetic amino acid residue is D-alanine.

An additional example for such an extension may be provided by peptides extended both at the N-terminus and/or C-terminus thereof with a cysteine residue. Naturally, such an extension may lead to a constrained conformation due to Cys-Cys cyclization resulting from the formation of a disulfide bond.

Another example may be the incorporation of an *N*-terminal lysyl-palmitoyl tail, the lysine serving as sinker and the palmitic acid as a hydrophobic anchor.

In addition, the peptides may be extended by aromatic amino acid residue/s, which may be naturally occurring or synthetic amino acid residue/s. A preferred aromatic amino acid residue may be tryptophan.

Nonetheless, according to the invention, the peptides of the invention may be extended at the *N*-terminus and/or C-terminus thereof with various identical or different organic moieties which are not a naturally occurring or synthetic amino acids. As an example for such extension, the peptide may be extended at the N-terminus and/or *C-*terminus thereof with an *N*-acetyl group.

For every single peptide sequence used by the invention and disclosed herein, this invention includes the corresponding- retro-inverso sequence wherein the direction of the peptide chain has been inverted and wherein all the amino acids belong to the D-series.

According to a second aspect, the invention relates to a composition comprising as an active ingredient a purified peptide of the invention or any combination thereof and optionally further comprises pharmaceutically acceptable carrier, diluent, adjuvant and/or excipient.

The invention further provides a composition according to the invention, for use in a method of treatment of an immune disorder related to an imbalance in the Th1-Th2 response in a subject in need thereof, wherein said immune disorder is any one of an autoimmune disease, malignant and non-malignant proliferative disorder, graft rejection pathology, and a disorder induced by a pyrogenic exotoxin or by a mixture of at least two pyrogenic exotoxins selected from toxic shock and incapacitation or for use in a method of preventing death induced by a pyrogenic exotoxin or by a mixture of at least two pyrogenic exotoxins.

The compositions of the invention may also comprise additional active agents, e. g. protease inhibitors.

More specifically, immune disorders related to an imbalance in the Th1-Th2 response immune-related disorder may be for example, an autoimmune disease, (for example, multiple sclerosis (MS), Type-1 diabetes, lupus, Graves disease and thyroiditis), malignant and non- malignant proliferative disorders, graft rejection pathology and graft versus host disease, and disorders induced by a pyrogenic exotoxin or by a mixture of at least two pyrogenic exotoxins selected from toxic shock, incapacitation and death, septic shock and severe sepsis.

According to a preferred embodiment, the composition of the invention protects against toxic shock induced by a pyrogenic exotoxin or by a mixture of pyrogenic exotoxins. The composition of the invention comprises as an active ingredient any of the purified immunomodulatory peptides of the invention or any combination thereof in an amount effective to inhibit exotoxin-induced expression of an RNA encoded by the IL2 and/or IFN-γ genes, and optionally further comprises pharmaceutically acceptable carrier, diluent, adjuvant and/or excipient.

In one specifically preferred embodiment, such composition may comprise as an active ingredient a peptide selected from the group consisting of p*1TA* (as denoted by SEQ ID NO: 9), p*2TA* (as denoted by SEQ ID NO: 12), p*1TB* (as denoted by SEQ ID NO: 14), p*2TB* (as denoted by SEQ ID NO: 15), p*1TC* (as denoted by SEQ ID NO: 16), p*2TC* (as denoted by SEQ ID NO: 17), p1TD (as denoted by SEQ ID NO: 57), p*2TD* (as denoted by SEQ ID NO: 58) and any combination thereof.

Still further, the invention relates to a composition for inhibiting the direct interaction between a superantigen and a superantigen binding site in any one of CD28, CTLA4, ICOS and PD-1. This composition comprises as active ingredient an isolated and purified peptide, in an amount effective to inhibit said interaction.

The pharmaceutical composition of the invention can be administered and dosed in accordance with good medical practice. Administration. may be carried out in various ways, including intravenous, intramuscular or subcutaneuos injection. However, other methods of administration such as oral, rectal and intranasal administration are also possible.

The composition of the invention may comprise the active substance in free form and be administered directly to the subject to be treated. Alternatively, depending on the size of the active molecule, it may be desirable to conjugate it to a carrier prior to administration. Therapeutic formulations may be administered in any conventional dosage formulation. Formulations typically comprise at least one active ingredient, as defined above, together with one or more acceptable carriers thereof.

Each carrier should be both pharmaceutically and physiologically acceptable in the sense of being compatible with the other ingredients and not injurious to the patient. Formulations include those suitable for oral, rectal, nasal, or parenteral (including subcutaneous, intramuscular, intraperitoneal (IP), intravenous (IV) and intradermal) administration. The formulations may conveniently be presented in unit dosage form and may be prepared by any methods well known in the art of pharmacy. The nature, availability and sources, and the administration of all such compounds including the effective amounts necessary to produce desirable effects in a subject are well known in the art and need not be further described herein.

The pharmaceutical forms suitable for injection use include sterile aqueous solutions or dispersions and sterile powders for the extemporaneous preparation of sterile injectable solutions or dispersions. In all cases the form must be sterile and must be fluid to the extent that easy syringeability exists. It must be stable under the conditions of manufacture and storage and must be preserved against the contaminating action of microorganisms, such as bacteria and fungi.

The prevention of the action of microorganisms can be brought about by various antibacterial and antifungal agents, for example, parabens, chlorobutanol, phenol, sorbic acid, thimerosal,-and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above.

In the case of sterile powders for the preparation of the sterile injectable solutions, the preferred method of preparation are vacuum-drying and freeze drying techniques which yield a powder of the active ingredient plus any additional desired ingredient from a previously sterile-filtered solution thereof.

The pharmaceutical compositions of the invention generally comprise a buffering agent, an agent which adjusts the osmolarity thereof, and optionally, one or more pharmaceutical acceptable carriers, excipients and/or additives as known in the art. Supplementary active ingredients can also be incorporated into the compositions. The carrier can be solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants.

As used herein "pharmaceutically acceptable carrier" includes any and all solvents, dispersion media, coatings, antibacterial and antifungal agents and the like. The use of such media and agents for pharmaceutical active substances is well known in the art. Except as any conventional media or agent is incompatible with the active ingredient, its use in the therapeutic composition is contemplated.

The preparation of pharmaceutical compositions is well known in the art and has been described in many articles and textbooks, see e.g., Remington's Pharmaceutical Sciences, Gennaro A. R. ed., Mack Publishing Co., Easton, PA, 1990, and especially pp. 1521-1712 therein. The ability to discriminate between self and non-self is perhaps the most fundamentally important aspect of immune regulation. This property translates into the immune- recognition and destruction of infectious invaders while normal host tissues are left untouched. This highly selective response is characterized by a complicated set of T cell regulatory mechanisms that have been described over the past decades. One such mechanism designed to maintain the fidelity of the immune response is the requirement of two distinct signals for effective activation of antigen-specific T cells: an antigen-specific signal via the T cell receptor (Signal 1) and a noncognate costimulatory signal (Signal 2) that is provided by soluble factors or cell-surface molecules on the antigen presenting cell (APC). The integration of these two signals triggers cell division and differentiation of effectors and regulators of the immune response. Aside from the critical biological implications of co stimulation, the identification of a costimulatory signal has important implications for clinical intervention as the effects of costimulation blockade would be restricted to only those T cells whose antigen-specific receptors have already been engaged, i.e. T cells already receiving signal 1. Thus, in principle, the selective blockade of T cell costimulation offers an antigen-specific mode of targeting immune responses without actual knowledge of the specific antigen involved. In fact, in some instances, costimulatory pathway antagonists can induce antigen-specific tolerance that prevents the progression of autoimmune diseases and organ graft rejection.

The critical importance of CD28/B7 costimulation in T cell activation has led to multiple studies that examine the role of costimulation in experimental models of autoimmune diseases. Early studies showed that disruption of CD28/B7 costimulation at the time of immunization was associated invariably with the reduction of the severity of the pathology and, in some cases, with complete disease prevention. For instance, CD28/B7 blockade by CTLA-4Ig or, in some cases, anti-B7 mAbs reduced disease severity in mouse models of multiple sclerosis, myocarditis, arthritis, thyroiditis, and myasthenia gravis.

The therapeutically'effective amount'for purposes herein is that determined by such considerations as are known in the art. The amount must be sufficient to inhibit the direct interaction between a T cell co-stimulatory pathway member, which is the CD28, CTLA-4, ICOS and PD-lmolecules and a component of a pathogenic agent, such as the pyrogenic exotoxin and to antagonize toxin-mediated activation of T cells.

According to one embodiment, the invention relates to the peptides and compositions of the invention for use in a method for the treatment of immune disorders related to an imbalance in the Th1-Th2 response. Examples of said disorders are autoimmune diseases (for example, multiple sclerosis (MS), Type-1 diabetes, lupus, Graves disease and thyroiditis), malignant and non-malignant proliferative disorders, graft rejection pathology and graft versus host disease.

As used herein to describe the present invention, the terms"malignant proliferative disorder", "cancer","tumor"and"malignancy"all relate equivalently to a hyperplasia of a tissue or organ. If the tissue is a part of the lymphatic or immune systems, malignant cells may include non- solid tumors of circulating cells. Malignancies of other tissues or organs may produce solid tumors. In general, the composition as well as the peptides of the present invention may be used in the treatment of non-solid and solid tumors, for example, carcinoma, melanoma, leukemia, and lymphoma.

Therefore, according to a preferred embodiment, the immunomodulatory peptide of the invention or a composition comprising the same, can be used for the treatment or inhibition of non-solid cancers, e. g. hematopoietic malignancies such as all types of leukemia, e. g. acute lymphocytic leukemia (ALL), acute myelogenous leukemia (AML), chronic lymphocytic leukemia (CLL), chronic myelogenous leukemia (CML), myelodysplastic syndrome (MDS), mast cell leukemia, hairy cell leukemia, Hodgkin's disease, non-Hodgkin's lymphomas, Burkitt's lymphoma and multiple myeloma, as well as for the treatment or inhibition of solid tumors such as tumors in lip and oral cavity, pharynx, larynx, paranasal sinuses, major salivary glands, thyroid gland, esophagus, stomach, small intestine, colon, colorectum, anal canal, liver, gallbladder, extraliepatic bile ducts, ampulla of vater, exocrine pancreas, lung, pleural mesothelioma, bone, soft tissue sarcoma, carcinoma and malignant melanoma of the skin, breast, vulva, vagina, cervix uteri, corpus uteri, ovary, fallopian tube, gestational trophoblastic tumors, penis, prostate, testis, kidney, renal pelvis, ureter, urinary bladder, urethra, carcinoma of the eyelid, carcinoma of the conjunctiva, malignant melanoma of the conjunctiva, malignant melanoma of the uvea, retinoblastoma, carcinoma of the lacrimal gland, sarcoma of the orbit, brain, spinal cord, vascular system, hemangiosarcoma and Kaposi's sarcoma.

According to a specifically preferred embodiment, any of the peptides defined by the invention, or any combination conjugates and composition thereof may be used for such methods.

It should be noted that also peptides derived from the "antagonist domain" (such as p*12A* and p*12B*), may be used by the method of the invention.

More specifically, the method of the invention may use a peptide, that may be selected from the group consisting of p*1TA* (as denoted by SEQ ID NO: 9), p*2TA* (as denoted by SEQ ID NO: 12), p*1TB* (as denoted by SEQ ID NO: 14), p*2TB* (as denoted by SEQ ID NO: 15), p*1TC* (as denoted by SEQ ID NO: 16), p*2TC* (as devoted by SEQ ID NO: 17), *p1TD* (as denoted by SEQ ID NO : 57), *p2TD* (as denoted by SEQ ID NO: 58) and any combination, and composition thereof.

In yet another embodiment, the invention relates to use of a peptide of the invention for the preparation of a composition for use in the treatment of immune disorders related to an imbalance in the Th1-Th2 response in a subject in need thereof, wherein said immune disorder is any one of an autoimmune disease, malignant and non-malignant proliferative disorder, graft rejection pathology, and a disorder induced by a pyrogenic exotoxin or by a mixture of at least two pyrogenic exotoxins selected from toxic shock and incapacitation or for use in a method of preventing death induced by a pyrogenic exotoxin or by a mixture of at least two pyrogenic exotoxins.

In the case of sterile powders for the preparation of the sterile injectable solutions, the preferred method of preparation are vacuum-drying and freeze drying techniques which yield a powder of the active ingredient plus any additional desired ingredient from a previously sterile-filtered solution thereof.

The pharmaceutical compositions of the invention generally comprise a buffering agent, an agent which adjusts the osmolarity thereof, and optionally, one or more pharmaceutically acceptable carriers, excipients and/or additives as known in the art. Supplementary active ingredients can also be incorporated into the compositions. The carrier can be solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants.

As used herein "pharmaceutically acceptable carrier" includes any and all solvents, dispersion media, coatings, antibacterial and antifungal agents and the like. The use of such media and agents for pharmaceutical active substances is well known in the art. Except as any conventional media or agent is incompatible with the active ingredient, its use in the therapeutic composition is contemplated.

The preparation of pharmaceutical compositions is well known in the art and has been described in many articles and textbooks, see e.g., Remington's Pharmaceutical Sciences, Gennaro A. R, ed., Mack Publishing Co., Easton, PA, 1990, and especially pp. 1521-1712 therein. p*1TD* (as denoted by SEQ ID NO: 57), p*2TD* (as denoted by SEQ ID NO: 58) and any combination thereof.

Engagement of MHC class II molecule and TCR by a superantigen is insufficient for induction of Th1 cytokines that mediate lethal toxic shock. SPR affinity studies showed that in absolute terms, the interaction of superantigens with either MHC II or TOR is very weak [Seth (1994) *ibid*.; Redpath (1999) *ibid.].* By contrast, the affinity of SEB for CD28 is far greater, giving this interaction a pivotal role in the formation of a stable immunological synapse. By engaging the three ligands simultaneously, the superantigen is able to deliver an activation signal to the Th1 cell. Peptide mimetics that interfere with the binding of superantigen to CD28 will disrupt synapse formation, preventing the induction of a Th1 response (Figure 7J). The findings of the present invention explain why short peptide mimetics of the antagonist domain are potent superantigen antagonists whereas mimetic of the TOR and/or MHC II binding sites in SEB failed to inhibit the Th1 response [Arad (2000) *ibid*.].

CD28 exists as a homodimer on the cell surface but dimerization of CD28 and of CTLA4 is not required for B7 binding, nor is it sufficient to trigger signaling by conventional antigens [Linsley, P.S. et al., J. Biol. Chem. 270(25):15417-24 (1995)]. Only one monomeric B7-2 molecule is thought to engage the CD28 dimer [Collins (2002) *ibid*.]. SEB induced a transient change in cell surface presentation of CD28, rendering it accessible to an αCD28 mAb specific for one rim of the predicted CD28 dimer interface (Figures 3A, 6A, and 6B). The finding that αCD28 alone can trigger a Th1 response (Figures 3B, 3C, and 6B) supports the existence of an equilibrium between states of accessibility of CD28 that can be shifted by SEB (Figure 3A). The inventors have shown that SEB engages CD28 at both rims of the dimer interface. When it interacts with one CD28 monomer, the superantigen may displace the other monomer, which now becomes accessible to αCD28.

During their convergent evolution, the superantigen toxins from S. *aureus* and *S. pyogenes* acquired structures designed to recognize the receptors of the human immune system critical for their function, among them TCR and MHC class II molecules. Yet, individual superantigens exhibit wide diversity in the way they interact with these two ligands [Sundberg (2002a) *ibid*.; Sundberg (2002b) *ibid*.]. By contrast, binding of a superantigen to the third receptor, CD28, is not only with higher affinity but involves a conserved structure in both molecules, rendering TSST-1 as sensitive as SEB to mimetics of the antagonist domain [Arad (2000) *ibid*.] and of the dimer interface in CD28 (Figures 6G and 6H).

CD28 has a unique role as early signal transducer for innate immunity. Whereas CD28 is expressed constitutively and is essential for an immediate Th1 response, ICOS is induced later in dependence on CD28 and promotes primarily a Th2 response. The late induction of CTLA4, also dependent on CD28, acts to shut off these earlier responses [reviewed by Rudd and Schneider (2003) *ibid*.], Without being bound by any theory, the inventors hypothesize that because SEB has the potential to bind not only to CD28 but also to ICOS and CTLA44, it may use the latter ligands to modulate the Th1 response induced via CD28.

The CD28 coligand B7-2 is expressed constitutively and induced rapidly during the innate immune response [reviewed by Sharpe and Freeman (2002) *ibid*.]. The action of SEB was totally dependent on B7-2 (Figures 2A and 2B). sB7-2 bound directly to SEB in SPR kinetic analysis, with an affinity resembling that for sCD28 but with lower avidity (Figure 4). Simultaneous engagement of B7-2 and CD28 by the superantigen may stabilize binding of B7-2 to CD28 and thus trigger extensive costimulation. The results of the present invention lead to the novel concept that by directly engaging CD28 and B7-2 in addition, to the MHC Class II molecule and TOR, the superantigen recruits four ligands in an unconventional manner, two from the antigen-presenting cell and two from the T cell, to create an unusually stable immunological synapse leading to an excessive Th1 response.

Signaling through the TCR is amplified by CD28 in a B7-2-dependent manner [see Acuto and Michel (2003) *ibid*.]. Induction of IL2 and IFN-γ gene expression by αCD3 jointly with sB7-2 or with αCD28, which engage the TCR and CD28 to mimic immunological synapse formation, was blocked by superantigen mimetic peptide p*12B* (Figures 3C and 3F). Apparently, such signaling occurs mainly through the superantigen binding site in CD28, rendering it sensitive to p*12B*. Hence, this site in CD28, the dimer interface as distinct from the B7-2 binding site (Fig. 6A) and OD28₆₀₋₆₅ described by Luhder et al. [Luhder (2003) *ibid*.], is critical for TOR signaling.

Induction of Th1 cytokine genes by SEB was attenuated severely by a concomitant induction of Th2 cytokines IL4 and IL10 (Figure 1). In contrast to the Th1 response, however, induction of a Th2 cytokine response by superantigen does not require signaling through CD28. □CD28 or sB7-2 failed to induce IL10 (Figures 3D and 3G). In all cases where the Th1 response was blocked by soluble ligands or by peptide mimetics of superantigen or of CD28, CTLA4 and ICOS, the Th2 response, measured through IL10 or IL4, remained unabated. Inhibition of the CD28-dependent Th1 response by superantigen antagonist peptides thus leaves the Th2 response intact, with the concomitant induction of protective immunity (Figure IF; [Arad (2000) *ibid*.]). The selective requirement for CD28 signaling in Th1 cytokine gene expression renders this response more sensitive to regulation. By contrast, activation of the Th2 response bypasses the CD28 requirement and therefore, is less stringently controlled.

According to another embodiment, the invention relates to the inhibition of activation of a T cell costimulatory pathway by a pathogenic agent. Reference to pathogenic agents includes a prokaryotic microorganism, a lower eukaryotic microorganism, a complex eukaryotic organism, a virus, fungi, prions, parasite, yeast and venoms.

A prokaryotic microorganism includes bacteria such as Gram positive, Gram negative and Gram variable bacteria and intracellular bacteria. Examples of bacteria contemplated herein include the speices of the genera *Treponema sp., Borrelia sp., Neisseria sp., Legionella sp., Bordetella sp., Escherichia sp., Salmonella sp., Shigella sp., Klebsiella sp., Yersinia sp., Vibrio sp., Hemophilus sp., Rickettsia sp., Chlamydia sp., Mycoplasma sp., Staphylococcus sp., Streptococcus sp., Bacillus sp.,* Clostridium *sp., Corynebacterium sp., Proprionibacterium sp*., *Myeobacterium sp., Ureaplasma sp. and Listeria sp.*

Particularly preferred species include *Treponema pallidum, Borrelia burgdorferi, Neisseria gonorrhea, Neisseria meningitidis, Legionella pneumophila, Bordetella pertussis, Escherichia coli, Salmonella typhi, Salmonella typhimurium, Shigella dysenteriae, Klebsiella pneumoniae, Yersinia pestis, Vibrio cholera, Hemophilus influenzae, Rickettsia rickettsii, Chlamydia trachomatis*, *Mycoplasma pneumoniae, Staphylococcis aureus, Streptococcus pneumoniae, Streptococcus pyogenes, Bacillus anthracis, Clostridium botulinum, Clostridium tetani, Clostridium perfringens*, *Corynebacterium diphtheriae, Proprionibacterium acnes, Mycobacterium tuberculosis, Mycobacterium leprae* and *Listeria monocytogenes.*

A lower eukaryotic organism includes a yeast or fungus such as but not limited to *Pneumocystis carinii*, *Candida albicans,* Aspergillus, *Histoplasma capsulatum, Blastomyces dermatitidis, Cryptococcus neoformans*, Trichophyton and Microsporum.

A complex eukaryotic organism includes worms, insects, arachnids, nematodes, aemobe, *Entamoeba histolytica, Giardia lamblia, Trichomorcas vaginalis, Trypanosoma brucei gambiense, Trypanosoma cruzi, Balantidittm coli, Toncoplasma gondii,* Cryptosporidium or Leishmania.

The term "viruses" is used in its broadest sense to include viruses of the families adenoviruses, papovaviruses, herpesviruses: simplex, varicella-zoster, Epstein-Barr, CMV, pox viruses: smallpox, vaccina, hepatitis B, rhinoviruses, hepatitis A, poliovirus, rubellavirus, hepatitis C, arboviruses, rabiesvirus, influenzaviruses A and B, measlesvirus, mumpsvirus, HIV, HTLV I and II.

The term "fungi" includes for example, fungi that cause diseases such as ringworm, histoplasmosis, blastomycosis, aspergillosis, cryptococcosis, sporotrichosis, coccidioidomycosis, paracoccidio- idoinycosis, and candidiasis.

The term parasite includes, but not limited to, infections caused by somatic tapeworms, blood flukes, tissue roundworms, ameba, and Plasmodium, Trypanosoma, Leishniania, and Toxoplasma species.

According to a preferred embodiment, the peptides and compositions of the invention are particularly useful for inhibiting the activation of a T cell co- stimulatory pathway by a pathogenic bacterium selected from the group consisting of *Staphylococcus aureus* and *Streptococcus pyogenes.*

In yet another preferred embodiment, a component of said bacterium is a superantigen which is a pyrogenic exotoxin. Preferably, the pyrogenic exotoxin may be a bacterial exotoxin and most preferably, this exotoxin may be produced by any one of *Staphylococcus aureus* and *Streptococcus pyogenes.* The superantigen-related disorder treated may be according to a specific embodiment any one of toxic shock, incapacitation and death, induced by a pyrogenic exotoxin or by a mixture of at least two pyrogenic exotoxins.

Accordingly, a preferred component of such pathogenic agent may be a superantigen, preferably a pyrogenic exotoxin.

According to another preferred embodiment, the invention is based on the use of a substance which inhibits the binding of such superantigen to a specific site within a molecule belonging to the CD28/B7 pathway which is CD28, CTLA-4, ICOS or PD-1.

According to one specific embodiment, the superantigen binding site may be within the dimer interface of the CD28 which comprises amino acid residues 10-15 or 116-121 of the human CD28 amino acid sequence as denoted by SEQ ID NO: 19, where the peptide of the invention consists of:
(a) a peptide consisting of an amino acid sequence that is selected from the amino acid sequence HVKGKHLCP as denoted by SEQ ID NO: 9 and the amino acid sequence SPMLVAYD as denoted by SEQ ID NO: 12;
(b) a peptide which is at least 80% homologous to the peptide of (a), wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a peptide of (a), (b) or (c) that is extended at the N terminus and/or the C terminus:
   (v) by a lauryl cysteine at the N terminus and a cysteine at the C terminus; or
   (vi) by an organic moiety that is not a naturally occurring or synthetic amino acid residue; or
   (vii) by identical hydrophobic amino acid residue(s) which may be naturally occurring or synthetic amino acid residues; or
   (viii) by a lysyl-palmitoyl tail, wherein said tail is at the N terminus, wherein the resultant peptide of (c) maintains the ability to inhibit the interaction between a T cell costimulatory pathway member and a superantigen; or
(e) a dimer or multimer of (a), (b), (c) or (d), wherein the resultant peptide of (e) maintains the ability to inhibit the interaction between a T Cell costimulatory pathway member and a superantigen.

According to another embodiment, the superantigen binding site may be within the dimer interface of the CTLA-4 molecule which comprises amino acid residues 10-15 or 115-120 of the human CTLA-4 amino acid sequence as denoted by SEQ ID NO: 20, where the peptide of the invention consists of:
(a) a peptide consisting of an amino acid sequence that is selected from one of the amino acid sequence YVIDPEPCP as denoted by SEQ ID NO: 14 and the amino acid sequence PAVVLASS as denoted by SEQ ID NO: 15;
(b) a peptide of (a) that is extended at the N terminus and/or the C terminus:
   (i) by lauryl cysteine at the N terminus and a cysteine at the C terminus; or
   (ii) by an organic moiety that is not a naturally occurring or synthetic amino acid residue; or
   (iii) by identical hydrophobic amino acid residue(s) which may be naturally occurring or synthetic amino acid residues; or
   (iv) by a lysyl-palmitoyl tail, wherein said tail is at the N terminus, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a dimer or multimer of (a), (b) or (c), wherein the resultant peptide of (e) maintains the ability to inhibit the interaction between a T Cell costimulatory pathway member and a superantigen.

Alternatively, the superantigen binding site may be within the dimer interface of the ICOS molecule, which comprises part or all of amino acid residues 10-15 or 119-124 of the human ICOS amino acid sequence as denoted by SEQ ID NO: 21, where the peptide of the invention consists of:
(a) peptide consisting of an amino acid sequence that is selected from the amino acid sequence YESQLCCQL as denoted by SEQ ID NO: 16 and the amino acid sequence GEINGSAN as denoted by SEQ ID NO: 17;
(b) a peptide of (a) or (b) that is extended at the N terminus and/or the C terminus:
   (i) by lauryl cysteine at the N terminus and a cysteine at the C terminus; or
   (ii) by an organic moiety that is not a naturally occurring or synthetic amino acid residue; or
   (iii) by identical hydrophobic amino acid residue(s) which may be naturally occurring or synthetic amino acid residues; or
   (iv) by a lysyl-palmitoyl tail, wherein said tail is at the N terminus, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a dimer or multimer of (a), (b) or (c), wherein the resultant peptide of (d) maintains the ability to inhibit the interaction between a T Cell costimulatory pathway member and a superantigen.

Alternatively, the superantigen binding site may be within the domains in the PD-1 molecule that correspond to the dimer interface in CTLA4, comprising amino acid residues 8-13 and 110-116 of the human PD-1 sequence as denoted by SEQ ID NO: 59, where the peptide of the invention consists of:
(a) a peptide consisting of an amino acid sequence that is selected from the amino acid sequence RVTERRAEV as denoted by SEQ ID NO: 57 and the amino acid sequence PALLVVTE as denoted by SEQ ID NO: 58;
(b) a peptide of (a) that is extended at the N terminus and/or the C terminus:
   (i) by lauryl cysteine at the N terminus and a cysteine at the Cterminus; or
   (ii) by an organic moiety that is not a naturally occurring or synthetic amino acid residue; or
   (iii) by identical hydrophobic amino acid residue(s) which may be naturally occurring or synthetic amino acid residues; or
   (iv) by a lysyl-palmitoyl tail, wherein said tail is at the N terminus, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a dimer or multimer of (a), (b) or (c), wherein the resultant peptide of (c) maintains the ability to inhibit the interaction between a T Cell costimulatory pathway member and a superantigen.

As shown by the Examples, the superantigen specifically binds to its binding site within the dimer interface of CD28, CTLA4 and ICOS. Figure 4 further indicates that the superantigen binds to a yet undefined site within the B7-2 molecule.

As shown by the inventors, the dimer interface of the CD28 family members, specifically and directly binds to a spatially conserved domain of a pyrogenic exotoxin. Preferably, this spatially conserved domain is not involved in the binding of any one of MHC Class II molecules and TCR. Most preferably, the said spatially conserved domain of pyrogenic exotoxin forms therein a central turn starting within a β-stand 7 and connecting the β-strand 7, via short β-strand 8, to an α-helix 4, and ending within α-helix 4, based on the domain numbering of SEB [Arad *et al.,* (2000), (2001) *ibid*.].

In one preferred embodiment, the substance used for inhibiting the direct interaction between a component derived from said pathogenic agent which is a superantigen, and a binding site within a T cell co-stimulatory pathway member molecule, which is a peptide derived from the dimer interface of a T cell costimulatory pathway member.

According to a specific embodiment, a peptide of the invention from the dimer interface of a T cell co-stimulatory pathway member may be provided for use in inhibiting the specific interaction between the superantigen and the CD28 family member.

According to a preferred embodiment, the peptide used by the invention inhibits the direct interaction between CD28B7 family molecules and said pyrogenic exotoxin. According to a preferred embodiment of this aspect of the invention, inhibition of binding of said component of a pathogenic agent. The component is a pyrogenic exotoxin to said T cell co-stimulatory pathway member, of the CD28B7 family, by the substance of the invention, which leads to antagonizing toxin-mediated activation of Th1 lymphocytes and may also lead to indirect elicitation of protective immunity against toxic shock induced by said pyrogenic exotoxin or by a mixture of at least two pyrogenic exotoxins. More particularly, this binding is mediated by the superantigen binding site in CD28 as defined by the invention.

Therefore, an antagonist of a toxin-mediated activation of T lymphocytes, protects against toxic shock induced by a pyrogenic exotoxin or by a mixture of pyrogenic exotoxins and may also indirectly elicit protective immunity against toxic shock induced by a pyrogenic exotoxin or by a mixture of pyrogenic exotoxins. By blocking the ability of the toxin to induce a cellular immune response leading to toxic shock, the antagonist peptides of the invention may allow the superantigen to induce a vigorous humoral immune response directed against itself. Therefore, the treated subject may acquire protective immunity against further toxin challenges, and develop protective antitoxin antibodies. Thus, the antagonist peptide of the invention can be used for immediate treatment of acute toxic shock and of the harmful effects which may be due to, for example, accidental food poisoning, induced by pyrogenic exotoxins. In addition, it may indirectly confer long-term immunity against such toxic shock, as described above.

Where the peptide that inhibits the direct interaction between a T cell co-stimulatory pathway member, for example, CD28 and a component of a pathogenic agent, preferably, a superantigen, is for example a peptide having low immunogenicity and relative rapid clearance, antibodies against such antagonist peptide may not be detected. However, by blocking the ability of the toxin to induce a cellular immune response leading to toxic shock, the antagonist peptide allows the superantigen to induce a vigorous humoral immune response directed against itself. Under these conditions, the superantigen acts as its own adjuvant. Thus, when lethal toxic shock is prevented by antagonist peptide during exposure to a superantigen (by inhibiting the CD28-superantigen interaction), the treated subject may acquire protective immunity against further toxin challenges, even with different toxins, and develop protective antitoxin antibodies. Therefore, the use of the peptide of the invention that inhibits the direct interaction between CD28 molecule and a superantigen toxin, may indirectly elicit protective immunity against toxic shock induced by said pyrogenic exotoxin.

In a further aspect, the invention relates to treatment of pathological disorders related to an imbalance in the Th1-Th2 response caused by a pathogenic agent in a subject in need thereof. An inhibitory effective amount of peptide of the invention is administered. According to one embodiment, the invention is intended for the treatment of pathologies such as malignant and non-malignant proliferative disorder, and an immune related disorder, for example, inflammation, autoimmune disease, and also exotoxin related disorders.

Inflammation includes any inflammatory conditions wherein said inflammatory conditions may be any one of rheumatoid arthritis, adult respiratory distress syndrome (ARDS), asthma, rhinitis, idiopathic pulmonary fibrosis, peritonitis, cardiovascular inflammation, myocardial ischemia, reperfusion injury, atherosclerosis, sepsis, trauma, diabetes type II, retinopathy, psoriasis, gastrointestinal inflammation, cirrhosis and inflammatory bowel disease.

The T cell co-stimulatory pathway is the CD28/B7 T cell co-stimulatory pathway.

More specifically, the CD28/B7 pathway member may be any one of CD28, CTLA-4, ICOS and PD-1.

In one embodiment, the pathway member may be the CD28 molecule and the dimer interface within CD28 comprises amino acid residues 10-15 or 116-121 of the human CD28 amino acid sequence as denoted by SEQ ID NO: 19, where the peptide of the invention consists of:
(a) a peptide consisting of an amino acid sequence that is selected from the amino acid sequence HVKGKHLCP as denoted by SEQ ID NO: 9 and the amino acid sequence SPMLVAYD as denoted by SEQ ID NO: 12;
(b) a peptide which is at least 80% homologous to the peptide of (a),
   wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a peptide of (a), (b) or (c) that is extended at the N terminus and/or the C terminus:
   (v) by a lauryl cysteine at the N terminus and a cysteine at the C terminus; or
   (vi) by an organic moiety that is not a naturally occurring or synthetic amino acid residue; or
   (vii) by identical hydrophobic amino acid residue(s) which may be naturally occurring or synthetic amino acid residues; or
   (viii) by a lysyl-palmitoyl tail, wherein said tail is at the N terminus, wherein the resultant peptide of (c) maintains the ability to inhibit the interaction between a T cell costimulatory pathway member and a superantigen; or
(e) a dimer or multimer of (a), (b), (c) or (d), wherein the resultant peptide of (e) maintains the ability to inhibit the interaction between a T Cell costimulatory pathway member and a superantigen.

In another embodiment, the pathway member may be the CTLA-4 molecule and the dimer interface within CTLA-4 comprises amino acid residues 10-15 or 115-120 of the human CTLA-4 amino acid sequence as denoted by SEQ ID NO: 20, where the peptide of the invention consists of:
(a) a peptide consisting of an amino acid sequence that is selected from one of the amino acid sequence YVIDPEPCP as denoted by SEQ ID NO: 14 and the amino acid sequence PAVVLASS as denoted by SEQ ID NO: 15;
(b) a peptide of (a) that is extended at the N terminus and/or the C terminus:
   (i) by lauryl cysteine at the N terminus and a cysteine at the C terminus; or
   (ii) by an organic moiety that is not a naturally occurring or synthetic amino acid residue; or
   (iii) by identical hydrophobic amino acid residue(s) which may be naturally occurring or synthetic amino acid residues; or
   (iv) by a lysyl-palmitoyl tail, wherein said tail is at the N terminus, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a dimer or multimer of (a), (b) or (c), wherein the resultant peptide of (e) maintains the ability to inhibit the interaction between a T Cell costimulatory pathway member and a superantigen.

Alternatively, the pathway member may be the ICOS molecule, and the dimer interface within ICOS comprises amino acid residues 10-15 or 119-124 of the human ICOS amino acid sequence as denoted by SEQ ID NO: 21, where the peptide of the invention consists of:
(a) peptide consisting of an amino acid sequence that is selected from the amino acid sequence YESQLCCQL as denoted by SEQ ID NO: 16 and the amino acid sequence GEINGSAN as denoted by SEQ ID NO: 17;
(b) a peptide of (a) or (b) that is extended at the N terminus and/or the C terminus:
   (i) by lauryl cysteine at the N terminus and a cysteine at the C terminus; or
   (ii) by an organic moiety that is not a naturally occurring or synthetic amino acid residue; or
   (iii) by identical hydrophobic amino acid residue(s) which may be naturally occurring or synthetic amino acid residues; or
   (iv) by a lysyl-palmitoyl tail, wherein said tail is at the N terminus, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a dimer or multimer of (a), (b) or (c), wherein the resultant peptide of (d) maintains the ability to inhibit the interaction between a T Cell costimulatory pathway member and a superantigen.

Alternatively, the pathway member may be the PD-1 molecule and the domains within the PD-1 molecule that correspond to the dimer interface in CTLA4 comprise amino acid residues 8-13 or 110-116 of the human PD-1 sequence as denoted by SEQ ID NO: 59, where the peptide of the invention consists of:
(a) a peptide consisting of an amino acid sequence that is selected from the amino acid sequence RVTERRAEV as denoted by SEQ ID NO: 57 and the amino acid sequence PALLVVTE as denoted by SEQ ID NO: 58;
(b) a peptide of (a) that is extended at the N terminus and/or the C terminus:
   (i) by lauryl cysteine at the N terminus and a cysteine at the Cterminus; or
   (ii) by an organic moiety that is not a naturally occurring or synthetic amino acid residue; or
   (iii) by identical hydrophobic amino acid residue(s) which may be naturally occurring or synthetic amino acid residues; or
   (iv) by a lysyl-palmitoyl tail, wherein said tail is at the N terminus, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a dimer or multimer of (a), (b) or (c), wherein the resultant peptide of (c) maintains the ability to inhibit the interaction between a T Cell costimulatory pathway member and a superantigen.

In yet another embodiment, the method of the invention is intended for the use in treatment of pathologies caused by a pathogenic agent such as bacterial pathogens, viruses, fungi, prions, parasites, yeast and venoms.

In a specifically preferred embodiment, such pathogenic agent may be a pathogenic bacterium selected from the group consisting of *Staphylococcus aureus* and *Streptococcus pyogenes.* Accordingly, a component of said bacteria which specifically binds to a specific binding site within a T cell co-stimulatory pathway member, may be a superantigen, preferably a pyrogenic exotoxin.

More particularly, the inhibition of the direct interaction between the T cell co-stimulatory pathway member, preferably the CD28 molecule and the pyrogenic exotoxin leads to inhibition of exotoxin-mediated activation of Th1-lymphocytes, protection against toxic shock and may also leads to indirect elicitation of protective immunity against toxic shock induced by a pyrogenic exotoxin or by a mixture of at least two pyrogenic exotoxins.

According to a preferred embodiment, the peptides and compositions of the invention are particularly useful for use in methods of treatment of toxic shock, incapacitation and death, induced by a pyrogenic exotoxin or by a mixture of at least two pyrogenic exotoxins.

By the term 'immunologically effective amount'is meant any amount sufficient to enhance the production of antibodies that block T cell activation, preferably, Th1 response induced by pyrogenic exotoxins, and confer immunity against toxic shock induced by a pyrogenic exotoxin or by a mixture of pyrogenic exotoxins.

The term toxin-mediated activation as used throughout this application can mean activation of T cells mediated by a single pyrogenic exotoxin or a mixture of such toxins.

The antagonist peptide of the invention can be used for immediate treatment of acute toxic shock and of the harmful effects which may be due to, for example, accidental food poisoning, induced by pyrogenic exotoxins. In addition, by blocking the ability of the toxin to induce a cellular immune response leading to toxic shock, the antagonist peptide may also allow the superantigen to induce a vigorous humoral immune response directed against itself, and therefore, these peptides may indirectly confer long-term immunity against such toxic shock.

In humans for example, toxic shock resulting from exposure to pyrogenic exotoxins has two distinct components: (1) incapacitation and (2) death. Even at concentrations several logs below lethal ones, pyrogenic exotoxins severely incapacitate, causing high morbidity [USAMRIID Manual, (1998) Eitzen, E. Pavlin, J. Cieslak, T. Christopher, G. Culpepper, R. eds. Medical Management of Biological Casualties Handbook. 3rd ed. Fort Detrick, Maryland: United States Army Medical Research Institute of Infectious Diseases, 1998]. Incapacitation response observed, e. g. in food poisonings, which may be mass poisonings, affects large numbers of people. Moreover, the incapacitation response may be a military threat and a national security threat.

According to one embodiment, the invention uses as a substance which inhibits the direct interaction between a component derived from said pathogenic agent and a binding site within a T cell co-stimulatory pathway member molecule, a peptide of the invention from the dimer interface of a T cell co-stimulatory pathway member.

According to a specifically preferred embodiment, inhibition of the interaction of a pathogenic agent, such as a pyrogenic exotoxin and a CD28 family member, may be performed by a peptide of the invention from the dimer interface of a T cell co-stimulatory pathway member.

According to a specifically preferred embodiment, the peptide used by the invention inhibits the direct interaction between CD28 molecule and said pyrogenic exotoxin.

In another preferred embodiment, the peptide used by the invention is an antagonist of a toxin-mediated activation of T lymphocytes and protects against toxic shock induced by a pyrogenic exotoxin or by a mixture of pyrogenic exotoxins.

Various methods of administration may be used for delivering the peptides or the compositions of the invention to a subject in need. Peptides may be delivered via intravenous (i.v.), intramuscular (i.m.) intraperitoneal (i.p.) injections, orally (in liquid form or prepared as dosage unit forms like capsules, pills, lozenges, etc.). In order to be effective therapeutically, peptides should be prepared in a way that would enable their stability in the system following injection, or yet more preferably, following oral administration. Alternatively, the peptides of the invention may also be delivered via transdermal delivery using patches, ointment or cream.

The pharmaceutical compositions of the present invention may be administered in a number of ways depending upon whether local or systemic treatment is desired and upon the area to be treated. Administration may be topical (including ophthalmic and to mucous membranes including vaginal and rectal delivery), pulmonary, e.g., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, e.g., intrathecal or intraventricular, administration.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful.

Compositions and formulations for oral administration include powders or granules, suspensions or solutions in water or non-aqueous media, capsules, sachets or tablets. Thickeners, flavoring agents, diluents, emulsifiers, dispersing aids or binders may be desirable.

Compositions and formulations for parenteral, intrathecal or intraventricular administration may include sterile aqueous solutions which may also contain buffers, diluents and other suitable additives such as, but not limited to, penetration enhancers, carrier compounds and other pharmaceutically acceptable carriers or excipients. Pharmaceutical compositions of the present invention include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general the formulations are prepared by uniformly and intimately bringing into association the active ingredients with liquid carriers or finely divided solid carriers or both, and then, if necessary, shaping the product.

The compositions of the present invention may be formulated into any of many possible dosage forms such as, but not limited to, tablets, capsules, liquid syrups, soft gels, suppositories, and enemas. The compositions of the present invention may also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers.

In one embodiment of the present invention the pharmaceutical compositions may be formulated and used as foams. Pharmaceutical foams include formulations such as, but not limited to, emulsions, microemulsions, creams, jellies and liposomes. While basically similar in nature these formulations vary in the components and the consistency of the final product. The preparation of such compositions and formulations is generally known to those skilled in the pharmaceutical and formulation arts and may be applied to the formulation of the compositions of the present invention.

As described by the present invention and also recently shown by the inventors [WO 03/084995], the cellular target of the superantigen is CD28.

As shown in Figure 4, the present invention further demonstrates that all the three members of the CD28 family binds to the sAg. This finding provides cellular drug targets for the design of antagonists that will inhibit toxic shock and other outcomes of superantigen-mediated overstimulation of the cellular immune response (and in particular, the Th1 response), such as death and toxic incapacitation (manifested by nausea, vomiting, and diarrhea). Most importantly, the invention now allows the design of novel antagonists of the interaction between superantigens and the CD28 receptor, whether by antagonist peptides as illustrated herein or by small molecules or enzymes or proteins. The invention provides a new strategy for discovery of toxic shock antagonists, through use of the peptides of the invention which derived from the dimer interface within the CD28, CTLA-4 and ICOS molecules as bait for binding of antagonist molecules. The latter may be selected, for example; through phage display from random or dedicated peptide libraries, positional scanning of peptide libraries, or from libraries of cyclic peptidomimetics.

Thus, in a further aspect, the present invention relates to a method of screening for a test substance which specifically binds to a T cell costimulatory pathway member and thereby inhibits activation of T-lymphocytes mediated by a pathogenic-agent, which screening method comprises the steps of:
(a) obtaining candidate antagonist substances which bind to a T cell costimulatory pathway member;
(b) selecting from the substances obtained in step (a), a substance that inhibits direct interaction between said T cell costimulatory pathway member and said pathogenic agent or a component derived from said agent, preferably said component is a superantigen; and
(c) determining the inhibitory effect of the substance obtained in step (b) on the superantigen-mediated activation of T-lymphocytes, preferably, Th1 lymphocytes, wherein said candidate antagonist substance is obtained by the steps of: (i) providing a mixture comprising an isolated peptide of the invention; (ii) contacting said mixture with said test substance under suitable conditions for said binding; and (iii) determining the effect of the test substance on an end-point indication, whereby modulation of said end point is indicative of binding of said test substance to said peptide.

Key to the application of high-throughput screening for high-affinity binding of antagonist peptides generated by positional scanning and cyclization chemistry is the development of a sensitive and convenient screening assay.

Development of a robust screening assay for antagonist substances through their affinity for any one of CD28, CTLA-4, ICOS and PD-1 target in the domain recognized by superantigens, will be the first step in said screening method.

In a preferred embodiment, the candidate antagonist substance utilized by the screening method of the invention may be obtained by the steps of:
(a) providing a mixture comprising any of the isolated defined by the invention; (b) contacting the mixture with a test substance under suitable conditions for binding; and (c) determining the effect of the test substance on an end-point indication, whereby modulation of said end point is indicative of binding of said test substance to said peptide.

According to another embodiment, the candidate substance screened by the method of the invention may be selected from the group consisting of: protein based, carbohydrates based, lipid based, natural organic based, synthetically derived organic based, inorganic based, and peptidomimetics based substances.

In another embodiment, the substance may be a product of any one of positional scanning of combinatorial libraries of peptides, libraries of cyclic peptidomimetics, and random or dedicated phage display libraries.

According to another specifically preferred embodiment, the candidate antagonist substance may be evaluated by a method for determining the ability of said substance to antagonize toxin-mediated activation of Th1 lymphocytes and optionally its ability to elicit protective immunity against toxic shock induced by a pyrogenic exotoxin or by a mixture of such pyrogenic exotoxins.

According to a specific embodiment, the end point indication may be the binding of an anti-peptide antibody to the peptide of the invention, which leads to a visually detectable signal. Modulation of binding of the antibody to said peptide, which leads to inhibition or enhancement of such signal may indicate that the test substance binds to the peptide of the invention.

More particularly, each candidate substance, or preferably, peptide, may be placed in a well and direct binding of the peptides of the invention is detected preferably by an antibody specific for said peptides. Conditions for effective binding of the peptides of the invention to a candidate antagonist peptide on the plate may be optimized involving study of pH, salt and buffer composition and, carrier proteins such as BSA. This robust screening yields substances, preferably peptides that bind to the superantigen binding site within the dimer interface of any one of CTLA-4, ICOS, CD28 and PD-1. Substances (particularly peptides) that bind to the peptides of the invention are pooled and then assayed as described below.

According to one preferred embodiment, where the peptide used by the screening method of the invention is a peptide designated p*1TA,* which has an amino acid sequence as denoted by SEQ ID NO: 9, the antibody that should be used for detection may be the mouse anti-CD28 monoclonal antibody designated MAB342, clone 37407.111 of R&D Systems, Inc., Minneapolis, Minnesota, USA.

Alternatively, where the peptide used for the screening method is p*2TA,* p*1TB,* p*2TB,* p*1TC, p2TC,* p*1TD* and p*2TD* which has an amino acid sequence as denoted by SEQ ID NO: 12, 14, 15, 16, 17, 57 and 58, a specific antibody, preferably monoclonal antibody, should be used for detection.

The anti peptides antibody used for this first step of the screening method of the invention may be any one of polyclonal and monoclonal antibody. Generation of polyclonal antibodies against proteins is described in, for example, Chapter 2 of Current Protocols in Immunology, Wiley and Sons Inc.

Monoclonal antibodies may be prepared from B cells taken from the spleen or lymph nodes of immunized animals, in particular rats or mice, by fusion with immortalized B cells under conditions which favour the growth of hybrid cells. The technique of generating monoclonal antibodies is described in many articles and textbooks, such as the above-noted Chapter 2 of Current Protocols in Immunology.

The term "antibody" is meant to include intact molecules as well as fragments thereof, such as, for example, Fab and F(ab')₂, which are capable of binding antigen [Wahl, et al., J. Nucl. Med. 24:316-325 (1983)].

It will be appreciated that Fab and F(ab')₂ and other fragments of the antibodies useful in the present invention may be used for the detection and quantitation of the bound CD28, CTLA-4, ICOS and PD-1 molecules, according to the methods disclosed herein for intact antibody molecules. Such fragments are typically produced by proteolytic cleavage, using enzymes such as papain (to produce Fab fragments) or pepsin (to produce F(ab')₂ fragments).

According to a preferred embodiment, a candidate substance suitable for screening by the method of the invention may be selected from the group consisting of: protein based, carbohydrates based, lipid based, natural organic based, synthetically derived organic based, inorganic based, and peptidomimetics based substances.

Preferably, such substance may be a product of any one of positional scanning of combinatorial libraries of peptides, libraries of cyclic peptidomimetics, and random or dedicated phage display libraries.

In a specifically preferred embodiment, the candidate antagonist substance obtained and selected by the screening method of the invention, may be a peptide. Therefore, combinatorial phage libraries may be used to screen for superantigen antagonist peptides with nanomolar affinity for any one of the CD28, CTLA-4, ICOS and PD-1 receptors. In a particular and nonlimiting example, the PhD-12 library of New England Biolabs may be used.

Panning may be performed in two stages, in the first stage, bound phage are eluted from microplate-bound p*1TA,* p*2TA,* p*1TB,* p*2TB,* p*1TC,* p*2TC,* p*1TD* and p*2TD* of the invention using elution at pH 2.2.

This will select all phages that bind the superantigen binding site of CD28. In the second stage, phage selected as above are bound to p*1TA,* p*2TA,* p*1TB,* p*2TB,* p*1TC,* p*2TC,* p*1TD,* and p*2TD* or to sCD28, sICOS, sCTLA-4 and sPD-1 and eluted specifically with an excess of free SEB. Bound phages are eluted and subjected to between two and three further cycles of panning. Then, direct binding of phage to immobilized p*1TA*, p*2TA,* p*1TB,* p*2TB,* p*1TC, p2TC,* p*1TD,* p*2TD,* sCD28, sICOS, sCTLA-4, sPD-1 or to any fragment thereof comprising the superantigen binding site, may be detected by phage ELISA, scoring for M13 on the plate. Positive phage clones are amplified and sequenced, before synthesis of the peptides in linear form.

The candidate antagonist substances which bind to the peptides of the invention and therefore to the superantigen binding site within CD28, that were preferably obtained as described above, may be further selected for their ability to prevent the interaction between any one of the CD28, ICOS, CTLA-4, PD-1 molecules and the superantigen.

One possible approach to examine the ability of the candidate substance to inhibit the interaction between the superantigen and any one of the CD28, ICOS, CTLA-4, PD-1 molecules, is to use biotinylated SEB (Toxin Technologies) and assay for the ability of peptides to displace labeled SEB from binding to sCD28, sICOS, sCTLA-4 or sPD-1 on a plate.

The candidate antagonist peptide obtained and selected by the screening method of the invention may be further analyzed and improved by positional scanning.

In a pepscan positional scan, the start affinity can be as low as 10⁻³ M and the peptide length can easily be 15 residues. Lead peptides may be derived from any type of peptide library, including random combinatorial libraries or peptide libraries derived from given protein sequences. A sound signal-to-noise ratio allows detection of specific low-affinity interactions. It can be used on a solid support [Schroeijers et al., Cancer Res. 60:1104-1110 (2000)] or after split of the peptides from the support and their use in soluble form [Kast et al., Cell 59:603-614 (1989); Kast et al., Proc. Natl. Acad. Sci. U.S.A. 88:2283-2287 (1991); De Samblanx et al., Pept. Res. 9:262-268 (1996); Oosterom et al., J. Biol. Chem. 274:16853-16860 (1999)]. In constrained positional scanning, all candidate peptides are synthesized as non-reducable loops; it is used to further improve the affinity of lead peptides. Thus, peptides are linked to a solid support (pepscan-I) or assayed as free soluble peptides (pepscan-II) to optimize the affinity of lead peptides.

An alanine scan may be performed on a candidate antagonist peptide to identify residues critical for binding to the receptor and, separately, for superantigen antagonist activity in vitro. The in vitro antagonist activity may be evaluated according to the evaluation step of the screening method of the invention, described below. Peptides are synthesized in soluble form with *N*-terminal acetyl and *C*-terminal - CONH₂ and retain flanking *D*-alanines for greater protease resistance in in vitro assays with PBMC as an evaluating step. Further rounds of alanine scan may be performed on identified lead peptides. Because lysine is prominent in the superantigen antagonist domain, a lysine-scan of the peptide may likewise be performed.

Once residues critical for antagonist activity are identified by the alanine scan, 2 such positions are chosen for a fully permutated pepscan of all 20 amino acids (400 peptides) and then 2 additional positions are scanned likewise (400 peptides). Peptides are first in releasable form but held on the chip. Binding of any one of sCD28, sICOS, sCTLA-4 or sPD-1, to each peptide is scored by ELISA using commercial polyclonal antibodies or monoclonal antibodies to this receptor. Next, positive, peptides are released and pooled into groups for assay of binding the receptor in the screening assay ELISA format (see above) and also for antagonist activity *in vitro* in PBMC assays (detailed below) and the groups are then deconvoluted. In stepwise fashion, the resulting improved leads are subjected to additional rounds of positional scanning. In total, 4 rounds of positional scanning are performed, and further rounds of constrained positional scanning, on the peptide with highest affinity for the receptor.

For cyclization scan, a linker such as *m*-maleinimidobenzoic acid *N-*hydroxy-succinimide (MBS) ester may be used to react via its active ester with the *N*-terminus of a given peptide and via its maleinimide group with a free thiol group from cysteine. The cysteine is part of the peptide.

For loop scan, the *N*-terminus of each peptide may be linked with MBS to a free SH group from a cysteine that is coupled separately to the bottom of the same well. In this way, a constrained loop is formed.

Cyclic peptidomimetics are synthesized individually and evaluated for antagonist activity in PBMC.

It should be noted that a candidate antagonist selected and characterized by the screening method of the invention shown by Example 8, is a substance which binds to the superantigen binding site within any one of CD28, ICOS, CTLA-4 or PD-1, and should be further evaluated for its ability of to antagonize toxin-mediated activation of Th1 lymphocytes and optionally its ability to elicit protective immunity against toxic shock induced by a pyrogenic exotoxin or by a mixture of such pyrogenic exotoxins.

The test system used for evaluating the candidate antagonist isolated by the screening method of the invention may be an in-vitro/ex-vivo cell culture, or an in-vivo animal model. Such test system optionally further comprises endogenous and/or exogenous compounds which provide suitable conditions for the superantigen-induced activation of T cells and for the detection of an end-point indication for determining the antagonizing effect of the candidate antagonist. More specifically, the T cells are Th1 lymphocytes and said activation is determined by the induction of IL2 and/or IFN-γ gene expression.

According to one embodiment, the test system utilized by the screening method of the invention for evaluation may be an in-vitro/ex-vivo cell culture comprising an endogenously expressed CD28, ICOS, CTLA-4 or PD-1 molecules. In a particular example, the cell culture used as the test system may be a PBMC culture isolated from a mammalian donor. Such mammal may preferably be any one of human and rhesus monkey.

The end point indication in this particular test system may therefore be the superantigen-induced expression of IL2 and/or of IFN-γ, which leads to a visually detectable signal. Thus, any inhibition or even reduction of said end point is indicative of the ability of the candidate substance to specifically antagonize and inhibit the interaction of a superantigen with CD28 molecule. Such inhibition leads to antagonizing toxin-mediated activation of Th1 lymphocytes. The superantigen-induced expression of IL2 and/or of IFN-γ may be detected, for example, by quantitative dot blot hybridization and RNAase protection assay.

As demonstrated by the following Examples, the inventors have developed a powerful in vitro screening tool for super antigen antagonist activity based on the ability of an antagonist peptide to inhibit the superantigen-induced expression of Th1 cytokine mRNA in freshly isolated whole human PBMC populations that contain all cell subsets that participate in a cellular immune response [Arad *et al*., (2000) *ibid.*]. The inventors have shown that this system closely reflects the human immune response in a variety of diseases where it detected dysregulation of that response [Gerez et al., Clin. Immunol. Immunopathol. 58:152-266 (1991); Gerez et al., Kidney International 40:266-272 (1991); Gerez et al., Clin. Exp. Immunol. 109:296-303 (1997); Kaempfer et al., J. Clin. Oncol. 14:1778-1786 (1996)], thus providing an excellent surrogate marker. This PBMC assay proved effective in the discovery of the antagonist peptides *p12* and *p14* by the present inventors [Arad *et al*., (2000), (2001) *ibid.*] and for showing lack of toxin agonist activity [Arad *et al*., (2000) *ibid*.]. This system therefore may be efficiently used for evaluating the antagonist activity of the candidate antagonist substances obtained by the screening method of the invention.

The inventors have devised a sensitive, quantitative method for measuring expression of IL2 and IFN-γ mRNA induced in human PBMC, quantitating their low-abundancy mRNA species in small numbers of cells. The method allows for convenient processing of large numbers of samples, and as such, is suitable for screening potential toxin antagonists. Moreover, it allows study of responses of PBMC from several different human donors at once, for a large number of parameters. This creates an effective tool for showing antagonist activity in a reproducible manner. Measurements of IL2 and IFN-γ protein are less informative than of mRNA because these proteins appear only gradually during induction and are sequestered by binding to their cellular receptors, while mRNA is expressed promptly and can be assayed accurately. Determination of IL2 and IFN-γ mRNA gives dynamic information on the primary response of these genes within hours after immune stimulation. The assay is linear over a wide range. Information obtained from such analysis is verified by RNase protection analysis.

An essential property of the desired antagonist peptide is that it leaves the Th2 response intact. This is also a requirement for backups, and may be tested by ELISA for IL10, using culture medium from PBMC in which toxin-induced expression of Th1 cytokine mRNA is inhibited by the antagonist peptide.

In yet another alternative, the test system utilized by the screening method of the invention for evaluating candidate antagonists, may be an *in-vivo* system, particularly an animal model.

According to one specific embodiment, the animal model may be a D-galactosamine-sensitized mouse challenged with a superantigen, preferably a pyrogenic exotoxin. The end point indication for such test system may be the protection and rescue of said mouse from Iethal toxic shock. An increase in said end point is indicative of the ability of said candidate substance to specifically antagonize and inhibit the interaction between the superantigen and CD28 molecule, to antagonize the pyrogenic toxin-mediated activation of Th1 lymphocytes, to protect against toxic shock and may also indicate the ability of said candidate to indirectly elicit protective immunity against toxic shock induced by a pyrogenic exotoxin or by a mixture of at least two pyrogenic exotoxins.

An alternative animal model may be a pig challenged with a pyrogenic exotoxin. In such method the end point indication may be the protection and rescue of said pig from toxic shock and incapacitation. An increase in such end point is indicative of the capability of said candidate substance to specifically antagonize and inhibit the interaction between the superantigen and CD28 molecule, to antagonize the pyrogenic exotoxin mediated activation of Th1 lymphocytes, to protect against toxic shock and incapacitation and optionally to elicit protective immunity against toxic shock induced by a pyrogenic exotoxin or by a mixture of at least two pyrogenic exotoxins.

Also referred to is a method of preparing a therapeutic composition for the treatment of a superantigen-related disorder in a mammalian subject. This method comprises the steps of:
(a) identifying an antagonist substance that is capable of antagonizing superantigen-mediated activation of Th1 lymphocytes and preferably further capable of eliciting protective immunity against toxic shock induced by a pyrogenic exotoxin or by a mixture of at least two pyrogenic exotoxins; and
(b) admixing said antagonist substance with at least one of a pharmaceutically acceptable carrier, diluent, excipient and/or additive.

The antagonist substance used by the method of preparing a therapeutic composition may preferably be identified by the screening method of the invention, as exemplified by example 8.

Disclosed and described, it is to be understood that this invention is not limited to the particular examples, methods steps, and compositions disclosed herein as such methods steps and compositions may vary somewhat. It is also to be understood that the terminology used herein is used for the purpose of describing particular embodiments only and not intended to be limiting since the scope of the present invention will be limited only by the appended claims and equivalents thereof.

It must be noted that, as used in this specification and the appended claims, the singular forms "a", "an" and "the" include plural referents unless the content clearly dictates otherwise.

Throughout this specification and the Examples and claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The following examples are representative of techniques employed by the inventors in carrying out aspects of the present invention.

### Examples

### Experimental Procedures

### Peptides

Peptides were synthesized using fluoronyl-methoxycarbonyl chemistry, cleaved and the side chain deprotected with triflouroacetic acid. Peptides were >95% pure by high-pressure liquid chromatography and their molecular weight was verified by MALDI-TOF mass spectrometry. All peptides except p*12CC* were abutted with D-Ala residues four greater protease resistance. Scrambled sequences were obtained using a true random number generator (http://www.random.org/).

### Soluble CD28, CTLA4, ICOS and B7-2

sCD28, sICOS and sB7-2 (R&D Systems), expressed in mouse myeloma NS0 cells, are chimeric disulfide-linked homodimers comprising the 19-152, 21-141 and 20-239 amino acid extracellular domain, respectively, of the mature human ligands fused to C-terminal, His₆-tagged human IgG 1 Fc. Carrier-free sCTLA4 (R&D Systems), expressed in Sf21 cells using baculovirus, is a chimeric disulfide-linked homodimer comprising the extracellular 37-162 amino acid domain of mature human CTLA4 fused likewise to human IgG 1 Fc.

### Cell Culture and Induction of Cytokine Gene Expression

PBMC from healthy human donors were separated on Ficoll Paque (Pharmacia), washed twice with 50 ml of RPMI 1640 medium, resuspended at a density of 4x10⁶ cells/ml and cultured in this medium supplemented with 2% fetal calf serum, 2 mM glutamine, 10 mM MEM nonspecific amino acids, 100 mM Na-pyruvate, 10 mM Hepes pH 7.2, 5x10⁻⁵ M 2-mercaptoethanol, 100 u/ml penicillin, 100 µg/ml streptomycin and 5 µg/ml nystatin. SEB (Department of Toxinology, U.S. Army Medical Research Institute of Infectious Diseases) and TSST-1 (Sigma) were added to 100 ng/ml.

### mRNA Analysis

For RNase protection analysis (Arad et al., 2000), total RNA was extracted from aliquots of 3x10⁷ human PBMC with Trizol reagent (Invitrogen) and hybridized for 18 h at 42°C with genomic antisense RNA probes transcribed with α-[³²P]UTP in vitro from DNA inserted into pBS (Promega). The 600-nt IL2 probe, transcribed from the T7 promoter, is complementary to IL2 exon 3 and part of intron 3; in 8 M urea-polyacrylamide gels, it yields an RNA of 117 nt protected by IL2 mRNA. The 274-nt IFN-γ probe, transcribed from the T3 promoter, is complementary to IFN-γ exon 3 and part of intron 3 and yields an RNA of 183 nt protected by IFN-γ mRNA. Antisense RNA probes for 18S rRNA and β-actin mRNA protect 90 and 415 nt, respectively. For quantitative dot blot hybridization of IL2 and IFN-γ mRNA, aliquots of 4x10⁶ PBMC were lysed in 7.5 M guanidinium-HCl. Four serial twofold dilutions of total RNA in 10x saline sodium citrate were applied to nitrocellulose sheets in a 96-well dot blot apparatus. Each sheet was hybridized with a ³²P-labeled antisense RNA probe. Autoradiograms were scanned at 630 nm in an ELISA reader. Serial dilutions yielded linear A₆₃₀ responses over a wide range [Arad (2000) ibid.].

### Surface Plasmon Resonance Spectroscopy (SPR)

SEB, sCD28 and p12CC were diluted to 100 µg/ml in 10 mM Na acetate pH 4.0 and immobilized on a CM5 sensorchip (BIAcore) by amine-thiol coupling using the manufacturer's kit (BIAcore). Soluble CD28, CTLA4, ICOS, B7-2 or human IgG (Jackson Laboratories) were injected at 10 µl/min in 25 mM HEPES pH 7.4, 150 mM NaCl, 3.4 mM EDTA, and 0.005% surfactant P20 under conditions showing no mass transfer limitation. Affinity and kinetic analyses were performed at 25°C in a BIAcore 3000 instrument, using BIAevaluation 3.0 software.

### Phage Display

For epitope mapping, the PhD-12 combinatorial phage display library in M13KE (New England Biolabs) was panned on immobilized αCD28 mAb (MAB342, clone 37407.111, R&D Systems) following instructions of the manufacturer; displacement was with sCD28. Phages from the fourth panning were immobilized on ECL-plus membranes (Pharmacia) and binding of αCD28 was detected with horseradish peroxidase (HRP)-linked anti-mouse IgG (Jackson Laboratories). Sequences of 19 distinct inserts were aligned with CD28, without gaps. For CD28 affinity selection, the same library was panned on immobilized sCD28; displacement was with SEB.

### Mouse Lethality Assay

Female BALB/c mice (10-12 weeks; Harlan) were challenged by intraperitoneal injection of SEB (Sigma) and 20 mg D-galactosamine (Sigma). When present, antagonist peptide was injected intraperitoneally 30 min before challenge. Survival was monitored. Viability remained constant beyond 72 hr for as long as followed, two weeks. Experiments involving mice were approved by the institutional animal care and use committee.

### Example 1

### Induction of Th1 Cytokine mRNA by SEB is Attenuated by Concomitant Induction of Th2 Cytokines

When superantigen-mediated lethal toxic shock was prevented by a superantigen mimetic peptide, mice swiftly acquired immunity against further toxin challenges and developed broadly protective Abs [Arad (2000) ibid.]. The inventors reasoned that when the antagonist peptide blocks induction of a Th1 response leading to lethal shock, it might leave the Th2 response intact, allowing the development of protective immunity. Indeed, IL4 and IL10 were induced promptly by SEB in human peripheral blood mononuclear cells (PBMC)(Figure 1D) to levels that strongly inhibited the expression of IL2 and IFN-γ mRNA. Within 4-6 hr, the amplitude of the induced IL2 mRNA wave increased significantly when neutralizing Abs against IL4 or IL10 were present, whereas isotype-matched control IgG had no effect (Figure 1A). The IFN-γ gene responded even more extensively to depletion of IL4 or IL10, with enhanced amplitude and duration of the induced mRNA wave as well as earlier expression (Figures 1B and 1C). Thus, although superantigens such as SEB induce an excessive Th1 cytokine gene expression, this response remains below its full potential because it is attenuated by concomitant induction of IL4 and IL10. Early expression of these Th2 cytokines forms an integral part of the primary response to superantigen.

### Example 2

### Selective Inhibition of the Th1 Response by a Superantigen Mimetic Peptide

Induction of Th1 cytokine genes in human PBMC can be blocked by a superantigen mimetic peptide YNKKKATVQELD (p*12A*, also denoted by SEQ ID NO: 1) partly homologous to SEB₁₅₀₋₁₆₁ (TNKKKVTAQELD; the 'antagonist domain', also denoted by SEQ ID NO:2) [Arad (2000) *ibid.*]. The inventors have synthesized a related peptide VQYNKKKATVQELD (p*12B.* also denoted by SEQ ID NO: 3) in which VQ are homologous to SEB₁₄₈₋₁₄₉. p*12B* was indistinguishable from p*12A* in its ability to inhibit induction of human Th1 cytokine genes by superantigens and to protect mice from lethal challenge with SEB (PCT IL 03/00278]. Whereas p*12B* blocked the SEB-mediated induction of IL2 and IFN-γ mRNA it did not inhibit induction of IL4 and IL10 (Figure 1D). This result was reproducible and shows that the balance between Th1 and Th2 responses induced by SEB is modulated by the antagonist peptide.

Interaction of SEB with the TCR and MHC class II molecule leaves the antagonist domain accessible (Figure 1E). To explain the Th1 selectivity of p*12B* (SEQ ID NO:3), the inventors postulated that activation of Th1 cells but not of Th2 cells by a superantigen requires an additional receptor and that binding of superantigen to this receptor occurs through the antagonist domain, rendering it sensitive to inhibition by the mimetic peptide (Figure 1F).

### Example 3

### Induction of a The Response by SEB is Dependent on B7-2

Studies with CD28-deficient mice suggested that CD28 costimulation is needed for the toxicity of superantigens [Saha, B. et al., J. Exp. Med. 183:2675-2680 (1996); Mittrucker, H.W. et al. J. Exp. Med. 183(6):2481-8 (1996)]. However, it was not resolved whether B7-1 (CD80), B7-2 (CD86) or both are involved as coligands [Muraille, E. et al., Immunology 89: 245-249 (1996); Krummel, M.F. et al., Int. Immunol. 8(4):519-23 (1996)]. Induction of IL2 and IFN-γ mRNA by SEB was blocked by anti-B7-2 but not by anti-B7-1 (Figures 2A and 2B). By contrast, the concomitant induction of IL10 was resistant to either mAb (Figure 2C). These results were reproducible and show that as for conventional antigens [reviewed by Carreno and Collins (2002) *ibid*.; Collins, A.V. et al., Immunity 17(2):201-10 (2002)], induction of a Th1 response by SEB relies selectively on signaling via B7-2/CD28.

### Example 4

### Soluble CD28, CTLA4 and B7-2 Inhibit Induction of a Th1 Response by SEB

The similar selectivity of the inhibitory effect of p*12B* (SEQ ID NO: 3) and of anti-B7-2 on the Th1 response led the inventors to investigate the possibility that SEB may engage CD28 or B7-2 through its antagonist domain. Induction of IL2 and IFN-γ mRNA by SEB was inhibited by a soluble form of human CD28 comprising its 152-amino-acid extracellular domain (sCD28)(Figure 2D). By contrast, induction of IL10 by SEB in the same cell culture was essentially unaffected by sCD28 (Figure 2E). sCD28 alone did not induce these genes. sCD28 inhibited the induction of IL2 mRNA in a dose-dependent manner (Figure 2F). Like sCD28, sB7-2 inhibited SEB-mediated induction of IL2 mRNA (Figures 2G and 2H) but not the induction of IL10 (Figure 2I). Although these results could suggest that SEB uses CD28 and B7-2 as costimulatory ligands, the inventors conjectured that to elicit a Th1 response, SEB may bind directly to one or both.

CTLA4 and CD28 are structurally homologous and interact with the same B7 coligands to deliver opposite signals [reviewed by Collins (2002) *ibid*.; Sansom, D.M. et al., Trends Immunol. 24(6):314-9 (2003)]. The inventors examined the effect of sCTLA4 on expression of IL2 and IFN-γ mRNA induced by SEB. sCTLA4 blocked this expression (Figure 2J) yet as for sCD28 and sB7-2, left the induction of IL10 intact (Figure 2K). sCTLA4 could inhibit the action of SEB by competing for B7-2 as previously suggested [Zhou, T. et al., Eur. J. Immunol. 24(5):1019-25 (1994)] but the inventors considered the possibility that by binding to SEB it might impede the interaction of the superantigen with cell surface CD28 and/or B7-2.

### Example 5

### SEB Induces a Transient Change in Surface Accessibility of CD28

The inventors next used a CD28 mAb (αCD28) or a polyclonal CD28 Ab (CD28 Ab) to stain CD4-enriched human PBMC during the course of activation by SEB. CD28 was accessible to CD28 Ab on resting and activated cells but became available to αCD28 only transiently after induction with SEB, by 6 hr (Figure 3A). Hence, although CD28 is expressed constitutively on the cell surface, it undergoes a transient change in epitope presentation induced by SEB, further evidence for a functional interaction between these ligands.

### Example 6

### Superantigen Mimetic Peptide Blocks Signaling Through CD28

To examine the role of CD28 in activation of Th1 cytokine genes independently of other ligands, the inventors induced IL2 and IFN-γ mRNA with αCD28 alone. This induction was blocked by superantigen mimetic peptide p*12B* (SEQ ID NO: 3, Figure 3B). The combination of αCD28 and αCD3 elicited a higher expression than did either mAb alone; this induction was eliminated virtually in full by p*12B* (Figure 3C). By contrast, induction of IL10 by αCD28/αCD3 was resistant to p*12B* (Figure 3D). Thus, in the absence of a superantigen, the antagonist peptide selectively blocked activation of a Th1 response transduced by CD28. αCD28 bound sCD28 as expected but failed to bind to p*12B* (Figure 3E), indicating that the peptide competes with αCD28 for CD28.

The inventors next examined the effect of p*12B* on signaling of B7-2 through CD28. sB7-2 induced early and sustained expression of IFN-γ mRNA; only by 20 hr was this induction inhibited by p*12B* (Figure 3F). The superantigen mimetic peptide did not significantly inhibit induction by αCD3 but abolished induction by sB7-2 and αCD3 in combination, which was greater than that with either ligand alone. αCD28 or sB7-2 failed to induce IL10 and the induction of IL10 by αCD3 was neither augmented by αCD28 or sB7-2 nor inhibited by p*12B* (Figures 3D and 3G).

These functional studies show that induction of IL10 is independent of CD28. This provides an explanation for the resistance of the SEB-mediated induction of IL10 to p*12B* and to anti-B7-2, sB7-2, sCD28 and sCTLA4 (Figures ID and 2). Signaling through CD28 is needed selectively for the Th1 response to superantigen. The finding that even in the absence of SEB, a superantigen mimetic peptide blocked CD28-meditated Th1 activation led the inventors to examine whether superantigens use the homologous antagonist domain to bind to CD28.

### Example 7

### SEB Binds CD28, CTLA4 and ICOS Through its Antagonist Domain

Surface plasmon resonance (SPR) equilibrium binding studies have shown that the interaction of superantigens with the TCR or MHC class II molecule is weak, with dissociation constants in the micromolar range [Seth, A. et al., Nature 369:324-327 (1994); Redpath, S. et al., J. Immunol 163:6-10 (1999)]. As shown by Figure 4, when SEB was immobilized on a Biacore chip, direct binding of soluble CD28, CTLA4 or ICOS, with K_{D} values of 28, 31 and 21 nM, respectively (Figures 4A, 4B, and 4C) was detected, but not of another member of the immunoglobulin superfamily, human IgG (not shown). Thus, SEB binds directly to each member of the triad of costimulatory receptors, CD28, CTLA4 and ICOS, with an affinity well above that for the TCR or MHC class II molecule.

The inventors next immobilized p*12CC* (p*12A* abutted by terminal cysteines). This peptide was as active as p12 in blocking SEB-mediated induction of IL2 and IFN-γ mRNA in human PBMC (not shown). Soluble CD28, CTLA4 and ICOS bound directly to p*12CC,* with K_{D} values of 76, 33 and 42 nM, respectively (Figures 4D, 4E, and 4F). Similar results were obtained when p*12B* (SEQ ID NO: 3) was immobilized (not shown). No binding was detected for human IgG. Affinities for the p*12* peptide did not differ significantly from those for intact SEB. The inventors conclude that SEB uses its antagonist domain to bind CD28. Consistent with a key role of this domain in superantigen function, αp*12B* Ab was fully protective against lethal challenge with SEB in the D-galactosamine-sensitized mouse (Figure 4G), an established model for the lethality of superantigens [Arad (2000) *ibid*.].

The CD28 coligand sB7-2 also bound directly to SEB, with a K_{D} of 25 nM (Figure 4H). This affinity was similar to that of sCD28 for SEB (Figure 4A) and of sCD28 for sB7-2 (K_{D}, 19 nM; Figure 4I) but association and dissociation rates were greater, attesting to lower avidity. Collins et al., [Collins (2002) *ibid*.] reported a far lower affinity of sCD28 for sB7-2 (K_{D}, 20 µM at 37°C). Most plausibly, the discrepancy results from the temperature, method of sCD28 presentation and activity of the recombinant sCD28 preparations used. By comparison, αCD28 reproducibly bound to sCD28 with a K_{D} of 2 nM.

The results from Th1 cytokine gene expression analysis (Figures 1D, 2, and 3) are readily accommodated by the finding that SEB interacts directly with CD28 and that this binding occurs through the antagonist domain in the superantigen.

### Example 8

### Selection of SEB Antagonist Peptides by Affinity for CD28

To obtain independent evidence for a direct interaction between superantigen and CD28, the inventors repeatedly panned a random 12-mer phage display library on immobilized sCD28, displacing bound phages with SEB. Over 10% of selected phages bound tightly to sCD28 (Figure 5A), and more then. 40 peptides were isolated from these phages (as denoted by SEQ ID NO: 25 to 56). Peptides from four phages were analyzed for antagonist activity. pe*12* (SHFTHNRHGHST, also denoted by SEQ ID NO: 6) strongly inhibited induction of IL2 and IFN-γ mRNA by SEB yet lacked superantigen agonist activity (Figure 5B) and failed to inhibit induction of IL10 (Figure 5C); p*d7* (WHAHPHKKPVVA, also denoted by SEQ ID NO: 7) (Figure 5E) and pc3 (FHKHKNPGSPII, also denoted by SEQ ID NO: 8) similarly inhibited the induction of Th1 cytokine genes (Figure 5F). These peptides exhibited SEB antagonist activity also in vivo. In only 2- and 5-fold molar excess over SEB, respectively, p*e12* and p*c3* protected 8/10 and 7/10 mice from lethal challenge that left no survivors in the control group (Figure 5D). Peptide p*d7* also protected mice from lethal challenge with SEB (Figure 5G). Alone, p*e12*, p*c3* and p*d7* lacked detectable toxicity (Figure 5D and 5G).

Thus, novel superantigen antagonists can be selected from random peptide sequences solely by their affinity for the SEB binding site in CD28.

### Example 9

### The Binding Site for SEB in CD28/CTLA4 Maps to the Dimer Interface

Since superantigen mimetic peptide p*12* binds CD28 (Figure 4D), the observation that p*12B* (SEQ ID NO: 3) blocked signaling by αCD28 for Th1 cytokine gene expression (Figures 3H and 3I) suggested that this peptide competes with the mAb for its epitope in CD28. Epitope mapping defined the sequence CD28₁₁₆₋₁₂₄, HVKGKHLCP, also denoted by SEQ ID NO: 9, in which HVKGKH (SEQ ID NO: 10) corresponds to the dimerization domain in CTLA4, YVIDPE (also denoted by SEQ ID NO: 18) [Schwartz (2001) *ibid*.] (Figure 6A). Though they are highly homologous and thought to fold similarly [Luhder, F. et al., J. Exp. Med. 197(8):955-66 (2003)], CD28 and CTLA4 sequences differ in this domain as well as in residues 10-15; in the folded CTLA4 protein, the two domains are juxtaposed, creating the dimer interface (Figure 6A, red and green). The conserved B7 binding domain in residues 97-105 is located on the opposite side of CTLA4 (Figure 6A, yellow), leaving the dimer interface accessible.

To verify the CD28 epitope mapping, the inventors synthesized peptide A₇HVKGKHLCP (p*TA,* also denoted by SEQ ID NO: 11). Like sCD28, p*TA* abrogated induction of IFN-γ mRNA in PBMC by αCD28, whether this induction occurred early or late (Figure 6B). This provides functional evidence that the mAb engages the CD28₁₁₆₋₁₂₄ domain, which most likely includes part of the CD28 dimer interface.

### Example 10

### Peptide Mimetics of the Predicted Dimer Interface in CD28 Block Induction of a Th1 Response by Superantigens

The above results imply that to elicit a Th1 response, SEB must bind to CD28 at a site that overlaps with the HVKGKHLCP motif (SEQ ID NO: 9). Like sCD28 (Figure 2), p*TA* blocked the induction of IL2 and IFN-γ mRNA by SEB (Figure 6C), in a dose-dependent manner (Figure 6F) [as shown by WO 03/084995]. By contrast, it left the induction of IL10 and IL4 intact (Figures 6D and 6E), reflecting the Th1 specificity of superantigen mimetic peptide p*12B* (Figures 1 and 3). The inventors next synthesized CD28 mimetic peptides HVKGKHLCP (p*1TA,* also denoted by SEQ ID NO: 9) and SPMLVAYD (p*2TA;* OD28₈₋₁₅, also denoted by SEQ ID NO: 12) [as shown by a previous application of the inventors, 16824wo, unpublished]. Based merely on epitope mapping, *p2TA* would not be expected to act as SEB antagonist. The inventors posited that p*2TA* might be an antagonist if, to induce a Th1 response, the superantigen must contact both rims of the dimer interface predicted for CD28 (Figure 6A). Indeed, P*1TA* (SEQ ID NO: 9) and *p2TA* (SEQ ID NO: 12) each antagonized SEB-induced expression of IL2 and IFN-γ mRNA (Figure 6G). The combination of p*1TA* and p*2TA* was not significantly more potent. p*1TAsc*, which contains the amino acids of p*1TA* in a randomly scrambled order, lacked antagonist activity (Figure 7A). These results provide strong evidence that the functional binding site for superantigens in CD28 is composite, formed from sequences in *p1TA* and *p2TA.*

Within the bacterial superantigen family, toxic shock syndrome toxin-1 (TSST-1) differs most extensively from the other members, showing only 6% overall sequence homology with SEB. Although TSST-1 exhibits in its antagonist domain FDKKQLAISTLD (also denoted by SEQ ID NO: 13) far less sequence homology than other superantigens. to SEB domain TNKKKVTAQELD (also denoted by SEQ ID NO: 2), this domain nonetheless shows spatial conservation [Arad (2000) *ibid.*]. Indeed, p*1TA* and *p2TA* inhibited induction of IL2 and IFN-γ mRNA by TSST-1 (Figure 6H). Like p*12A,* therefore, p*1TA* and p*2TA* exhibit broad-spectrum activity as superantigen antagonists. Most likely, they act by competing with cell surface CD28 for the antagonist domain in superantigens.

### Example 11

### CD28 Mimetic Peptides from the Dimer Interface Protect Mice from Lethal Shock

The inventors used the mouse model to examine whether p*1TA* (SEQ ID NO: 9) and p*2TA* (SEQ ID NO: 12) exhibit SEB antagonist activity in vivo. Whereas all (10/10) controls were killed by SEB, 7/10 mice survived that had received a single dose of p*1TA* shortly before SEB (Figure 7B). p*1TA* provided 70% protection when present in 3.6-fold molar excess over SEB. By contrast, p*1TAsc* failed to provide protection. In 12-fold molar excess over SEB, p*12B* also gave 70% protection. p*2TA,* but not p*2TAsc*, protected mice from lethal shock (Figure 7C). Among controls challenged with SEB alone, 3/10 survived as compared to 8/10 survivors for mice that also received a single dose of p*2TA.* p*2TA* was effective as antagonist in vivo in only 0.8-fold molar ratio to SEB. These results were reproducible. Alone, p*1TA* and p*2TA* lacked detectable toxicity even at concentrations 25- and 125-fold greater, respectively, than needed for protection (Figures 7B and 7C).

The ability of p*1TA* and p*2TA* to protect mice from SEB-induced lethal shock in very low molar excess over the toxin and to block induction of Th1 cytokine mRNA (Figures 6G and 6H) shows that each of the two rims of the predicted CD28 dimer interface plays a critical role in the activation of a deleterious Th1 response by the superantigen.

### Example 12

### Peptide Mimetics of the Dimer Interface in CTLA4 or ICOS Are SEB Antagonists

SEB bound not only to CD28 but also to the related receptors CTLA4 and ICOS (Figure 4). The inventors examined whether interaction with CTLA4 also takes place at the dimer interface. Indeed, peptides derived from each of the two rims of the CTLA4 dimer interface, YVIDPEPCP (p*1TB,* also denoted by SEQ ID NO; 14) and PAVVLASS (p*2TB,* also denoted by SEQ ID NO; 15), were potent SEB antagonists that inhibited the induction of IL2 and IFN-γ genes yet left induction of IL10 intact (Figures 7D and 7E). When present in only twofold molar excess over SEB, p*1TB* and p*2TB* protected mice from lethal challenge (Figure 7F).

ICOS, the third member of the coreceptor triad [Hutloff, A. et al., Nature 397(6716):263-6 (1999); Coyle, A.J. et al., Immunity 13(1):95-105 (2000)], uses a different coligand, ICOSL [reviewed by Sharpe and Freeman, (2002) *ibid*.] and thus appears to function distinctly from CD28 and CTLA4. As shown by Figure 6I, the inventors aligned human ICOS with CD28 and synthesized two peptides, YESQLCCQL, (p*1TC*, also denoted by SEQ ID NO: 16) and GEINGSAN (p*2TC,* also denoted by SEQ ID NO: 17), postulating that these may correspond to the bipartite dimer interface in CTLA4/CD28. Indeed, p*1TC* and p*2TC* inhibited the Th1 cytokine response to SEB but not the induction of IL10 (Figures 7G and 7H) and protected mice from lethal challenge with the superantigen (Figure 7I).

The inventors conclude that SEB has the potential to bind directly not only to the (predicted) dimer interface in CD28 but also in CTLA4 and ICOS. Peptides derived from the dimer interfaces in the CD28/CTLA4/ICOS triad, though totally lacking in homology, are potent superantigen antagonists that block the induction of a Th1 cytokine response and protect against lethal toxic shock. Thus, two distinct classes of antagonist peptides define a critical role for the direct engagement of superantigen and CD28: superantigen mimetics that compete with superantigen for CD28 and mimetics of the coreceptor triad that compete with CD28 for superantigen (Figure 7J).

## Claims

1. An isolated and purified peptide consisting of:
(a) a peptide comprising an amino acid sequence that is a dimer interface of a T cell costimulatory pathway member selected from the dimer interface within the human CD28, the human CTLA4, the human ICOS and the human PD-1, said amino acid sequence being selected from amino acid residues 10-15 or 116-121 of SEQ ID NO: 19, amino acid residues 10-15 or 115-120 of SEQ ID NO: 20, amino acid residues 10-15 or 119-124 of SEQ ID NO: 21, and amino acid residues 8-13 or 110-116 of SEQ ID NO: 59, where the peptide:
(i) is a human CD28 dimer interface peptide consisting of the amino acid sequence HVKGKHLCP as denoted by SEQ ID NO: 9 or the amino acid sequence SPMLVAYD as denoted by SEQ ID NO: 12;
(ii) is a human CTLA4 dimer interface peptide consisting of the amino acid sequence YVIDPEPCP as denoted by SEQ ID NO: 14 or the amino acid sequence PAWLASS as denoted by SEQ ID NO: 15;
(iii) is a human ICOS dimer interface peptide consisting of the amino acid sequence YESQLCCQL as denoted by SEQ ID NO: 16 or the amino acid sequence GEINGSAN as denoted by SEQ ID NO: 17; or
(iv) is a human PD-1 dimer interface peptide consisting of the amino acid sequence RVTERRAEV as denoted by SEQ ID NO: 57 or the amino acid sequence PALLWTE as denoted by SEQ ID NO: 58;
(b) a peptide which is at least 80% homologous to the human CD28 peptide of SEQ ID No: 9 or 12, wherein the resultant peptide of (b) maintains the ability to inhibit the interaction between a T cell costimulatory pathway member and a superantigen;
(c) a peptide of (a) which has an insertion of one amino acid residue, wherein the resultant peptide of (c) maintains the ability to inhibit the interaction between a T cell costimulatory pathway member and a superantigen;
(d) a peptide of (a), (b) or (c) that is extended at the N terminus and/or the C terminus:
(i) by a lauryl cysteine at the N terminus and a cysteine at the C terminus; or
(ii) by an organic moiety that is not a naturally occurring or synthetic amino acid residue; or
(iii) by identical hydrophobic amino acid residue(s) which may be naturally occurring or synthetic amino acid residue(s); or
(iv) by a lysyl-palmitoyl tail, wherein said tail is at the N terminus, wherein the resultant peptide of (c) maintains the ability to inhibit the interaction between a T cell costimulatory pathway member and a superantigen;
(e) a dimer or multimer of (a), (b), (c), or (d) wherein the resultant peptide of (e) maintains the ability to inhibit the interaction between a T Cell costimulatory pathway member and a superantigen.

2. The peptide according to claim 1, wherein said isolated and purified peptide consists of:
(a) a peptide consisting of an amino acid sequence that is selected from the amino acid sequence HVKGKHLCP as denoted by SEQ ID NO: 9 and the amino acid sequence SPMLVAYD as denoted by SEQ ID NO: 12;
(b) a peptide which is at least 80% homologous to the peptide of (a), wherein the resultant peptide of (b) maintains the ability to inhibit the interaction between a T cell costimulatory pathway member and a superantigen;
(c) a peptide of (a) or (b) that is extended at the N terminus and/or the C terminus:
(v) by a lauryl cysteine at the N terminus and a cysteine at the C terminus; or
(vi) by an organic moiety that is not a naturally occurring or synthetic amino acid residue; or
(vii) by identical hydrophobic amino acid residue(s) which may be naturally occurring or synthetic amino acid residue(s); or
(viii) by a lysyl-palmitoyl tail, wherein said tail is at the N terminus, wherein the resultant peptide of (c) maintains the ability to inhibit the interaction between a T cell costimulatory pathway member and a superantigen; or
(d) a dimer or multimer of (a), (b) or (c), wherein the resultant peptide of (d) maintains the ability to inhibit the interaction between a T Cell costimulatory pathway member and a superantigen.

3. The peptide according to claim 2, wherein said peptide is designated p1TA and has the amino acid sequence HVKGKHLCP as denoted by SEQ ID NO: 9.

4. The peptide according to claim 2, wherein said peptide is designated p2TA and has the amino acid sequence SPMLVAYD as denoted by SEQ ID NO: 12.

5. The peptide according to claim 1, wherein said isolated and purified peptide consists of:
(a) a peptide consisting of an amino acid sequence that is selected from one of the amino acid sequence YVIDPEPCP as denoted by SEQ ID NO: 14 and the amino acid sequence PAWLASS as denoted by SEQ ID NO: 15;
(b) a peptide of (a) that is extended at the N terminus and/or the C terminus:
(i) by lauryl cysteine at the N terminus and a cysteine at the C terminus; or
(ii) by an organic moiety that is not a naturally occurring or synthetic amino acid residue; or
(iii) by identical hydrophobic amino acid residue(s) which may be naturally occurring or synthetic amino acid residue(s); or
(iv) by a lysyl-palmitoyl tail, wherein said tail is at the N terminus, wherein the resultant peptide of (b) maintains the ability to inhibit the interaction between a T cell costimulatory pathway member and a superantigen; or
(c) a dimer or multimer of (a) or (b), wherein the resultant peptide of (c) maintains the ability to inhibit the interaction between a T Cell costimulatory pathway member and a superantigen.

6. The peptide according to claim 5, wherein said peptide is designated p1TB and has the amino acid sequence YVIDPEPCP as denoted by SEQ ID NO: 14.

7. The peptide according to claim 5, wherein said peptide is designated p2TB and has the amino acid sequence PAVVLASS as denoted by SEQ ID NO: 15.

8. The peptide according to claim 1, wherein said isolated and purified peptide consists of:
(a) peptide consisting of an amino acid sequence that is selected from the amino acid sequence YESQLCCQL as denoted by SEQ ID NO: 16 and the amino acid sequence GEINGSAN as denoted by SEQ ID NO: 17;
(b) a peptide of (a) or (b) that is extended at the N terminus and/or the C terminus:
(i) by lauryl cysteine at the N terminus and a cysteine at the Cterminus; or
(ii) by an organic moiety that is not a naturally occurring or synthetic amino acid residue; or
(iii) by identical hydrophobic amino acid residue(s) which may be naturally occurring or synthetic amino acid residue(s); or
(iv) by a lysyl-palmitoyl tail, wherein said tail is at the N terminus, wherein the resultant peptide of (b) maintains the ability to inhibit the interaction between a T cell costimulatory pathway member and a superantigen; or
(c) a dimer or multimer of (a) or (b), wherein the resultant peptide of (c) maintains the ability to inhibit the interaction between a T Cell costimulatory pathway member and a superantigen.

9. The peptide according to claim 8, wherein said peptide is designated p1TC and has the amino acid sequence YESQLCCQL as denoted by SEQ ID NO: 16.

10. The peptide according to claim 8, wherein said peptide is designated p2TC and has the amino acid sequence GEINGSAN, as denoted by SEQ ID NO: 17.

11. The peptide according to claim 1, wherein said isolated and purified peptide consists of:
(a) a peptide consisting of an amino acid sequence that is selected from the amino acid sequence RVTERRAEV as denoted by SEQ ID NO: 57 and the amino acid sequence PALLWTE as denoted by SEQ ID NO: 58;
(b) a peptide of (a) that is extended at the N terminus and/or the C terminus:
(i) by lauryl cysteine at the N terminus and a cysteine at the C-terminus; or
(ii) by an organic moiety that is not a naturally occurring or synthetic amino acid residue; or
(iii) by identical hydrophobic amino acid residue(s) which may be naturally occurring or synthetic amino acid residue(s); or
(iv) by a lysyl-palmitoyl tail, wherein said tail is at the N terminus, wherein the resultant peptide of (b) maintains the ability to inhibit the interaction between a T cell costimulatory pathway member and a superantigen; or
(c) a dimer or multimer of (a) or (b), wherein the resultant peptide of (c) maintains the ability to inhibit the interaction between a T Cell costimulatory pathway member and a superantigen.

12. The peptide according to claim 11, wherein said peptide is designated p1TD and has the amino acid sequence RVTERRAEV as denoted by SEQ ID NO: 57.

13. The peptide according to claim 11, wherein said peptide is designated p2TD and has the amino acid sequence PALLWTE, as denoted by SEQ ID NO: 58.

14. The peptide according to claim 1, wherein said peptide is extended at the N-terminus and/or C-terminus thereof with D-alanine.

15. The peptide according to claim 1, wherein the peptide is as defined in any one of claims 3, 4, 6, 7, 9, 10, 12 or 13 and is abutted with D-Ala residue(s) at the N-terminus and/or C-terminus thereof.

16. The peptide according to claim 15, wherein the peptide has the amino acid sequence defined in claim 3 of SPMLVAYD, as denoted by SEQ ID NO: 12, which is abutted with a D-Ala residue at the N-terminus and/or C-terminus thereof.

17. The peptide according to claim 16, wherein the peptide has the amino acid sequence defined in claim 3 of SPMLVAYD, as denoted by SEQ ID NO: 12, which is abutted with a D-Ala residue at the N-terminus and C-terminus thereof.

18. The peptide according to claim 1, wherein the peptide is as herein designated p*2TA,* comprising the amino acid sequence SPMLVAYD as denoted by SEQ ID NO: 12, wherein said peptide is extended at the N-terminus and/or C-terminus thereof with D-alanine.

19. A composition comprising as an active ingredient a purified peptide as defined in any one of claims 1 to 18 or any combination thereof and optionally further comprising pharmaceutically acceptable carrier, diluent, adjuvant and/or excipient.

20. The composition according to claim 19 , for use in a method of treatment of an immune disorder related to an imbalance in the Thl-Th2 response in a subject in need thereof, wherein said immune disorder is any one of an autoimmune disease, malignant and non-malignant proliferative disorder, graft rejection pathology, and a disorder induced by a pyrogenic exotoxin or by a mixture of at least two pyrogenic exotoxins selected from toxic shock and incapacitation or for use in a method of preventing death induced by a pyrogenic exotoxin or by a mixture of at least two pyrogenic exotoxins.

21. The composition for use in a method of treatment according to claim 20, wherein said pyrogenic exotoxin is a superantigen from a bacterial pathogen selected from the group consisting of *Staphylococcus aureus* and *Streptococcus pyogenes.*

22. The composition for use in a method of treatment according to claim 21, where the method protects against toxic shock induced by a pyrogenic exotoxin or by a mixture of pyrogenic exotoxins.

23. The composition according to claim 19, wherein said peptide comprises an amino acid sequence as denoted by any one of p*1TA* (as denoted by SEQ ID NO: 9), p*2TA* (as denoted by SEQ ID NO: 12), p*1TB* (as denoted by SEQ IDNO: 14), p*2TB* (as denoted by SEQ ID NO: 15), p*1TC* (as denoted by SEQ ID NO: 16), p*2TC* (as denoted by SEQ ID NO: 17), p*1TD* (as denoted by SEQ ID NO: 57), p*2TD* (as denoted by SEQ ID NO: 58) and any combination thereof.

24. The composition according to claim 19, wherein said peptide is extended at the N-terminus and/or C-terminus thereof with D-alanine.

25. Use of a peptide according to any one of claims 1 to 18 for the preparation of a composition for use in the treatment of immune disorders related to an imbalance in the Thl-Th2 response in a subject in need thereof, wherein said immune disorder is any one of an autoimmune disease, malignant and non-malignant proliferative disorder, graft rejection pathology, and a disorder induced by a pyrogenic exotoxin or by a mixture of at least two pyrogenic exotoxins selected from toxic shock and incapacitation or for use in a method of preventing death induced by a pyrogenic exotoxin or by a mixture of at least two pyrogenic exotoxins.

26. The use according to claim 25, wherein said peptide is as defined in any one of claims 1 to 18 or any combination thereof.

27. The use according to claim 26, wherein preferably, said peptide is selected from the group consisting of *pta* (as denoted by SEQ ID NO: 11), p*1TA* (as denoted by SEQ ID NO: 9), p*2TA* (as denoted by SEQ ID NO: 12), p*1TB* (as denoted by SEQ ID NO: 14), p*2TB* (as denoted by SEQ ID NO: 15), p*1TC* (as denoted by SEQ ID NO: 16), p*2TC* (as denoted by SEQ ID NO: 17), p*1TD* (as denoted by SEQ ID NO: 57), p*2TD* (as denoted by SEQ ID NO: 58) and any combination thereof.

28. The use according to claim 25 or 26, wherein said peptide is extended at the N-terminus and/or C terminus thereof with D-alanine.

29. A method of screening for a test substance which specifically binds to a T cell costimulatory pathway member and thereby inhibits activation of T-lymphocytes mediated by a pathogenic-agent, which screening method comprises the steps of:
(a) obtaining candidate antagonist substances which bind to a T cell costimulatory pathway member;
(b) selecting from the substances obtained in step (a), a substance that inhibits direct interaction between said T cell costimulatory pathway member and said pathogenic agent or a component derived from said agent, preferably said component is a superantigen; and
(c) determining the inhibitory effect of the substance obtained in step (b) on the superantigen-mediated activation of T-lymphocytes, preferably, Th1 lymphocytes, wherein said candidate antagonist substance is obtained by the steps of:
(i) providing a mixture comprising an isolated peptide as defined in any one of claims 1 to 18;
(ii) contacting said mixture with said test substance under suitable conditions for said binding; and
(iii) determining the effect of the test substance on an end-point indication, whereby modulation of said end point is indicative of binding of said test substance to said peptide.

30. The screening method according to claim 29, wherein said candidate antagonist substance is evaluated by a method for determining the ability of said substance to inhibit activation of T-lymphocytes by a pathogenic agent.

## Patentansprüche

1. Isoliertes und gereinigtes Peptid, bestehend aus:
(a) einem Peptid, das eine Aminosäuresequenz umfasst, die ein Dimer-Interface eines Mitglieds des costimulatorischen Signalwegs für T-Zellen, ausgewählt aus dem Dimer-Interface innerhalb des humanen CD28, des humanen CTLA4, des humanen ICOS und des humanen PD-1, ist, wobei die Aminosäuresequenz aus Aminosäureresten 10-15 oder 116-121 von SEQ ID NO: 19, Aminosäureresten 10-15 oder 115-120 von SEQ ID NO: 20, Aminosäureresten 10-15 oder 119-124 von SEQ ID NO: 21 und Aminosäureresten 8-13 oder 110-116 von SEQ ID NO: 59 ausgewählt ist, wobei das Peptid:
(i) ein humanes CD28-Dimer-Interface-Peptid, bestehend aus der Aminosäuresequenz HVKGKHLCP, wie sie durch SEQ ID NO: 9 angegeben ist, oder der Aminosäuresequenz SPMLVAYD, wie sie durch SEQ ID NO: 12 angegeben ist, ist;
(ii) ein humanes CTLA4-Dimer-Interface-Peptid, bestehend aus der Aminosäuresequenz YVIDPEPCP, wie sie durch SEQ ID NO: 14 angegeben ist, oder der Aminosäuresequenz PAVVLASS, wie sie durch SEQ ID NO: 15 angegeben ist, ist;
(iii) ein humanes ICOS-Dimer-Interface-Peptid, bestehend aus der Aminosäuresequenz YESQLCCQL, wie sie durch SEQ ID NO: 16 angegeben ist, oder der Aminosäuresequenz GEINGSAN, wie sie durch SEQ ID NO: 17 angegeben ist, ist oder
(iv) ein humanes PD-1-Dimer-Interface-Peptid, bestehend aus der Aminosäuresequenz RVTERRAEV, wie sie durch SEQ ID NO: 57 angegeben ist, oder der Aminosäuresequenz PALLVVTE, wie sie durch SEQ ID NO: 58 angegeben ist, ist;
(b) einem Peptid, das zu wenigstens 80% homolog zu dem humanen CD28-Peptid von SEQ ID NO: 9 oder 12 ist, wobei das resultierende Peptid von (b) die Fähigkeit aufrechterhält, die Interaktion zwischen einem Mitglied des costimulatorischen Signalwegs für T-Zellen und einem Superantigen zu inhibieren;
(c) einem Peptid von (a), welches eine Insertion eines Aminosäurerestes hat, wobei das resultierende Peptid von (c) die Fähigkeit aufrechterhält, die Interaktion zwischen einem Mitglied des costimulatorischen Signalwegs für T-Zellen und einem Superantigen zu inhibieren;
(d) einem Peptid von (a), (b) oder (c), das am N-Terminus und/oder C-Terminus verlängert ist:
(i) durch ein Laurylcystein am N-Terminus und ein Cystein am C-Terminus, oder
(ii) durch eine organische Gruppierung, die kein natürlich vorkommender oder synthetischer Aminosäurerest ist, oder
(iii) durch (einen) identische(n) hydrophobe(n) Aminosäurerest(e), der/die (ein) natürlich vorkommende(r) oder synthetische(r) Aminosäurerest(e) sein kann/können, oder
(iv) durch einen Lysyl-Palmitoyl-Tail, wobei der Tail am N-Terminus ist, wobei das resultierende Peptid von (c) die Fähigkeit aufrechterhält, die Interaktion zwischen einem Mitglied des costimulatorischen Signalwegs für T-Zellen und einem Superantigen zu inhibieren;
(e) einem Dimer oder Multimer von (a), (b), (c) oder (d), wobei das resultierende Peptid von (e) die Fähigkeit aufrechterhält, die Interaktion zwischen einem Mitglied des costimulatorischen Signalwegs für T-Zellen und einem Superantigen zu inhibieren.

2. Peptid gemäß Anspruch 1, wobei das isolierte und gereinigte Peptid besteht aus:
(a) einem Peptid, bestehend aus einer Aminosäuresequenz, die aus der Aminosäuresequenz HVKGKHLCP, wie sie durch SEQ ID NO: 9 angegeben ist, und der Aminosäuresequenz SPMLVAYD, wie sie durch SEQ ID NO: 12 angegeben ist, ausgewählt ist;
(b) einem Peptid, das zu wenigstens 80% homolog zu dem Peptid von (a) ist, wobei das resultierende Peptid von (b) die Fähigkeit aufrechterhält, die Interaktion zwischen einem Mitglied des costimulatorischen Signalwegs für T-Zellen und einem Superantigen zu inhibieren;
(c) einem Peptid von (a) oder (b), das am N-Terminus und/oder C-Terminus verlängert ist:
(v) durch ein Laurylcystein am N-Terminus und ein Cystein am C-Terminus, oder
(vi) durch eine organische Gruppierung, die kein natürlich vorkommender oder synthetischer Aminosäurerest ist, oder
(vii) durch (einen) identische(n) hydrophobe(n) Aminosäurerest(e), der/die (ein) natürlich vorkommende(r) oder synthetische(r) Aminosäurerest(e) sein kann/können, oder
(viii) durch einen Lysyl-Palmitoyl-Tail, wobei der Tail am N-Terminus ist, wobei das resultierende Peptid von (c) die Fähigkeit aufrechterhält, die Interaktion zwischen einem Mitglied des costimulatorischen Signalwegs für T-Zellen und einem Superantigen zu inhibieren, oder
(d) einem Dimer oder Multimer von (a), (b) oder (c), wobei das resultierende Peptid von (d) die Fähigkeit aufrechterhält, die Interaktion zwischen einem Mitglied des costimulatorischen Signalwegs für T-Zellen und einem Superantigen zu inhibieren.

3. Peptid gemäß Anspruch 2, wobei das Peptid p1TA genannt wird und die Aminosäuresequenz HVKGKHLCP, wie sie durch SEQ ID NO: 9 angegeben ist, hat.

4. Peptid gemäß Anspruch 2, wobei das Peptid p2TA genannt wird und die Aminosäuresequenz SPMLVAYD, wie sie durch SEQ ID NO: 12 angegeben ist, hat.

5. Peptid gemäß Anspruch 1, wobei das isolierte und gereinigte Peptid besteht aus:
(a) einem Peptid, bestehend aus einer Aminosäuresequenz, ausgewählt aus der Aminosäuresequenz YVIDPEPCP, wie sie durch SEQ ID NO: 14 angegeben ist, und der Aminosäuresequenz PAVVLASS, wie sie durch SEQ ID NO: 15 angegeben ist;
(b) einem Peptid von (a), das am N-Terminus und/oder C-Terminus verlängert ist:
(i) durch Laurylcystein am N-Terminus und ein Cystein am C-Terminus, oder
(ii) durch eine organische Gruppierung, die kein natürlich vorkommender oder synthetischer Aminosäurerest ist, oder
(iii) durch (einen) identische(n) hydrophobe(n) Aminosäurerest(e), der/die (ein) natürlich vorkommende(r) oder synthetische(r) Aminosäurerest(e) sein kann/können, oder
(iv) durch einen Lysyl-Palmitoyl-Tail, wobei der Tail am N-Terminus ist, wobei das resultierende Peptid von (b) die Fähigkeit aufrechterhält, die Interaktion zwischen einem Mitglied des costimulatorischen Signalwegs für T-Zellen und einem Superantigen zu inhibieren, oder
(c) einem Dimer oder Multimer von (a) oder (b), wobei das resultierende Peptid von (c) die Fähigkeit aufrechterhält, die Interaktion zwischen einem Mitglied des costimulatorischen Signalwegs für T-Zellen und einem Superantigen zu inhibieren.

6. Peptid gemäß Anspruch 5, wobei das Peptid p1TB genannt wird und die Aminosäuresequenz YVIDPEPCP, wie sie durch SEQ ID NO: 14 angegeben ist, hat.

7. Peptid gemäß Anspruch 5, wobei das Peptid p2TB genannt wird und die Aminosäuresequenz PAVVLASS, wie sie durch SEQ ID NO: 15 angegeben ist, hat.

8. Peptid gemäß Anspruch 1, wobei das isolierte und gereinigte Peptid besteht aus:
(a) einem Peptid, bestehend aus einer Aminosäuresequenz, die ausgewählt ist aus der Aminosäuresequenz YESQLCCQL, wie sie durch SEQ ID NO: 16 angegeben ist, und der Aminosäuresequenz GEINGSAN, wie sie durch SEQ ID NO: 17 angegeben ist;
(b) einem Peptid von (a) oder (b), das am N-Terminus und/oder C-Terminus verlängert ist:
(i) durch Laurylcystein am N-Terminus und ein Cystein am C-Terminus, oder
(ii) durch eine organische Gruppierung, die kein natürlich vorkommender oder synthetischer Aminosäurerest ist, oder
(iii) durch (einen) identische(n) hydrophobe(n) Aminosäurerest(e), der/die (ein) natürlich vorkommende(r) oder synthetische(r) Aminosäurerest(e) sein kann/können, oder
(iv) durch einen Lysyl-Palmitoyl-Tail, wobei der Tail am N-Terminus ist, wobei das resultierende Peptid von (b) die Fähigkeit aufrechterhält, die Interaktion zwischen einem Mitglied des costimulatorischen Signalwegs für T-Zellen und einem Superantigen zu inhibieren, oder
(c) einem Dimer oder Multimer von (a) oder (b), wobei das resultierende Peptid von (c) die Fähigkeit aufrechterhält, die Interaktion zwischen einem Mitglied des costimulatorischen Signalwegs für T-Zellen und einem Superantigen zu inhibieren.

9. Peptid gemäß Anspruch 8, wobei das Peptid p1TC genannt wird und die Aminosäuresequenz YESQLCCQL, wie sie durch SEQ ID NO: 16 angegeben ist, hat.

10. Peptid gemäß Anspruch 8, wobei das Peptid p2TC genannt wird und die Aminosäuresequenz GEINGSAN, wie sie durch SEQ ID NO: 17 angegeben ist, hat.

11. Peptid gemäß Anspruch 1, wobei das isolierte und gereinigte Peptid besteht aus:
(a) einem Peptid, bestehend aus einer Aminosäuresequenz, die ausgewählt ist aus der Aminosäuresequenz RVTERRAEV, wie sie durch SEQ ID NO: 57 angegeben ist, und der Aminosäuresequenz PALLVVTE, wie sie durch SEQ ID NO: 58 angegeben ist;
(b) einem Peptid von (a), das am N-Terminus und/oder C-Terminus verlängert ist:
(i) durch Laurylcystein am N-Terminus und ein Cystein am C-Terminus, oder
(ii) durch eine organische Gruppierung, die kein natürlich vorkommender oder synthetischer Aminosäurerest ist, oder
(iii) durch (einen) identische(n) hydrophobe(n) Aminosäurerest(e), der/die (ein) natürlich vorkommende(r) oder synthetische(r) Aminosäurerest(e) sein kann/können, oder
(iv) durch einen Lysyl-Palmitoyl-Tail, wobei der Tail am N-Terminus ist, wobei das resultierende Peptid von (b) die Fähigkeit aufrechterhält, die Interaktion zwischen einem Mitglied des costimulatorischen Signalwegs für T-Zellen und einem Superantigen zu inhibieren, oder
(c) einem Dimer oder Multimer von (a) oder (b), wobei das resultierende Peptid von (c) die Fähigkeit aufrechterhält, die Interaktion zwischen einem Mitglied des costimulatorischen Signalwegs für T-Zellen und einem Superantigen zu inhibieren.

12. Peptid gemäß Anspruch 11, wobei das Peptid p1TD genannt wird und die Aminosäuresequenz RVTERRAEV, wie sie durch SEQ ID NO: 57 angegeben ist, hat.

13. Peptid gemäß Anspruch 11, wobei das Peptid p2TD genannt wird und die Aminosäuresequenz PALLVVTE, wie sie durch SEQ ID NO: 58 angegeben ist, hat.

14. Peptid gemäß Anspruch 1, wobei das Peptid am N-Terminus und/oder C-Terminus mit D-Alanin verlängert ist.

15. Peptid gemäß Anspruch 1, wobei das Peptid wie in einem der Ansprüche 3, 4, 6, 7, 9, 10, 12 oder 13 definiert ist und mit D-Ala-Rest(en) am N-Terminus und/oder C-Terminus abgeschlossen ist.

16. Peptid gemäß Anspruch 15, wobei das Peptid die in Anspruch 3 definierte Aminosäuresequenz von SPMLVAYD, wie sie durch SEQ ID NO: 12 angegeben ist, hat, welche mit einem D-Ala-Rest am N-Terminus und/oder C-Terminus abgeschlossen ist.

17. Peptid gemäß Anspruch 16, wobei das Peptid die in Anspruch 3 definierte Aminosäuresequenz von SPMLVAYD, wie sie durch SEQ ID NO: 12 angegeben ist, hat, die mit einem D-Ala-Rest am N-Terminus und C-Terminus abgeschlossen ist.

18. Peptid gemäß Anspruch 1, wobei das Peptid wie hierin bezeichnetes p2TA ist, das die Aminosäuresequenz SPMLVAYD, wie sie durch SEQ ID NO: 12 angegeben ist, hat, wobei das Peptid am N-Terminus und/oder C-Terminus mit D-Alanin verlängert ist.

19. Zusammensetzung, umfassend als aktives Ingrediens ein gereinigtes Peptid, wie es in einem der Ansprüche 1 bis 18 definiert ist, oder eine beliebige Kombination davon und gegebenenfalls außerdem umfassend einen pharmazeutisch annehmbaren Träger, ein pharmazeutisch annehmbares Verdünnungsmittel, Adjuvans und/oder Exzipiens.

20. Zusammensetzung gemäß Anspruch 19 zur Verwendung in einem Verfahren zur Behandlung einer Immunstörung, die mit einem Ungleichgewicht bei der Th1-Th2-Antwort in Beziehung steht, bei einem Subjekt, das einer solchen bedarf, wobei die Immunstörung eine beliebige aus einer Autoimmunerkrankung, malignen und nicht-malignen proliferativen Störung, Transplantatabstoßungspathologie und einer Störung, induziert durch ein pyrogenes Exotoxin oder durch ein Gemisch von wenigstens zwei pyrogenen Exotoxinen, ausgewählt aus toxischem Schock und Unfähigmachen (incapacitation), oder zur Verwendung in einem Verfahren zur Verhinderung von Tod, induziert durch ein pyrogenes Exotoxin oder durch ein Gemisch von wenigstens zwei pyrogenen Exotoxinen.

21. Zusammensetzung zur Verwendung in einem Verfahren zur Behandlung gemäß Anspruch 20, wobei das pyrogene Exotoxin ein Superantigen aus einem bakteriellen Pathogen, ausgewählt aus der Gruppe, bestehend aus *Staphylococcus aureus* und *Streptococcus pyogenes,* ist.

22. Zusammensetzung zur Verwendung in einem Verfahren zur Behandlung gemäß Anspruch 21, wobei das Verfahren gegen toxischen Schock, induziert durch ein pyrogenes Exotoxin oder durch ein Gemisch aus pyrogenen Exotoxinen, schützt.

23. Zusammensetzung gemäß Anspruch 19, wobei das Peptid eine Aminosäuresequenz, wie sie durch eines von p*1TA* (wie angegeben durch SEQ ID NO: 9), p*2TA* (wie angegeben durch SEQ ID NO: 12), p*1TB* (wie angegeben durch SEQ ID NO: 14), p*2TB* (wie angegeben durch SEQ ID NO: 15), p*1TC* (wie angegeben durch SEQ ID NO: 16), p*2TC* (wie angegeben durch SEQ ID NO: 17), p*1TD* (wie angegeben durch SEQ ID NO: 57), p*2TD* (wie angegeben durch SEQ ID NO: 58) und einer beliebigen Kombination daraus umfasst.

24. Zusammensetzung gemäß Anspruch 19, wobei das Peptid am N-Terminus und/oder C-Terminus mit D-Alanin verlängert ist.

25. Verwendung eines Peptids gemäß einem der Ansprüche 1 bis 18 für die Herstellung einer Zusammensetzung zur Verwendung bei der Behandlung von Immunstörungen, die mit einem Ungleichgewicht bei der Th1-Th2-Antwort in Beziehung stehen, bei einem Subjekt, das einer solchen bedarf, wobei die Immunstörung eine beliebige aus einer Autoimmunerkrankung, malignen und nicht-malignen proliferativen Störung, Transplantatabstoßungspathologie und einer Störung, induziert durch ein pyrogenes Exotoxin oder durch ein Gemisch von wenigstens zwei pyrogenen Exotoxinen, ausgewählt aus toxischem Schock und Unfähigmachen (incapacitation), oder zur Verwendung in einem Verfahren zur Verhinderung von Tod, induziert durch ein pyrogenes Exotoxin oder durch ein Gemisch von wenigstens zwei pyrogenen Exotoxinen.

26. Verwendung gemäß Anspruch 25, wobei das Peptid wie in einem der Ansprüche 1 bis 18 definiert ist oder eine Kombination davon ist.

27. Verwendung gemäß Anspruch 26, wobei das Peptid vorzugsweise ausgewählt ist aus der Gruppe, bestehend aus pta (wie angegeben durch SEQ ID NO: 11), von p*1TA* (wie angegeben durch SEQ ID NO: 9), p2TA (wie angegeben durch SEQ ID NO: 12), p*1TB* (wie angegeben durch SEQ ID NO: 14), p*2TB* (wie angegeben durch SEQ ID NO: 15), p*1TC* (wie angegeben durch SEQ ID NO: 16), p*2TC* (wie angegeben durch SEQ ID NO: 17), p*1TD* (wie angegeben durch SEQ ID NO: 57), p*2TD* (wie angegeben durch SEQ ID NO: 58) und einer beliebigen Kombination davon.

28. Verwendung gemäß Anspruch 25 oder 26, wobei das Peptid am N-Terminus und/oder C-Terminus mit D-Alanin verlängert ist.

29. Verfahren zur Durchmusterung auf eine Testsubstanz, welche spezifisch an ein Mitglied des costimulatorischen Signalwegs für T-Zellen bindet und dadurch die Aktivierung von T-Lymphocyten, welche durch ein pathogenes Agens vermittelt wird, inhibiert, wobei das Durchmusterungsverfahren die folgenden Schritte umfasst:
(a) Erhalten von Kandidaten-Antagonisten-Substanzen, welche an ein Mitglied des costimulatorischen Signalwegs für T-Zellen binden;
(b) Auswählen aus den in Schritt (a) erhaltenen Substanzen eine Substanz, die eine direkte Interaktion zwischen dem Mitglied des costimulatorischen Signalwegs für T-Zellen und dem pathogenen Agens oder einer Komponente, die von dem Agens abgeleitet ist, inhibiert, wobei die Komponente vorzugsweise ein Superantigen ist, und
(c) Bestimmen der inhibitorischen Wirkung der in (b) erhaltenen Substanz auf die Superantigen-vermittelte Aktivierung von T-Lymphocyten, vorzugsweise Th1-Lymphocyten, wobei die Kandidaten-Antagonisten-Substanz erhalten wird durch die folgenden Schritte:
(i) Bereitstellen eines Gemisches, das ein isoliertes Peptid, wie in einem der Ansprüche 1 bis 18 definiert, umfasst;
(ii) Inkontaktbringen des Gemisches mit der Testsubstanz unter geeigneten Bedingungen für die Bindung und
(iii) Bestimmen der Wirkung der Testsubstanz auf eine Endpunktanzeige, wobei eine Modulierung des Endpunktes für eine Bindung der Testsubstanz an das Peptid indikativ ist.

30. Durchmusterungsverfahren gemäß Anspruch 29, wobei die Kandidaten-Antagonisten-Substanz durch ein Verfahren zur Bestimmung der Fähigkeit der Substanz, die Aktivierung von T-Lymphocyten durch ein pathogenes Agens zu inhibieren, evaluiert wird.

## Revendications

1. Peptide isolé et purifié, qui consiste :
a) en un peptide comprenant une séquence d'acides aminés qui est une interface de dimère d'un élément de la voie de co-stimulation des lymphocytes T, choisie parmi les interfaces de dimère des molécules CD28 humaine, CTLA4 humaine, ICOS humaine et PD-1 humaine, laquelle séquence d'acides aminés est choisie parmi les résidus d'acides aminés 10-15 ou 116-121 de la Séquence N° 19, les résidus d'acides aminés 10-15 ou 115-120 de la Séquence N° 20, les résidus d'acides aminés 10-15 ou 119-124 de la Séquence N° 21, et les résidus d'acides aminés 8-13 ou 110-116 de la Séquence N° 59, étant entendu que ce peptide
i) est un peptide d'interface de dimère de CD28 humaine, constitué de la séquence d'acides aminés HVKGKHLCP, indiquée en tant que Séquence N° 9, ou de la séquence d'acides aminés SPMLVAYD, indiquée en tant que Séquence N° 12,
ii) est un peptide d'interface de dimère de CTLA4 humaine, constitué de la séquence d'acides aminés YVIDPEPCP, indiquée en tant que Séquence N° 14, ou de la séquence d'acides aminés PAVVLASS, indiquée en tant que Séquence N° 15,
iii) est un peptide d'interface de dimère d'ICOS humaine, constitué de la séquence d'acides aminés YESQLCCQL, indiquée en tant que Séquence N° 16, ou de la séquence d'acides aminés GEINGSAN, indiquée en tant que Séquence N° 17,
iv) ou est un peptide d'interface de dimère de PD-1 humaine, constitué de la séquence d'acides aminés RVTERRAEV, indiquée en tant que Séquence N° 57, ou de la séquence d'acides aminés PALLVVTE, indiquée en tant que Séquence N° 58 ;
b) en un peptide qui est, à un degré d'au moins 80 %, homologue au peptide de CD28 humaine de la Séquence N° 9 ou N° 12, lequel peptide (b) résultant conserve la capacité d'inhiber l'interaction entre un élément de la voie de co-stimulation des lymphocytes T et un super-antigène ;
c) en un peptide (a) au sein duquel est inséré un et un seul résidu d'acide aminé, lequel peptide (c) résultant conserve la capacité d'inhiber l'interaction entre un élément de la voie de co-stimulation des lymphocytes T et un super-antigène ;
d) en un peptide (a), (b) ou (c), prolongé, à l'extrémité ou aux extrémités N-terminale et/ou C-terminale :
i) par un résidu de lauryl-cystéine à l'extrémité N-terminale et un résidu de cystéine à l'extrémité C-terminale,
ii) par un fragment organique qui n'est pas un résidu d'un acide aminé, naturel ou synthétique,
iii) par un résidu, ou des résidus identiques, d'acide aminé hydrophobe, qui peut ou peuvent être un ou des résidu(s) d'un acide aminé naturel ou synthétique,
i) ou par une queue lysyl-palmitoyle, laquelle queue est placée à l'extrémité N-terminale,
lequel peptide (d) résultant conserve la capacité d'inhiber l'interaction entre un élément de la voie de co-stimulation des lymphocytes T et un super-antigène ;
e) ou en un dimère ou multimère de peptide (a), (b), (c) ou (d), lequel peptide (e) résultant conserve la capacité d'inhiber l'interaction entre un élément de la voie de co-stimulation des lymphocytes T et un super-antigène.

2. Peptide conforme à la revendication 1, lequel peptide isolé et purifié consiste :
a) en un peptide constitué d'une séquence d'acides aminés choisie parmi la séquence d'acides aminés HVKGKHLCP, indiquée en tant que Séquence N° 9, et la séquence d'acides aminés SPMLVAYD, indiquée en tant que Séquence N° 12 ;
b) en un peptide qui est, à un degré d'au moins 80 %, homologue au peptide (a), lequel peptide (b) résultant conserve la capacité d'inhiber l'interaction entre un élément de la voie de co-stimulation des lymphocytes T et un super-antigène ;
c) en un peptide (a) ou (b), prolongé, à l'extrémité ou aux extrémités N-terminale et/ou C-terminale :
v) par un résidu de lauryl-cystéine à l'extrémité N-terminale et un résidu de cystéine à l'extrémité C-terminale,
vi) par un fragment organique qui n'est pas un résidu d'un acide aminé, naturel ou synthétique,
vii) par un résidu, ou des résidus identiques, d'acide aminé hydrophobe, qui peut ou peuvent être un ou des résidu(s) d'un acide aminé naturel ou synthétique,
viii) ou par une queue lysyl-palmitoyle, laquelle queue est placée à l'extrémité N-terminale,
lequel peptide (c) résultant conserve la capacité d'inhiber l'interaction entre un élément de la voie de co-stimulation des lymphocytes T et un super-antigène ;
d) ou en un dimère ou multimère de peptide (a), (b) ou (c), lequel peptide (d) résultant conserve la capacité d'inhiber l'interaction entre un élément de la voie de co-stimulation des lymphocytes T et un super-antigène.

3. Peptide conforme à la revendication 2, lequel peptide est appelé p1TA et présente la séquence d'acides aminés HVKGKHLCP, indiquée en tant que Séquence N° 9.

4. Peptide conforme à la revendication 2, lequel peptide est appelé p2TA et présente la séquence d'acides aminés SPMLVAYD, indiquée en tant que Séquence N° 12.

5. Peptide conforme à la revendication 1, lequel peptide isolé et purifié consiste :
a) en un peptide constitué d'une séquence d'acides aminés choisie parmi la séquence d'acides aminés YVIDPEPCP, indiquée en tant que Séquence N° 14, et la séquence d'acides aminés PAVVLASS, indiquée en tant que Séquence N° 15 ;
b) en un peptide (a), prolongé, à l'extrémité ou aux extrémités N-terminale et/ou C-terminale :
i) par un résidu de lauryl-cystéine à l'extrémité N-terminale et un résidu de cystéine à l'extrémité C-terminale,
ii) par un fragment organique qui n'est pas un résidu d'un acide aminé, naturel ou synthétique,
iii) par un résidu, ou des résidus identiques, d'acide aminé hydrophobe, qui peut ou peuvent être un ou des résidu(s) d'un acide aminé naturel ou synthétique,
iv) ou par une queue lysyl-palmitoyle, laquelle queue est placée à l'extrémité N-terminale,
lequel peptide (b) résultant conserve la capacité d'inhiber l'interaction entre un élément de la voie de co-stimulation des lymphocytes T et un super-antigène ;
c) ou en un dimère ou multimère de peptide (a) ou (b), lequel peptide (c) résultant conserve la capacité d'inhiber l'interaction entre un élément de la voie de co-stimulation des lymphocytes T et un super-antigène.

6. Peptide conforme à la revendication 5, lequel peptide est appelé p1TB et présente la séquence d'acides aminés YVIDPEPCP, indiquée en tant que Séquence N° 14.

7. Peptide conforme à la revendication 5, lequel peptide est appelé p2TB et présente la séquence d'acides aminés PAVVLASS, indiquée en tant que Séquence N° 15.

8. Peptide conforme à la revendication 1, lequel peptide isolé et purifié consiste :
a) en un peptide constitué d'une séquence d'acides aminés choisie parmi la séquence d'acides aminés YESQLCCQL, indiquée en tant que Séquence N° 16, et la séquence d'acides aminés GEINGSAN, indiquée en tant que Séquence N° 17 ;
b) en un peptide (a), prolongé, à l'extrémité ou aux extrémités N-terminale et/ou C-terminale :
i) par un résidu de lauryl-cystéine à l'extrémité N-terminale et un résidu de cystéine à l'extrémité C-terminale,
ii) par un fragment organique qui n'est pas un résidu d'un acide aminé, naturel ou synthétique,
iii) par un résidu, ou des résidus identiques, d'acide aminé hydrophobe, qui peut ou peuvent être un ou des résidu(s) d'un acide aminé naturel ou synthétique,
iv) ou par une queue lysyl-palmitoyle, laquelle queue est placée à l'extrémité N-terminale,
lequel peptide (b) résultant conserve la capacité d'inhiber l'interaction entre un élément de la voie de co-stimulation des lymphocytes T et un super-antigène ;
c) ou en un dimère ou multimère de peptide (a) ou (b), lequel peptide (c) résultant conserve la capacité d'inhiber l'interaction entre un élément de la voie de co-stimulation des lymphocytes T et un super-antigène.

9. Peptide conforme à la revendication 8, lequel peptide est appelé p1TC et présente la séquence d'acides aminés YESQLCCQL, indiquée en tant que Séquence N° 16.

10. Peptide conforme à la revendication 8, lequel peptide est appelé p2TC et présente la séquence d'acides aminés GEINGSAN, indiquée en tant que Séquence N° 17.

11. Peptide conforme à la revendication 1, lequel peptide isolé et purifié consiste :
a) en un peptide constitué d'une séquence d'acides aminés choisie parmi la séquence d'acides aminés RVTERRAEV, indiquée en tant que Séquence N° 57, et la séquence d'acides aminés PALLVVTE, indiquée en tant que Séquence N° 58 ;
b) en un peptide (a), prolongé, à l'extrémité ou aux extrémités N-terminale et/ou C-terminale :
i) par un résidu de lauryl-cystéine à l'extrémité N-terminale et un résidu de cystéine à l'extrémité C-terminale,
ii) par un fragment organique qui n'est pas un résidu d'un acide aminé, naturel ou synthétique,
iii) par un résidu, ou des résidus identiques, d'acide aminé hydrophobe, qui peut ou peuvent être un ou des résidu(s) d'un acide aminé naturel ou synthétique,
iv) ou par une queue lysyl-palmitoyle, laquelle queue est placée à l'extrémité N-terminale,
lequel peptide (b) résultant conserve la capacité d'inhiber l'interaction entre un élément de la voie de co-stimulation des lymphocytes T et un super-antigène ;
c) ou en un dimère ou multimère de peptide (a) ou (b), lequel peptide (c) résultant conserve la capacité d'inhiber l'interaction entre un élément de la voie de co-stimulation des lymphocytes T et un super-antigène.

12. Peptide conforme à la revendication 11, lequel peptide est appelé p1TD et présente la séquence d'acides aminés RVTERRAEV, indiquée en tant que Séquence N° 57.

13. Peptide conforme à la revendication 11, lequel peptide est appelé p2TD et présente la séquence d'acides aminés PALLVVTE, indiquée en tant que Séquence N° 58.

14. Peptide conforme à la revendication 1, lequel peptide est doté, à son extrémité ou ses extrémités N-terminale et/ou C-terminale, d'un ou de prolongement(s) de D-alanine.

15. Peptide conforme à la revendication 1, lequel peptide est tel que défini dans l'une des revendications 3, 4, 6, 7, 9, 10, 12 et 13 et doté d'un ou de résidu(s) adjacent(s) de D-alanine, à son extrémité ou ses extrémités N-terminale et/ou C-terminale.

16. Peptide conforme à la revendication 15, lequel peptide présente la séquence d'acides aminés définie dans la revendication 3, soit SPMLVAYD, indiquée en tant que Séquence N° 12, et est doté d'un ou de résidu(s) adjacent(s) de D-alanine, à son extrémité ou ses extrémités N-terminale et/ou C-terminale.

17. Peptide conforme à la revendication 16, lequel peptide présente la séquence d'acides aminés définie dans la revendication 3, soit SPMLVAYD, indiquée en tant que Séquence N° 12, et est doté de résidus adjacents de D-alanine, à ses extrémités N-terminale et C-terminale.

18. Peptide conforme à la revendication 1, lequel peptide est tel que celui appelé ici p2TA, comprend la séquence d'acides aminés SPMLVAYD, indiquée en tant que Séquence N° 12, et lequel peptide est doté d'un ou de prolongement(s) de D-alanine, à son extrémité ou ses extrémités N-terminale et/ou C-terminale.

19. Composition comprenant, en tant qu'ingrédient actif, un peptide purifié conforme à l'une des revendications 1 à 18 ou n'importe quelle combinaison de tels peptides, et comprenant en outre, en option, un véhicule, un diluant, un adjuvant et/ou un excipient, pharmacologiquement admissible(s).

20. Composition conforme à la revendication 19, pour utilisation dans un procédé de traitement, chez un sujet qui en a besoin, d'un trouble immunitaire en relation avec un déséquilibre des réponses par lymphocytes auxiliaires de type Th1 et Th2, lequel trouble immunitaire est l'un des suivants : une maladie auto-immune, un trouble prolifératif malin ou non-malin, une pathologie de rejet de greffon, et un trouble induit par une exotoxine pyrogène ou par un mélange d'au moins deux exotoxines pyrogènes, choisi parmi un choc toxique et un trouble invalidant, ou pour utilisation dans un procédé de prévention d'une mort induite par une exotoxine pyrogène ou par un mélange d'au moins deux exotoxines pyrogènes.

21. Composition pour utilisation dans un procédé de traitement, conforme à la revendication 20, pour laquelle ladite exotoxine pyrogène est un super-antigène provenant d'une bactérie pathogène choisie parmi *Staphylococcus aureus* et *Streptococcus pyrogenes*.

22. Composition pour utilisation dans un procédé de traitement, conforme à la revendication 21, lequel procédé protège d'un choc toxique induit par une exotoxine pyrogène ou par un mélange d'exotoxines pyrogènes.

23. Composition conforme à la revendication 19, dans laquelle ledit peptide comprend une séquence d'acides aminés portant l'une des appellations suivantes : p1TA (indiquée en tant que Séquence N° 9), p2TA (indiquée en tant que Séquence N° 12), p1TB (indiquée en tant que Séquence N° 14), p2TB (indiquée en tant que Séquence N° 15), p1TC (indiquée en tant que Séquence N° 16), p2TC (indiquée en tant que Séquence N° 17), p1TD (indiquée en tant que Séquence N° 57), et p2TD (indiquée en tant que Séquence N° 58), ou n'importe quelle combinaison de celles-ci.

24. Composition conforme à la revendication 19, dans laquelle ledit peptide est doté, à son extrémité ou ses extrémités N-terminale et/ou C-terminale, d'un ou de prolongement(s) de D-alanine.

25. Utilisation d'un peptide conforme à l'une des revendications 1 à 18 pour la préparation d'une composition conçue pour être utilisée dans un procédé de traitement, chez un sujet qui en a besoin, de troubles immunitaires en relation avec un déséquilibre des réponses par lymphocytes auxiliaires de type Th1 et Th2, lequel trouble immunitaire est l'un des suivants : une maladie auto-immune, un trouble prolifératif malin ou non-malin, une pathologie de rejet de greffon, et un trouble induit par une exotoxine pyrogène ou par un mélange d'au moins deux exotoxines pyrogènes, choisi parmi un choc toxique et un trouble invalidant, ou pour être utilisée dans un procédé de prévention d'une mort induite par une exotoxine pyrogène ou par un mélange d'au moins deux exotoxines pyrogènes.

26. Utilisation conforme à la revendication 25, pour laquelle ledit peptide est tel que défini dans l'une des revendications 1 à 18, ou n'importe quelle combinaison de tels peptides.

27. Utilisation conforme à la revendication 26, pour laquelle ledit peptide est de préférence choisi dans l'ensemble constitué par les suivants : pTA (indiqué en tant que Séquence N° 11), p1TA (indiqué en tant que Séquence N° 9), p2TA (indiqué en tant que Séquence N° 12), p1TB (indiqué en tant que Séquence N° 14), p2TB (indiqué en tant que Séquence N° 15), p1TC (indiqué en tant que Séquence N° 16), p2TC (indiqué en tant que Séquence N° 17), p1TD (indiqué en tant que Séquence N° 57), et p2TD (indiqué en tant que Séquence N° 58), ou n'importe quelle combinaison de ceux-ci.

28. Utilisation conforme à la revendication 25 ou 26, pour laquelle ledit peptide est doté, à son extrémité ou ses extrémités N-terminale et/ou C-terminale, d'un ou de prolongement(s) de D-alanine.

29. Procédé de recherche par criblage d'une substance de test qui se lie spécifiquement à un élément de la voie de co-stimulation des lymphocytes T et inhibe ainsi l'activation de lymphocytes T provoquée par un agent pathogène, lequel procédé de recherche par criblage comporte les étapes suivantes :
a) obtenir des substances antagonistes candidates qui se lient à un élément de la voie de co-stimulation des lymphocytes T ;
b) sélectionner, parmi les substances obtenues dans l'étape (a), une substance qui inhibe une interaction directe entre un élément de la voie de co-stimulation des lymphocytes T et ledit agent pathogène ou un composant dérivé de cet agent, ce composant étant de préférence un super-antigène ;
c) et déterminer l'effet inhibiteur qu'a la substance obtenue dans l'étape (b) sur l'activation, provoquée par un super-antigène, de lymphocytes T, de préférence des lymphocytes auxiliaires Th1,
et dans lequel on obtient une substance antagoniste candidate en réalisant les étapes suivantes :
i) prendre un mélange comprenant un peptide isolé, tel que défini dans l'une des revendications 1 à 18 ;
ii) mettre ce mélange en contact avec ladite substance de test dans des conditions appropriées pour que ladite liaison se produise ;
iii) et déterminer l'effet de la substance de test sur un phénomène indiquant le point terminal, étant entendu qu'une modulation de ce point terminal est un indice révélateur de la liaison de ladite substance de test audit peptide.

30. Procédé de recherche par criblage conforme à la revendication 29, dans lequel on évalue ladite substance antagoniste candidate par un procédé permettant de déterminer la capacité qu'a cette substance d'inhiber l'activation de lymphocytes T par un agent pathogène.
